(19) Europäisches Patentamt
European Patent Office
Office européen des brevets

(11) Veröffentlichungsnummer: **0 237 962**
**A2**

# (12) EUROPÄISCHE PATENTANMELDUNG

(21) Anmeldenummer: **87103588.7**

(22) Anmeldetag: **12.03.87**

(51) Int.Cl.³: **C 07 D 405/14**
C 07 D 409/14, A 61 K 31/50-5
C 07 D 239/24
//A61K31/41, A61K31/415

(30) Priorität: **21.03.86 DE 3609598**

(43) Veröffentlichungstag der Anmeldung:
**23.09.87 Patentblatt 87/39**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kampe, Klaus-Dieter, Dr.**
**Am Rehsteig 1**
**D-6232 Bad Soden am Taunus(DE)**

(72) Erfinder: **Raether, Wolfgang, Dr.**
**Falkensteinstrasse 6**
**D-6072 Dreieich(DE)**

(72) Erfinder: **Dittmar, Walter, Dr.**
**Uhlandstrasse 10**
**D-6238 Hofheim am Taunus(DE)**

(72) Erfinder: **Hänel, Heinz, Dr.**
**Tannenwaldallee 80**
**D-6380 Bad Homburg(DE)**

(54) **2-Azolylmethyl-2-aryl-1,3-dioxolane und deren Salze, Verfahren zu ihrer Herstellung, sie enthaltende Mittel und ihre Verwendung.**

(57) Verbindungen der Formel I

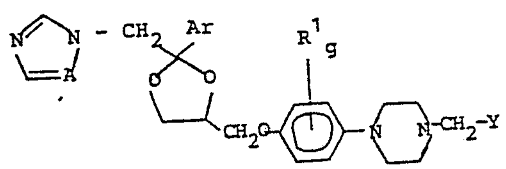

in der A gleich CH oder N, Ar gleich Phenyl, Naphtyl oder Thienyl, $R^1$ gleich Alkyl oder Halogen und Y eine Anzahl stickstoffhaltiger Heterocyclen ist, weisen hervorragende antimykotische Wirkung auf.

Verbindungen III a

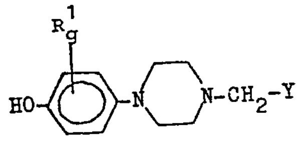

IIIa,

sind wertvolle Zwischenprodukte zu ihrer Herstellung.

EP 0 237 962 A2

Croydon Printing Company Ltd.

2-Azolylmethyl-2-aryl-1,3-dioxolane und deren Salze, Verfahren zu ihrer Herstellung, sie enthaltende Mittel und ihre Verwendung

Die Erfindung betrifft mit Heterocyclen substituierte 2-Azolylmethyl-2-aryl-4-[(4-piperazino-phenoxy)-methyl]-1,3-dioxolane einschließlich ihrer Salze, Verfahren zu ihrer Herstellung, diese Verbindungen enthaltende Arzneimittel und ihre Verwendung insbesondere als Antimykotika.

Antimykotisch bzw. fungizid wirksame 2-Azolylmethyl-2-aryl-4-[(4-piperazino-phenoxy)-methyl]-1,3-dioxolane sind bereits bekannt und werden u.a. in der DE-OS 2 804 096, und der EP-OS 7696 beschrieben. Die bekanntesten Vertreter aus der großen Zahl der beschriebenen Verbindungen sind 2-S,(R)-(2,4-Dichlorphenyl)-2-(imidazol-1-ylmethyl)-4-R,(S)-[4-(4-acetyl-piperazin-1-yl)-phenoxymethyl]-1,3-dioxolan (Ketoconazol) und 2-S,(R)-(2,4-Dichlorphenyl)-2-(1,2,4-triazol-1-ylmethyl)-4-R,(S)-[4-(4-isopropyl-piperazin-1-yl)-phenoxymethyl]-1,3-dioxolan (Terconazol), die als Antimykotika im Handel sind (vgl. DOS 2 804 096, Beispiel 20 und Beispiel 53), wobei Ketoconazol vorwiegend als systemisch wirksames und Terconazol als topisch wirksames Antimykotikum verwendet wird. Die antimykotische Wirkung sowie insbesondere die Verträglichkeit der bekannten Verbindungen sind jedoch nicht immer ganz befriedigend.

Es wurde nun gefunden, daß 2-Azolylmethyl-2-aryl-4-[(4-piperazino-phenoxy)-methyl]-1,3-dioxolane der Formel

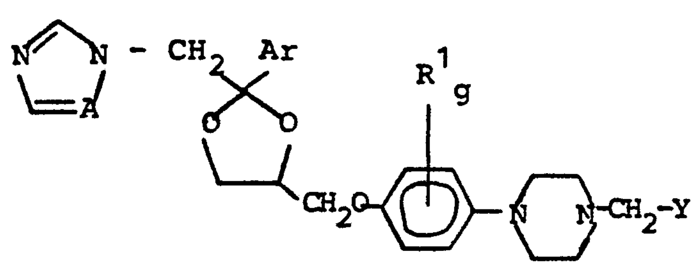

I,

in der

A CH oder N,

Ar Naphthyl, Thienyl, Halothienyl oder eine unsubstituierte oder eine bis zu 3 Substituenten tragende Phenylgruppe bedeutet, wobei die Substituenten gleich oder verschieden sein können und F, Cl, Br, J, $CF_3$, $OCH_3$, $OC_2H_5$, $CH_3$, $C_2H_5$ oder Phenoxy bedeuten,

$R^1$ $C_1$-$C_3$-Alkyl, F oder Cl,

g 0, 1 oder 2 und

Y die folgenden heterocyclischen Reste

a₁ ) 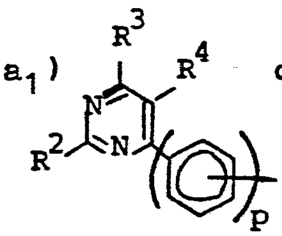 oder a₂) 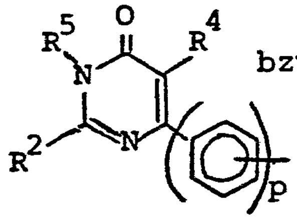 bzw. 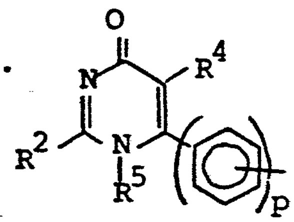

oder

b₁) 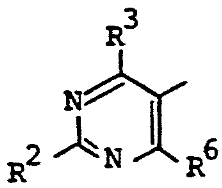 oder b₂) 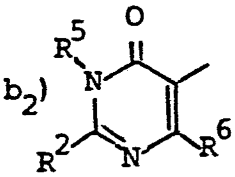 bzw. 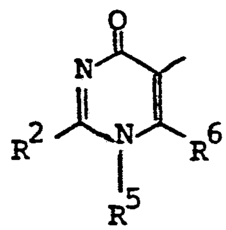

oder

c) 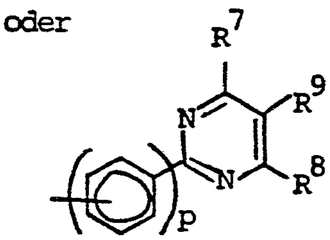

in denen

$R^2$ H, $C_1$-$C_8$-Alkyl, $C_1$-$C_4$-Alkoxy, eine unsubstituierte oder 1 oder 2 Substituenten tragende Phenylgruppe, wobei die Substituenten gleich oder verschieden sein können und F, Cl, Br, J, $CF_3$, $OCH_3$, $OC_2H_5$, $NO_2$ oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder

verschieden sein können und F, Cl, Br, $OC_2H_5$, $CH_3$ oder $C_2H_5$ bedeuten, oder beim Rest $a_1$ zusätzlich $C_1$-$C_4$-Alkylthio, eine unsubstituierte oder eine im Phenylrest 1 oder 2 Substituenten tragende Benzylthiogruppe, wobei die Substituenten gleich oder verschieden sein können und F, Cl, $OCH_3$, $OC_2H_5$, $CH_3$ oder $C_2H_5$ bedeuten, bedeutet und

$R^3$ H, OH, Cl, $C_1$-$C_4$-Alkoxy oder eine unsubstituierte oder eine im Phenylrest 1 oder 2 Substituenten tragende Benzyloxygruppe, wobei die Substituenten gleich oder verschieden sein können und F, Cl, Br, $OCH_3$, $OC_2H_5$, $CH_3$ oder $C_2H_5$ bedeuten, und beim heterocyclischen Rest $a_1$ zusätzlich $C_1$-$C_4$-Alkyl oder eine unsubstituierte oder mit einem F, Cl, Br, $OCH_3$, $OC_2H_5$, $CH_3$ oder $C_2H_5$ substituierte Phenylgruppe und beim heterocyclischen Rest $b_1$ zusätzlich $C_1$-$C_4$-Alkyl oder eine unsubstituierte oder eine 1 oder 2 Substituenten tragende Phenyloxygruppe, wobei die Substituenten gleich oder verschieden sein können und F, Cl, Br, $OCH_3$, $OC_2H_5$, $CH_3$ oder $C_2H_5$ bedeuten,

$R^4$ H, Cl, Br, $C_1$-$C_4$-Alkyl oder CN,

$R^5$ H, $C_1$-$C_4$-Alkyl, Prop-2-en-1-yl, Prop-2-in-1-yl oder Benzyl,

p 0 oder 1 bedeutet, wobei in $a_1$, $a_2$ und c der Phenylenrest über die 2,3 oder 4-Position gebunden ist, und

$R^6$ H, $C_1$-$C_8$-Alkyl, ($C_3$-$C_6$-Cycloalkyl)-$C_1$-$C_3$-alkyl, eine unsubstituierte oder 1 oder 2 Substituenten tragende Phenylgruppe, wobei die Substituenten gleich oder verschieden sein können und F, Cl, Br, J, $OCH_3$, $OC_2H_5$, 1,2--$O$-$CH_2$-$O$-, $CF_3$ oder $C_1$-$C_4$-Alkyl bedeuten, eine unsubstituierte oder im Phenylrest durch $OCH_3$, $OC_2H_5$, 1,2--$O$-$CH_2$-$O$-, $CF_3$, F, Cl oder $C_1$-$C_4$-Alkyl substituierte Phenyl-$C_1$-$C_2$-alkylgruppe, oder $CF_3$, und

$R^7$ und $R^8$ unabhängig voneinander H, $C_1$-$C_8$-Alkyl, $C_1$-$C_4$-Alkoxy oder eine unsubstituierte oder eine im Phenylrest 1 oder 2 Substituenten tragende Phenyl- oder Benzylgruppe, wobei die Substituenten gleich oder verschieden sein können und F, Cl, Br, $OCH_3$ oder $OC_2H_5$ bedeuten, und wobei $R^7$ darüberhinaus auch OH bedeuten kann, und

$R^9$ H, Cl,Br oder CN bedeutet, oder

$d_1$) 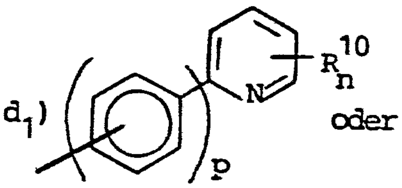 oder $d_2$) 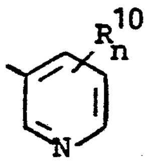 oder $d_3$) 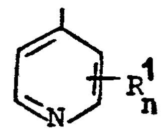

$d_4$) 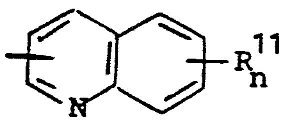 oder $d_5$) 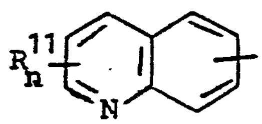 oder

$d_6$) 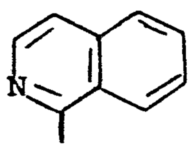 oder $d_7$) 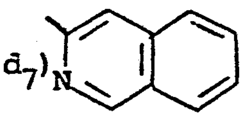

in denen

p 0 oder 1,

$R^{10}$ Cl, Br, $OCH_3$, $OC_2H_5$, $CH_3$ oder $C_2H_5$,

n 0, 1 oder 2 bedeutet, wobei $R^{10}$ in $d_1$ in 3,4,5 und/oder 6-, in $d_2$ in 2,4,5 und/oder 6- und in $d_3$ in 2,3,5 und/oder 6-Stellung stehen kann, und

$R^{11}$ Cl, $OCH_3$, $CH_3$ oder $C_2H_5$ bedeutet und in $d_4$ und $d_5$ in einer bzw. zwei der freien Positionen stehen kann, und der heterocyclische Rest $d_4$ über die 2, 3 oder 4-Stellung und der heterocyclische Rest $d_5$ über die 5,6,7 oder 8-Stellung und die Phenyleneinheit im Rest $d_1$ über die 2,3 oder 4-(para)-Stellung gebunden ist, bedeutet,

sowie deren physiologisch verträgliche Säureadditionssalze wertvolle antimykotische bzw. fungizide Eigenschaften besitzen. Sie sind daher zur Bekämpfung von Mykosen bei Mensch und Tier sowie zur Bekämpfung von Pilzbefall bei Pflanzen und auf Werkstoffen geeignet.

In diesem Zusammenhang ist unter dem Ausdruck "Halothienyl" ein in 2- oder 3-Stellung verknüpfter Thienylrest, der in beliebiger Position mit einem F-, Cl-, Br- oder J-Atom, vorzugsweise Br oder Cl, substituiert sein kann, unter den Ausdrücken "$C_1$-$C_3$-, $C_1$-$C_4$- und $C_1$-$C_8$-Alkyl" ein unverzweigter oder verzweigter Kohlenwasserstoffrest mit 1-3, 1-4 oder 1-8 C-Atomen, unter dem Ausdruck "$C_3$-$C_6$-bzw. $C_5$-$C_6$-Cycloalkyl" einen Cyclopropyl-, -butyl-, -pentyl- oder Cyclohexyl- bzw. Cyclopentyl- oder Cyclohexyl-Rest, unter dem Ausdruck "$C_1$-$C_4$-Alkoxy" bzw. "$C_1$-$C_4$-Alkylthio" eine Alkoxy- bzw. Alkylthiogruppe, deren Kohlenwasserstoffrest die unter dem Ausdruck "$C_1$-$C_4$-Alkyl" angegebene Bedeutung hat, zu verstehen.

Bevorzugt sind diejenigen Verbindungen der Formel I, in denen mindestens einer der Substituenten bzw. Indizes die folgende Bedeutung hat:

A CH oder N,
Ar eine durch 1 oder 2 F- oder Cl-Atome substituierte Phenylgruppe,
$R^1$ $CH_3$ oder $C_2H_5$
g 0 oder 2
Y
zu den heterocyclischen Resten $a_1$, $a_2$, $b_1$, $b_2$ und c:
$R^2$ H, $C_1$-$C_4$-Alkyl, $CH_3O$ oder eine unsubstituierte oder eine im Phenylrest 1 oder 2 Substituenten tragende Phenyl- oder eine Benzylgruppe, wobei die Substituenten gleich oder verschieden sein können und F, Cl, Br oder $OCH_3$ bedeuten,

$R^3$ H oder $C_1$-$C_4$-Alkoxy und beim heterocyclischen Rest $a_1$ zusätzlich $CH_3$ oder eine unsubstituierte oder eine mit einem F, Cl, $OCH_3$ oder $CH_3$ substituierte Phenylgruppe und beim Rest $b_1$ zusätzlich eine unsubstituierte oder eine 1 oder 2 Substituenten tragende Phenyloxygruppe, wobei die Substituenten gleich oder verschieden sein können und F, Cl, Br, $OCH_3$ oder $OC_2H_5$ bedeuten,

$R^4$ H oder CN und beim heteroxyclischen Rest $a_1$ zusätzlich $C_1$-$C_4$-Alkyl,

$R^5$ $CH_3$

p 0 oder 1

$R^6$ H, $C_1$-$C_8$-Alkyl, ($C_5$-$C_6$-Cycloalkyl)-$C_1$-$C_2$-alkyl, eine unsubstituierte oder 1 oder 2 Substituenten tragende Phenylgruppe, wobei die Substituenten gleich oder verschieden sein können und F, Cl, Br, $OCH_3$, $OC_2H_5$ oder $CF_3$ bedeuten, eine unsubstituierte oder im Phenylrest durch $OCH_3$, $CF_3$, F oder Cl substituierte Benzylgruppe oder $CF_3$,

$R^7$, $R^8$ unabhängig voneinander $C_1$-$C_8$-Alkyl, $C_1$-$C_4$-Alkoxy, eine unsubstituierte oder eine im Phenylrest durch F, Cl, $OCH_3$ oder $OC_2H_5$ substituierte Phenyl- oder Benzylgruppe und $R^7$ darüber hinaus auch H oder OH

$R^9$ H oder CN

zu den heterocyclischen Resten $d_1$, $d_2$, $d_3$, $d_4$, $d_5$, $d_6$ und $d_7$:

p 0 oder 1,

$R^{10}$ Cl, $CH_3$ oder $C_2H_5$,

n 0 oder 1, wobei $R^{10}$ in $d_3$ bevorzugt in 2-Stellung steht, und

$R^{11}$ Cl, wobei die Phenyleneinheit im Rest $d_1$ bevorzugt über die 4-Stellung gebunden ist.

Besonders bevorzugt sind Verbindungen der Formel I, in denen mindestens einer der Substituenten bzw. Indizes die folgende Bedeutung hat:

A CH oder N

Ar 2.4-Dichlorphenyl

$R^1$ $CH_3$

g 0 oder 2

Y

zu den heterocyclischen Resten $a_1$, $a_2$, $b_1$, $b_2$ und c:

$R^2$ $CH_3$ oder eine unsubstituierte oder eine im Phenylrest 1 oder 2 Substituenten tragende Phenyl- oder eine Benzylgruppe, wobei die Substituenten gleich oder verschieden sein können und F oder Cl und in der Phenylgruppe und beim Rest $a_1$ bei der Phenyl- und der Benzylgruppe zusätzlich $OCH_3$ bedeuten, und beim Rest $a_1$ zusätzlich $C_2$-$C_4$-Alkyl,

$R^3$ beim Rest $a_1$, falls p=0 ist, $CH_3$ oder eine unsubstituierte oder durch ein F, Cl oder $OCH_3$ substituierte Phenylgruppe, und falls p=1 ist, H, beim Rest $b_1$ H oder eine unsubstituierte oder durch F, Cl, $OCH_3$ oder $OC_2H_5$ substituierte Phenyloxygruppe,

$R^4$ H oder beim Rest $a_1$, falls p=0 ist, zusätzlich $CH_3$

$R^5$ $CH_3$

p bei den heterocyclischen Resten $a_1$ und $a_2$ 0 oder 1 und bei dem heterocyclischen Rest C 1

$R^6$ H, $CH_3$ oder eine unsubstituierte oder durch F, Cl, $OCH_3$ oder $OC_2H_5$ einfach substituierte Phenylgruppe,

$R^7$,$R^8$ unabhängig voneinander $CH_3$ oder eine unsubstituierte oder eine durch F, Cl oder $OCH_3$ substituierte Phenylgruppe und $R^7$ darüber hinaus auch H oder $C_1$-$C_4$-Alkoxy,

R9 H

zu den heterocyclischen Resten $d_1$, $d_2$, $d_3$, $d_4$, $d_5$, $d_6$ und $d_7$:

p    O oder 1

n    O,

wobei der Rest $d_4$ bevorzugt über die 2-Stellung und der Rest $d_5$ bevorzugt über die 6- oder 7-Stellung gebunden ist.

Verbindungen, bei denen Y den Rest $d_6$ oder $d_7$ bedeutet, sind nicht besonders bevorzugt.

Die Erfindung betrifft weiterhin die möglichen Stereoisomeren der Formel I, sowohl als Diastereomeren-Racemate wie auch als reine Enantiomere, sowie ihre pharmazeutisch akzeptablen Salze. Insbesondere betrifft das die wegen der 2,4-Disubstitution des 1,3-Dioxolan-Rings möglichen Stereoisomeren; die 2-Azolylmethyl-Gruppe kann zum 4-ständigen Substituenten, der substituierten Phenoxymethyl-Gruppe, cis- oder trans-ständig angeordnet sein. Die cis-Isomeren zählen zu den bevorzugten erfindungsgemäßen Verbindungen.

Als Salze der erfindungsgemäßen Verbindungen der Formel I kommen solche mit physiologisch unbedenklichen anorganischen und organischen Säuren, wie beispielsweise Chlor-, Brom- oder Jodwasserstoff-, Schwefel-, Phosphor-, Salpeter-, Benzolsulfon-, Toluolsulfon-, Sulfamin-, Methylschwefel-, Essig-, Propion-, Oel-, Palmitin-, Stearin, Malon-, Malein-, Bernstein-, Glutar-, Aepfel-, Wein-, Zitronen-, Fumar-, Milch-, Glykol-, Brenztrauben-, Benzoe-, Toluyl-, Glutamin-, Furancarbon-, Salicyl- oder Mandelsäure in Betracht. Bevorzugt werden Salze mit physiologisch verträglichen anorganischen Säuren, stark bis mittelstark sauren Derivaten solcher Säuren oder mit Fumarsäure.

Von den bekannten, gegen Pilze und Bakterien wirksamen, oben erwähnten Azolylmethyl-2-aryl-4-[(4-piperazino-phenoxy)-methyl]-1,3-dioxolanen unterscheiden sich die erfindungsgemäßen Verbindungen wesentlich durch die Art der Substituenten an der Piperazinophenoxymethyl-Einheit in der 4-Stellung des Dioxolanrings sowie durch die wahlweise vorhandene zusätzliche Substitution des Phenylrings der 4-ständigen Phenoxygruppe.

Überraschenderweise zeigen die erfindungsgemäßen 2-Azolyl-2-aryl-4-[(4-piperazino-phenoxy)-methyl]-1,3-dioxolane eine breitere und bessere antimykotische Wirkung als die bekannten 2-Azolylmethyl-2-aryl-1,3-dioxolan-Derivate und als das bekannte 2-S,(R)-(2,4-Dichlorphenyl)-2-(imidazol-1-ylmethyl)-4-R,(S)-[4-(4-acetylpiperazin-1-yl)-phenoxymethyl]-1,3-dioxolan (Ketoconazol).

Die Erfindung betrifft weiterhin ein Verfahren zur Herstellung der Verbindungen der Formel I und ihrer Salze, das dadurch gekennzeichnet ist, daß man

A) eine Verbindung der Formel II

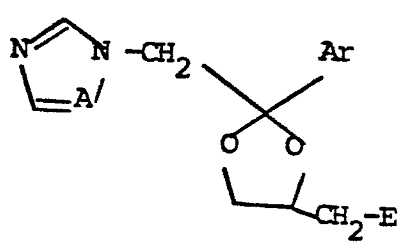

II

in der

A und Ar die zu Formel I angegebenen Bedeutungen haben und

E Halogen wie F, Cl, Br, J oder Acyloxy, wie Acetoxy, Trifluoracetyloxy, Benzoyloxy, Nitrobenzoyloxy, $C_1$-$C_3$-Alkylsulfonyloxy, wie Methansulfonyloxy, oder Arylsulfonyloxy, wie Benzol-, Nitrobenzol-, Brombenzol- oder Toluolsulfonyloxy, bedeutet,

mit einer Verbindung der Formel III

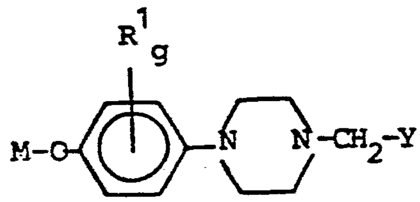

III,

in der

M H, ein Alkali- oder Erdalkalimetall, insbesondere Li, Na oder K, oder $NH_4$ bedeutet und

$R^1$, g und Y die zu Formel I angegebenen Bedeutungen haben, umsetzt, oder daß man

B) eine Verbindung der Formel IV,

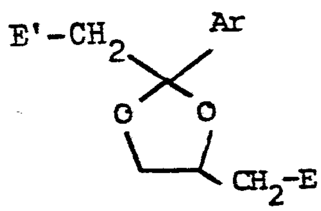

IV

in der

Ar die zu Formel I und E und E' die zu Formel II für E angegebenen Bedeutungen haben,
zunächst mit einer Verbindung der Formel III umsetzt und hierbei eine Verbindung der Formel V herstellt,

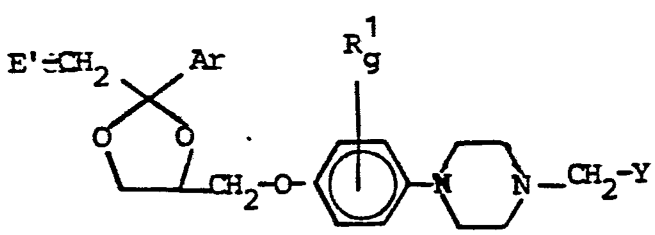

V

in der

Ar, $R^1$, g und Y die zu Formel I und E' die zu Formel II für E angegebenen Bedeutungen haben,
und anschließend eine Verbindung der Formel V mit einer

Verbindung der Formel VI umsetzt,

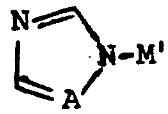

VI

in der A, CH oder N und

M' H, ein Alkali- oder Erdalkalimetall oder $Si(CH_3)_3$ bedeutet,

oder daß man

C) eine Verbindung der Formel VII,

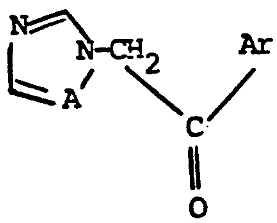

VII

in der A und Ar die zu Formel I angegebenen Bedeutungen haben, mit einem 1,2-Diol der Formel VIII

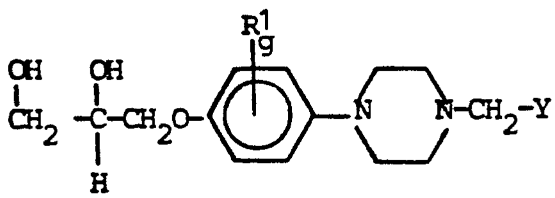

VIII

in der $R^1$, g und Y die zu Formel I angegebenen Bedeutungen haben, umsetzt,

oder daß man

D) eine Verbindung der Formel IX,

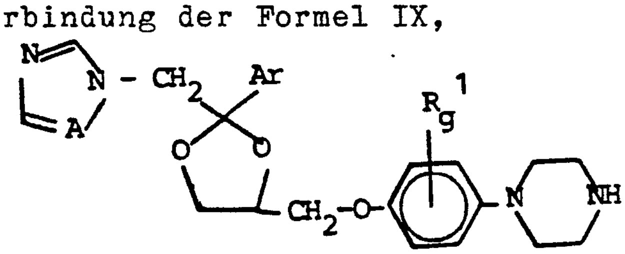

IX

in der A, Ar, $R^1$ und g die zu Formel I angegebenen Bedeutungen haben, mit einer Verbindung der Formel X,

$$E''\text{-}CH_2\text{-}Y \qquad X$$

in der E" Cl, Br, J, Acyloxy, wie Acetyloxy oder Benzoyloxy, Alkylsulfonyloxy, wie Methansulfonyloxy, oder Arylsulfonyloxy, wie Benzol-, Nitrobenzol- oder Toluolsulfonyloxy, bedeutet und Y die zu Formel I unter $a_1$, $a_2$, $b_1$, $b_2$, c, $d_1$, $d_2$, $d_3$, $d_4$, $d_5$, $d_6$ und $d_7$ angegebenen Bedeutungen hat, umsetzt, und gegebenenfalls die nach Weg A)-D) erhaltenen Verbindungen der Formel I mit anorganischen oder organischen Säuren in ihre physiologisch verträglichen Säureadditionssalze überführt.

In diesem Zusammenhang ist unter dem Ausdruck "Acyloxy" ein geradkettiger oder verzweigter $C_1$-$C_4$-Alkanoyloxy-Rest oder ein im Phenylkern unsubstituierter oder mit bis zu 2 gleichen oder verschiedenen Substituenten substituierter Benzoyloxy-Rest zu verstehen, wobei die Substituenten $CH_3$, $OCH_3$, F, Cl, oder Br bedeuten können, und unter dem Ausdruck "Arylsulfonyloxy" ein unsubstituierter oder ein mit einem Cl, Br, $CH_3$, $C_2H_5$, $OCH_3$, $OC_2H_5$ oder $NO_2$ substituierter Phenylsulfonyloxy- oder ein Naphthylsulfonyloxy-Rest zu verstehen und unter dem Ausdruck "$C_1$-$C_3$-Alkylsulfonyloxy" ein n-Alkansulfonsäure-Rest mit 1-3 C-Atomen zu verstehen.

Zur Umsetzung mit einer Verbindung der Formel IX werden bevorzugt verwendet entweder

a) eine Verbindung der Formel $Xa_1$ oder der Formel $Xa_2$

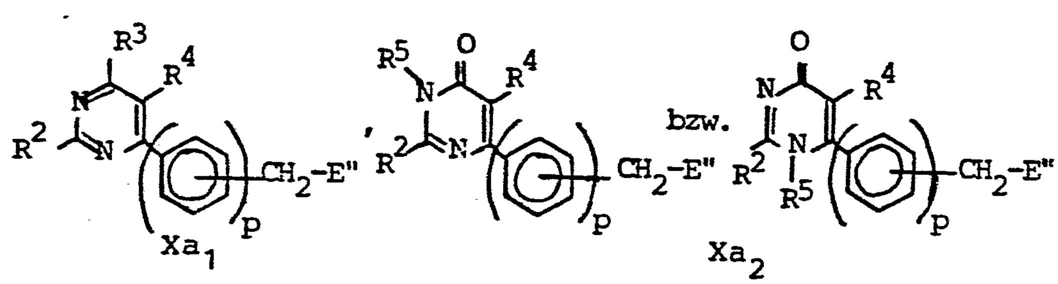

$Xa_1$ , bzw. $Xa_2$

in denen $R^2$-$R^5$ die zu Formel I angegebenen Bedeutungen haben und E", Cl bedeutet, oder

b) eine Verbindung der Formel $Xb_1$ oder der Formel $Xb_2$

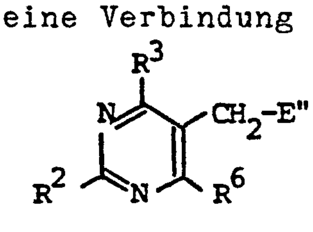

$Xb_1$

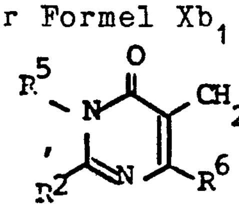

bzw.

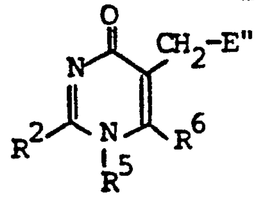

$Xb_2$

in denen $R^2$, $R^3$, $R^5$ und $R^6$ die zu Formel I angegebenen Bedeutungen haben und E" Br bedeutet, oder

c) eine Verbindung der Formel Xc,

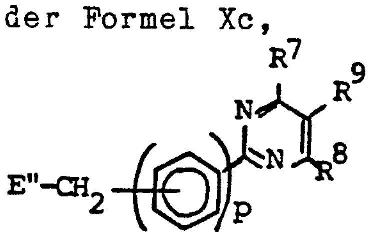

Xc,

in der $R^7$, $R^8$, $R^9$ und p die zu Formel I angegebenen Bedeutungen haben und

E" Cl oder Br bedeutet, oder

d) eine Verbindung der Formel $Xd_1$ der Formel $Xd_2$, der Formel $Xd_3$, der Formel $Xd_4$, der Formel $Xd_5$, der Formel $Xd_6$ oder der Formel $Xd_7$,

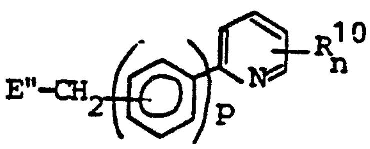

$Xd_1$,

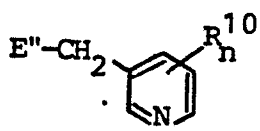

$Xd_2$,

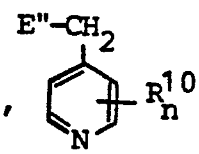

$Xd_3$

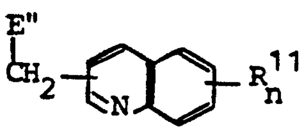

$Xd_4$,

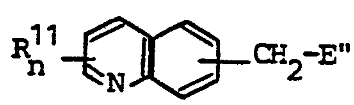

$Xd_5$

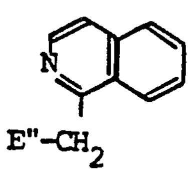

$Xd_6$

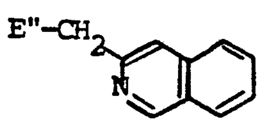

$Xd_7$

in denen $R^{10}$, $R^{11}$, p und n die zu Formel I angegebenen Bedeutungen haben und E" Cl oder Br bedeutet.

Die Verfahrens-Variante A), wobei bei den Verbindungen der Formel II E bevorzugt Cl, Br, Acetoxy, Trifluoracetoxy, Methansulfonyloxy oder (subst.) Phenylsulfonyloxy bedeutet, wird bei einer Temperatur zwischen 20°C und 160°C, vorteilhaft zwischen 40°C und 120°C, in Anwesenheit einer Base und zweckmäßig in einem inerten organischen Lösungsmittel, wie beispielsweise N,N-Dimethylformamid, N,N-Dimethylacetamid, Dimethylsulfoxid, N-Methylpyrrolidon-2, 1,2-Dimethoxyethan, Dioxan, Tetrahydrofuran, 4-Methyl-2-pentanon, Methanol, Ethanol, Isopropylalkohol, Propanol, Butanol, Pentanol, tert.-Butylalkohol, Methylglykol, Methylenchlorid, Acetonitril oder einem aromatischen Kohlenwasserstoff wie Benzol, Chlorbenzol, Toluol oder Xylol durchgeführt. Es können auch Gemische der beispielhaft genannten Lösungsmittel verwendet werden.

Geeignete Basen sind beispielsweise Alkalimetall- oder Erdalkalimetall-carbonate, -hydrogencarbonate, -hydroxide, -amide, -alkoholate oder -hydride wie z.B. Natriumcarbonat, Natriumhydrogencarbonat, Kaliumcarbonat, Natriumhydroxid, -amid oder -methylat, Kalium-t-butylat oder Natriumhydrid, oder organische Basen, z.B. tertiäre Amine wie Triethylamin, Tributylamin, Ethylmorpholin oder Pyridin, Dimethylaminopyridin, Chinolin oder 1,5-Diazabicyclo-[5,4,0]undec-5-en (DBU).

Die Umsetzung kann ebenso unter den Bedingungen einer Phasentransferreaktion ausgeführt werden, indem man die Reaktionspartner in einem geeigneten Lösungsmittel, wie z.B. Ether, Dioxan, Tetrahydrofuran, Methylenchlorid, N,N-Dimethylform- oder -acetamid, N-Methylpyrrolidon-(2), Butanol, tert.-Butanol, einem aliphatischen, cycloaliphatischen oder aromatischen Kohlenwasserstoff wie Hexan, Cyclohexan, Benzol, Toluol oder Xylol, Methylglykol, Anisol oder Chlorbenzol unter kräftigem Rühren in Gegenwart eines Phasentransferkatalysators und entweder einem gepulverten Alkalihydroxid, wie z. B. Natrium- oder Kaliumhydroxid

oder einer konzentrierten wäßrigen Lösung derselben, vorzugsweise in einem Temperaturbereich von 20°C bis 120°C aufeinander einwirken läßt.

Geeignete Phasentransferkatalysatoren sind z.B. Trialkylbenzylammonium- oder Tetraalkylammonium-halogenide, -hydroxide, -hydrogensulfate mit vorzugsweise 1 bis 12 C-Atomen im Alkylrest oder Kronenether, wie z.B. 12-Crown-4, 15-Crown-5, 18-Crown-6 oder Dibenzo-18-crown-6.

Herstellung der Ausgangsstoffe:

Die Ausgangsverbindungen der Formel II, in der Ar und A die zu Formel I angegebenen Bedeutungen haben, sind teilweise bekannt; die nicht bekannten können in Analogie zu den bekannten hergestellt werden.

Die Verfahrens-Variante B), wobei eine Verbindung der Formel IV, in der E bevorzugt Br, J oder Trifluoracetyloxy, Methansulfonyloxy, Benzol-, Nitrobenzol-, Brombenzol- oder Toluolsulfonyloxy und E' bevorzugt Cl oder Br bedeuten, mit einer Verbindung der Formel III, in der M, $R^1$, g und Y die angegebenen Bedeutungen haben, unter Bildung einer Verbindung der Formel V umgesetzt wird, wird unter den gleichen Reaktionsbedingungen wie bei Variante A zur Herstellung von Verbindungen der Formel I angegeben durchgeführt.

Die Herstellung von Verbindungen der Formel I durch Umsetzung von Verbindungen der Formel V mit Verbindungen der Formel VI erfolgt zweckmäßig in einem inerten Lösungsmittel in Gegenwart einer Base, wie zum ersten Herstellverfahren vorstehend angegeben, vorzugsweise in einem Temperaturbereich von 100 bis 190°C. Die Umsetzung erfolgt zweckmäßigerweise in Anwesenheit eines Alkalijodides wie z.B. Natrium- oder Kaliumjodid, gegebenenfalls in einem Autoklaven unter Druck.

Die vorstehend beschriebenen Umsetzungen können zweckmäßig als Eintopfreaktion durchgeführt werden, indem man zunächst bei 40 bis 100°C eine Verbindung der Formel VI mit einer Verbindung der Formel III in Gegenwart einer Base in einem inerten Lösungsmittel umsetzt. Anschliessend wird ohne Isolierung der Verbindung der Formel V eine Verbindung der Formel VI und gegebenenfalls ein weiteres Moläquivalent einer Base und ein Alkalijodid (z.B. Kaliumjodid) zugegeben und auf 100 bis 190°C erhitzt.

Herstellung der Ausgangsstoffe:

Verbindungen der Formel IV, in der E und E' die zur Formel II für E angegebenen Bedeutungen haben, sind bekannt. Sie werden hergestellt, indem man eine Verbindung der Formel

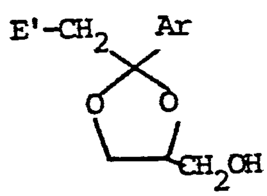

XI

XI in üblicher Weise in eine reaktionsfähige Estergruppe überführt. So werden z.B. die Verbindungen der Formel IV, in der E' bevorzugt Cl oder Br und E Methansulfonyloxy bedeuten, hergestellt, indem man eine Verbindung der Formel XI, in der Ar die zu Formel I angegebenen Bedeutungen hat und E' Cl oder Br bedeuten, mit Methansulfochlorid bei -10°C bis +50°C zweckmäßig in einem inerten Lösungsmittel, in Gegenwart einer Base umsetzt. Verbindungen der Formel IV, in der E beispielsweise Brom bedeutet, werden durch Umsetzung von Verbindungen der Formel XI (E' = Cl oder Br) mit Bromierungsmitteln, wie z.B. $PBr_3$ oder $POBr_3$ in einem inerten Lösungsmittel bei 0°C bis 100°C hergestellt. Derartige Verbindungen können auch hergestellt werden, indem man eine Verbindung der Formel XII,

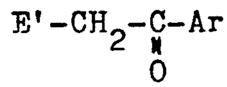

XII

in der E' Cl oder Br bedeutet und Ar die angegebenen Bedeutungen hat, mit 1-Brom-2,3-propandiol in einem inerten Lösungsmittel in Gegenwart einer starken Säure unter Bildung eines 1,3-Dioxolans nach für derartige Ketalisierungen bekannten Methoden umsetzt.

Die Verbindungen der Formel XI sind bekannt.

Die Verfahrens-Variante C), wobei man eine Verbindung der Formel VII mit einer Verbindung der Formel VIII unter Bildung einer Verbindung der Formel I umsetzt, wird im allgemeinen unter den gleichen Bedingungen durchgeführt, wie die Herstellung von Verbindungen der Formel IV (Variante B). Die Ketalisierung von Ketonen der Formel VII mit Glycerin-Derivaten der Formel VIII erfolgt vorteilhaft in einem Lösungsmittelgemisch, bestehend aus einem inerten Lösungsmittel, das mit Wasser ein azeotropes Gemisch bildet, wie z.B. Benzol, Toluol, Xylol, Chlorbenzol oder Cyclohexan, und einem Alkohol, in Anwesenheit einer starken Säure in einem Temperaturbereich von 75 bis 180°C. Vorteilhaft werden bei dieser Ketalisierung mindestens 2,5 Äquivalente einer starken Säure (bez. auf die Azolverbindung der Formel VII) und als Alkohole aliphatische Alkohole mit einem Kp zwischen 75° und 150°C und/oder Monoether von niederen Diolen, die zwischen 100° und 150°C sieden, verwendet.

Herstellung der Ausgangsstoffe:

Die Verbindungen der Formel VII sind bekannt und können nach bekannten Methoden hergestellt werden.

Verbindungen der Formel VIII, in der $R^1$, g und Y die zu Formel I angegebenen Bedeutungen haben, werden durch Umsetzung von Verbindungen der Formel III mit 1-Halogen-2,3-propandiol hergestellt, in analoger Weise wie in Org. Synth. Collect. Vol. I, S. 296 beschrieben.

Bei der Verfahrens-Variante D) wird eine Verbindung der Formel IX mit einer heterocyclischen Verbindung der Formel X, bevorzugt mit einer heterocyclischen Verbindung entweder der Formel $Xa_1$, $Xa_2$, $Xb_1$, $Xb_2$, Xc, $Xd_1$, $Xd_2$, $Xd_3$, $Xd_4$, $Xd_5$, $Xd_6$ oder $Xd_7$, in denen E" jeweils die angegebenen Bedeutungen hat, zweckmäßig in einem inerten organischen Lösungsmittel in einem Temperaturbereich von 20° bis 180°C, vorzugsweise von 50° bis 120°C, umgesetzt. Diese Umsetzung wird vorteilhaft in Gegenwart einer Base, die vorzugsweise in äquimolarer Menge angewandt wird, durchgeführt.

Die Synthese von Verbindungen der Formel I aus den Verbindungen der Formeln IX und X kann auch ohne Basenzusatz ausgeführt werden. Die Reaktanten der Formeln IX und X können in unterschiedlichen Molverhältnissen angewendet werden, d.h. es können entweder jeweils die Verbindungen der Formel IX oder diejenigen der Formel X im Überschuß angewandt werden, vorteilthaft werden äquimolare Mengen angewendet.

Als Lösungsmittel kommen beispielsweise Kohlenwasserstoffe, allgemein Ether wie Diethylether, 1,2-Dimethoxyethan, Tetrahydrofuran oder Dioxan, oder Acetonitril, Butyronitril, Dimethylform- oder -acetamid, Aceton, 4-Methyl-2-pentanon, Methylenchlorid, Chloroform, Dimethylsulfoxid, Anisol, Chlorbenzol, Tetrachlorethen oder Gemische dieser Lösungsmittel in Frage.

Geeignete Basen sind die beispielhaft bei der Verfahrens-Variante A) erläuterten.

Die Umsetzung kann ebenso unter den Bedingungen einer Phasentransferreaktion, wie sie bei der Beschreibung der Verfahrens-Variante A) erläutert sind, ausgeführt werden.

Herstellung der Ausgangsstoffe:

Die Verbindungen der Formel IX sind z.T. bekannt; solche, die von den bekannten abweichende Bedeutungen für Ar haben und/oder bei denen g 1 oder 2 bedeutet, können in Analogie zu den bekannten hergestellt werden.

Die Verbindungen der Formel X, in der E" und Y die angegebenen Bedeutungen haben, sind teilweise bekannt. Das trifft vor allem für Verbindungen der Formel $Xa_1$ und $Xd_1$-$Xd_7$ mit E" = Cl zu.

Sofern die Substituenten $R^2$ und $R^6$-$R^{11}$ an den heterocyclischen Verbindungen $Xa_1$, $Xa_2$, $Xb_1$, $Xb_2$, Xc, $Xd_1$-$Xd_5$ von bekannten Verbindungen abweichen und es sich bei den Substituenten um weitgehend inerte, also unempfindliche handelt, können diese Verbindungen bzw. deren Vorstufen, in denen die Substituenten $R^2$ und $R^6$-$R^{11}$, p und n die angegebenen Bedeutungen haben, in Analogie zu den bekannten Verbindungen dieser Strukturen hergestellt werden.

Verbindungen der Formel $Xa_1$ mit $R^3$ = Hydrocarbyloxy und $R^4$ = H oder Br werden durch Alkylierung aus den, in Bezug auf $R^2$ und $R^4$, entsprechenden Verbindungen der Formel $Xa_2$ ($R^5$ = H) mit üblichen Alkylierungsmitteln, wie Alkyliodiden, Allyl- oder Propargylbromid, Benzylbromid- oder -chlorid oder entsprechenden Alkan- oder Arylsulfonsäureestern, wie Butylmesylat oder Toluolsulfonsäureethylester hergestellt.
Bei dieser Alkylierung entstehen in der Regel überwiegend die O-alkylierten Verbindungen der Formel $Xa_1$ mit $R^3$ = Hydrocarbyloxy neben geringeren Anteilen der entsprechenden N-alkylierten Verbindungen der Formel $Xa_2$ mit $R^5$ =

Hydrocarbyl. Diese N-Alkylierung kann am N-1 oder am N-3 erfolgen. Zwischen diesen beiden Strukturen wird bei den erfindungsgemäßen Verbindungen nicht unterschieden. Deshalb sind für die Verbindungen der Formeln $Xa_2$ und $Xb_2$, sowie für die heterocyclischen Reste Y der Struktur $a_2$ und $b_2$ jeweils zwei Formeln aufgeführt. Sofern Verbindungen der Formel $Xa_2$ oder $Xb_2$ als Ausgangsstoff zur Herstellung von Verbindungen der Formel I, bei denen der heterocyclische Rest Y die Bedeutungen $a_2$ oder $b_2$ hat, Verwendung finden, handelt es sich gemäß NMR-Spektren und DC-Analyse um einheitliche Verbindungen. Das bedeutet, bei den Verbindungen $Xa_2$ bzw. $Xb_2$ ist $R^5$ entweder am N-1 oder am N-3 gebunden, wobei, wie bereits erläutert, jeweils zwischen diesen beiden Strukturen bei $a_2$ und $b_2$ nicht unterschieden wird.

Die bei der Herstellung zusammen anfallenden O-Alkyl-Verbindungen $Xa_1$ ($R^3$ = Hydrocarbyloxy) und N-Alkyl-Verbindungen $Xa_2$ ($R^5$ = Hydrocarbyl) können durch Säulenchromatographie an Kieselgel oder durch fraktionierte Umkristallisation getrennt werden.

Zur Herstellung der N-alkylierten Verbindungen der Formeln $Xa_2$ und $Xb_2$ werden Verbindungen der Formel $Xa_2$ mit $R^5$ = H und E" vorzugsweise Cl oder Verbindungen der Formel $Xb_2$ mit $R^5$ und E" = H mit Dialkylsulfaten im wässrig-alkalischen Medium alkyliert. Hierbei entstehen zwar ebenso wie vorstehend erläutert O- und N-alkylierte Verbindungen nebeneinander, aber mit einem höheren Anteil an N-alkylierten Verbindungen der Formel $Xa_2$ bzw. $Xb_2$ als bei der Alkylierung mit Alkylhalogeniden oder Sulfonsäureestern. Bevorzugt wird diese Alkylierung mit Dimethylsulfat ausgeführt, wobei überwiegend oder fast ausschließlich N-Methyl-pyrimidine der Formeln $Xa_2$ bzw. $Xb_2$ mit $R^5$ = $CH_3$ erhalten werden. Die Reinigung dieser Verbindungen, insbesondere die Abtrennung von O-Alkylverbindungen erfolgt säulenchromatographisch oder durch fraktionierte Umkristallisation. Das folgende Formelschema I dient einer Veranschaulichung dieser Synthesen.

Die Herstellung von 5-Brommethyl-pyrimidinen der Formeln $Xb_1$ und $Xb_2$ (E" = Br) erfolgt ausgehend von den entsprechenden 5-Methyl-pyrimidinen $Xb_1$ bzw. $Xb_2$ (E" = H) durch Einwirkung von N-Bromsuccinimid (NBS) (vgl. Formelschema I).

Formelschema I

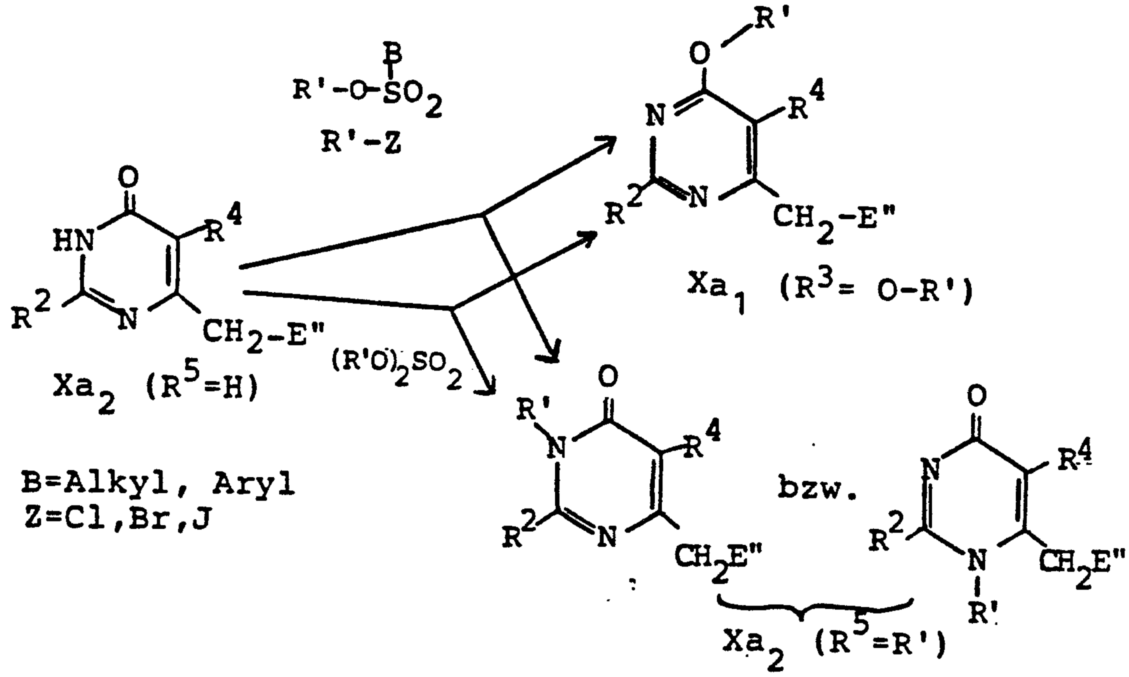

Formelschema I, Forts.

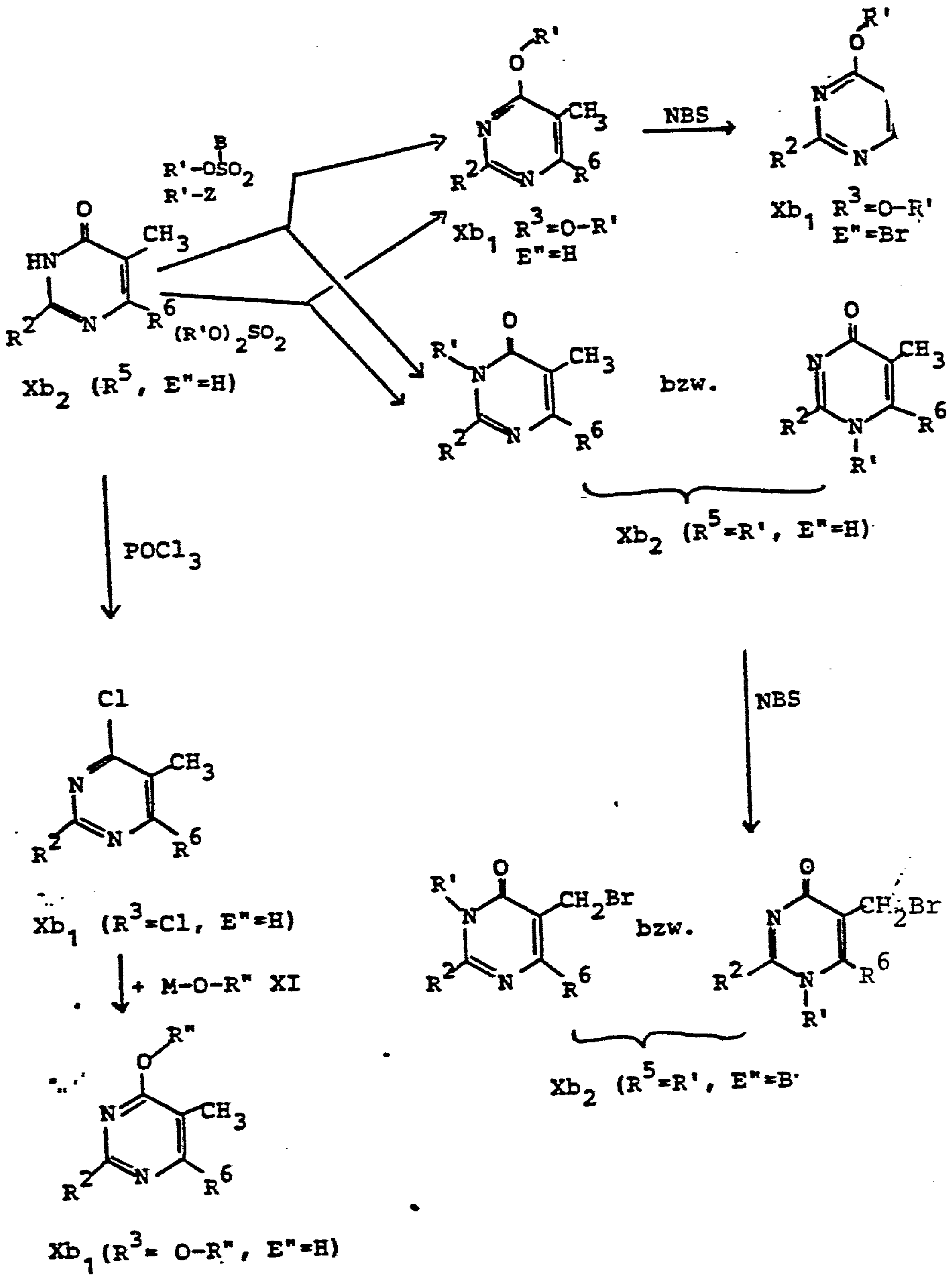

Die Verbindungen $Xa_2$ mit $R^5$ = H und E" = Cl und $Xb_2$ mit $R^5$ und E" = H sind bekannt oder können nach bekannten Methoden hergestellt werden. Verbindungen der Formel $Xa_2$ mit $R^4$ und $R^5$ = H und E" = Cl können durch Einwirkung von NBS in solche mit $R^4$ = Br, $R^5$ = H und E" = Cl übergeführt werden.

Einige Verbindungen der Formel $Xb_1$ mit $R^3$ = O-R" und E" = H, wobei R" allgemein einen, im Rahmen der bei der Formel I für $R^3$ angegebenen Bedeutungen, Kohlenwasserstoff- oder einen (substituierten) Phenylrest bedeutet, sind bekannt. Sie werden hergestellt, indem Verbindungen $Xb_2$ mit $R^5$ und E" = H in bekannter Weise durch Einwirkung von Phosphoroxidchlorid ($POCl_3$) in die entsprechenden 4-Chlorpyrimidine der Formel $Xb_1$ mit $R^3$ = Cl und E" = H umgewandelt und diese anschließend mit einer Verbindung der Formel XI (vgl. Formelschema I) umgesetzt werden. Die so erhaltenen Pyrimidine $Xb_1$ mit $R^3$ = O-R" und E" = H können, wie bereits erläutert, mit NBS in die entsprechenden 5-Brommethyl-pyrimidine übergeführt werden.

Die Verbindungen Xc mit E" = Cl und p = 0 sind zum Teil bekannt, solche, bei denen $R^7$-$R^9$ von den bekannten Verbindungen abweichende Bedeutungen haben, können in Analogie zu den beschriebenen hergestellt werden. Verbindungen der Formel Xc mit p = 1 können hergestellt werden, indem man Tolylamidine der Formel XII, bzw. deren Salze, nach bekannten Methoden mit entsprechend strukturierten 1,3-Diketonen oder 3-Ketosäureestern unter Bildung von 2-Tolylpyrimidinen der Formeln XIIIa oder XIIIb umsetzt und diese nach bekannten Methoden in das jeweils benötigte Pyrimidin-Derivat der Formel Xc, in dem $R^7$, $R^8$ und $R^9$ die angegebenen Bedeutungen haben und E" = H bedeutet, umwandelt (vgl. Formelschema II). Solche 2-(Tolyl)-pyrimidine der Formel Xc mit E" = H können nach bekannten Methoden, beispielsweise durch Bromierung mit NBS, in Verbindungen der

Formel Xc mit E" = Br oder Cl und p = 1 übergeführt werden (vgl. Formelschema II).

Formelschema II

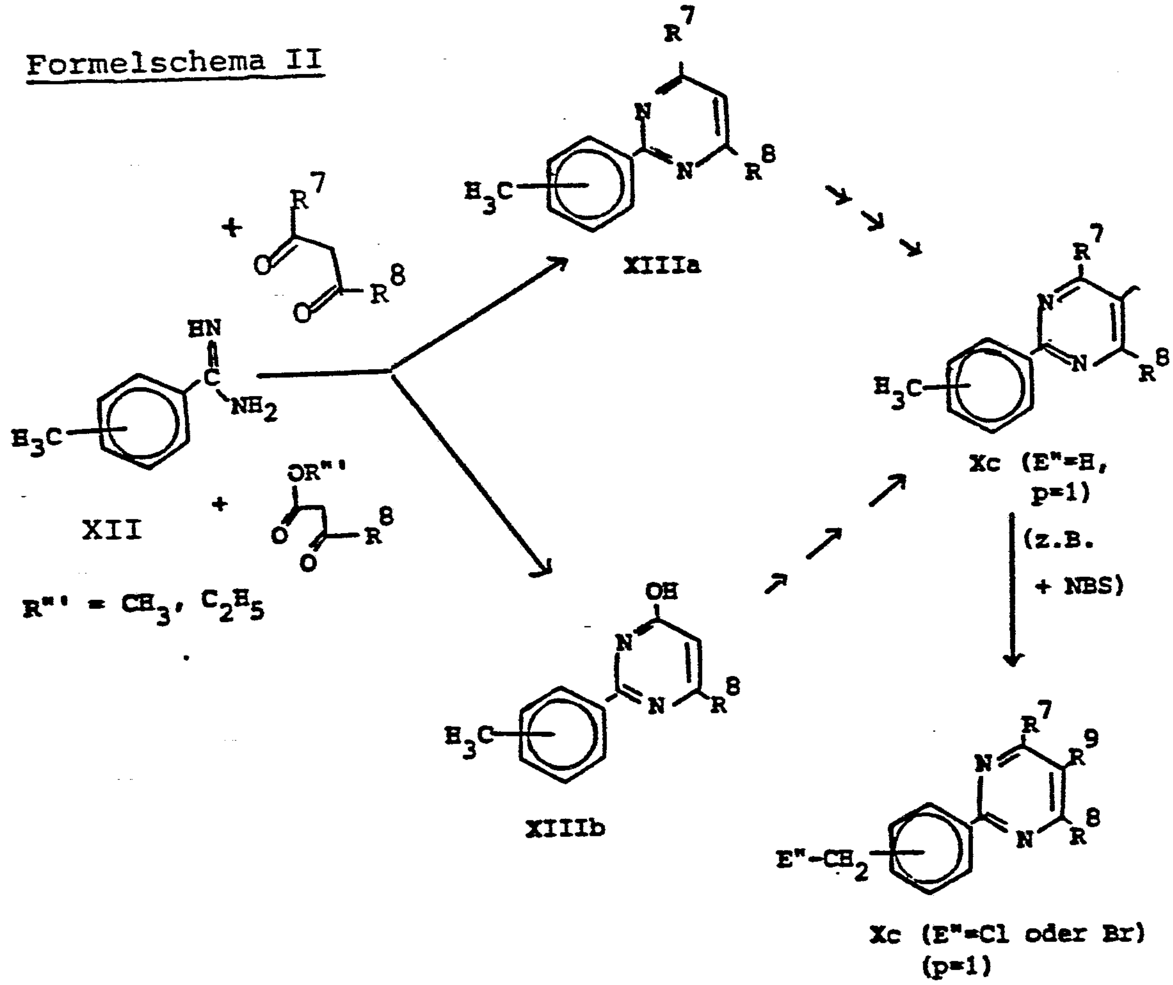


XII

R"' = $CH_3$, $C_2H_5$

XIIIa

XIIIb

Xc (E"=H, p=1)

Xc (E"=Cl oder Br) (p=1)

Verbindungen der Formeln $Xd_1$, $Xd_2$, $Xd_3$, $Xd_4$ und $Xd_5$, in denen E" = Cl oder Br, p = 0 und n = 0, 1 oder 2 bedeuten, sind bekannt, solche bei denen $R^{10}$ oder $R^{11}$ eine von den beschriebenen Verbindungen abweichende Bedeutung hat, können analog den bekannten hergestellt werden. Verbindungen der Formel Xd1 mit E" = H und p = 1 sind bekannt. Sie werden beispielsweise durch Einwirkung von NBS oder nach anderen bekannten Halogenierungsmethoden in solche, bei denen E" Cl oder Br bedeutet, übergeführt.
Verbindungen der Formeln $Xd_1$-$Xd_5$, bei denen E" = OH und p = 0 bedeutet, und $R^{10}$, n und $R^{11}$ die angegebenen Bedeutungen haben, sind bekannt und können nach bekannten Methoden, beispielsweise durch Einwirkung von Thionylchlorid, in entsprechende Verbindungen der Formel $Xd_1$-$Xd_5$ mit E" = Cl übergeführt werden. Verbindungen der Formel $Xd_4$, in denen E" H, n 1 oder 2 und $R^{11}$ Cl oder $OCH_3$ bedeuten, können nach den zur Synthese von Chinolinen bekannten Methoden hergestellt werden.
Die so erhaltenen Verbindungen der Formel $Xd_5$ mit E" = H werden durch Einwirkung von NBS in entsprechende Brom-methyl-chinoline der Formel $Xd_5$ (E" = Br), in denen $R^{11}$ und n die angegebenen Bedeutungen haben und n vorzugsweise 1 bedeutet, übergeführt (vgl. nachstehendes Formelschema III).

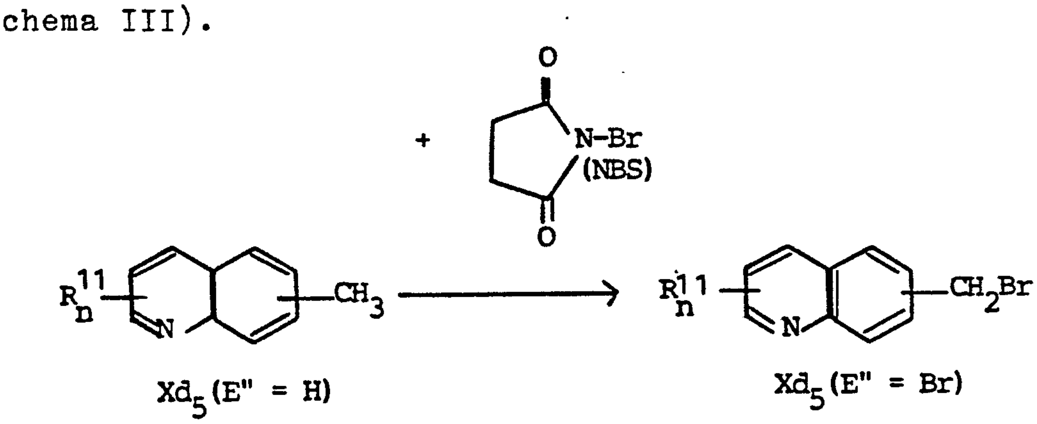

Die Reaktionszeiten betragen je nach Verfahrensvariante und je nach Temperaturbereich wenige Minuten bis einige Stunden.

Falls erforderlich, kann eine Reinigung der Verfahrenserzeugnisse durch Umkristallisation aus einem geeigneten Lösungsmittel oder Lösungsmittelgemisch oder durch Säulenchromatographie an Kieselgel erfolgen.

Die diastereomeren Racemate (cis- oder trans-Form) der Verbindungen der Formel I können in üblicher Weise, z.B. durch selektive, fraktionierte Kristallisation oder Säulenchromatographie getrennt werden.

Da die stereochemische Konfiguration bereits in den Zwischenprodukten der Formel II vorgegeben ist, kann die

Trennung in die cis- und trans-Form bereits auf dieser Stufe oder noch früher, beispielsweise auf der Stufe der Zwischenprodukte der allgemeinen Formel IV, oder bei den Zwischenprodukten der Formel IX erfolgen.

Die cis- und trans-diastereomeren Racemate können ihrerseits in üblicher Weise in ihre optischen Antipoden cis(+), cis(-) bzw. trans(+) und trans(-) getrennt werden.

Bevorzugt werden zur Herstellung von Verbindungen der Formel I die Verfahrens-Varianten A, B und D angewendet.

Die Erfindung betrifft weiterhin Verbindungen der Formel IIIa,

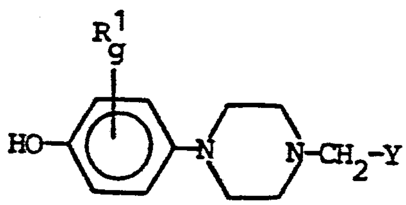

IIIa,

in der bedeuten:

$R^1$ $C_1$-$C_3$-Alkyl, F oder Cl,

g 0, 1 oder 2 und

Y die folgenden heterocyclischen Reste

$a_1$) 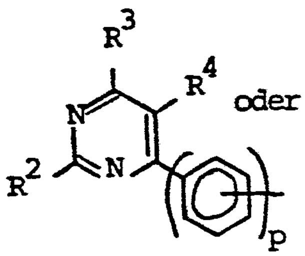 oder $a_2$) 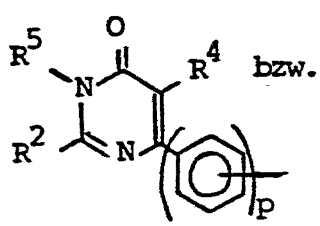 bzw. 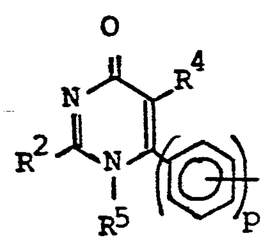

oder

$b_1$) 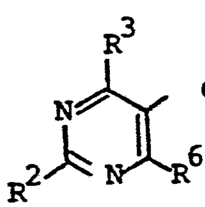 oder $b_2$) 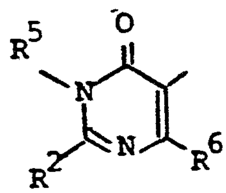 bzw. 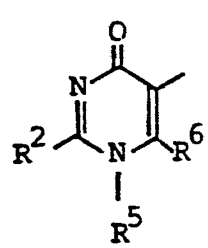

oder

c) 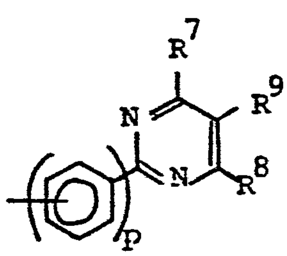

in denen

$R^2$ H, $C_1$-$C_8$-Alkyl, $C_1$-$C_4$-Alkoxy, eine unsubstituierte oder 1 oder 2 Substituenten tragende Phenylgruppe, wobei die Substituenten gleich oder verschieden sein können und F, Cl, Br, J, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Br, $OCH_3$, $OC_2H_5$, $CH_3$ oder $C_2H_5$ bedeuten, oder bei den heterocyclischen Resten $a_1$ und $a_2$, falls p Null bedeutet, darüberhinaus auch $C_1$-$C_4$-Alkylthio oder eine unsubstituierte oder eine im Phenylrest 1 oder 2 Substituenten tragende Benzylthiogruppe, wobei die Substituenten gleich oder verschieden sein können und F, Cl, $OCH_3$, $OC_2H_5$, $CH_3$ oder $C_2H_5$ bedeuten, bedeutet und

$R^3$ H, OH, Cl, $C_1$-$C_4$-Alkoxy oder eine unsubstituierte oder eine im Phenylrest 1 oder 2 Substituenten tragende Benzyloxygruppe, wobei die Substituenten gleich oder verschieden sein können und F, Cl, Br, $OCH_3$, $OC_2H_5$, $CH_3$ oder $C_2H_5$ bedeuten, und beim heterocyclischen Rest b1 zusätzlich $C_1$-$C_4$-Alkyl oder eine unsubstituierte oder eine 1 oder 2 Substituenten tragende Phenyloxygruppe, wobei die Substituenten gleich oder verschieden sein können und F, Cl, Br, $OCH_3$, $OC_2H_5$, $CH_3$ oder $C_2H_5$ bedeuten,

$R^4$ H, Cl, Br oder CN

$R^5$ H, $C_1$-$C_4$-Alkyl, Prop-2-en-1-yl, Prop-1-in-1-yl oder Benzyl

p 0 oder 1 bedeutet, wobei in $a_1$, $a_2$ und c der Phenylenrest über die 2,3 oder 4-Position gebunden ist und

$R^6$ H, $C_1$-$C_8$-Alkyl, ($C_3$-$C_6$-Cycloalkyl)-$C_1$-$C_3$-alkyl, eine unsubstituierte oder 1 oder 2 Substituenten tragende Phenylgruppe, wobei die Substituenten gleich oder

verschieden sein können und F, Cl, Br, J, $OCH_3$, $OC_2H_5$, 1.2--O-$CH_2$-O-, $CF_3$ oder $C_1$-$C_4$-Alkyl bedeuten, eine unsubstituierte oder im Phenylrest durch $OCH_3$, $OC_2H_5$, 1.2- O-$CH_2$-O-, $CF_3$, F, Cl oder $C_1$-$C_4$-Alkyl substituierte Phenyl-$C_1$-$C_2$-alkylgruppe, oder $CF_3$, und

$R^7$ und $R^8$ unabhängig voneinander H, $C_1$-$C_8$-Alkyl, $C_1$-$C_4$-Alkoxy oder eine unsubstituierte oder eine im Phenylrest 1 oder 2 Substituenten tragende Phenyl- oder Benzylgruppe, wobei die Substituenten gleich oder verschieden sein können und F, Cl, Br, $OCH_3$ oder $OC_2H_5$ bedeuten, und wobei $R^7$ darüberhinaus auch OH bedeuten kann, und

$R^9$ H, Cl, Br oder CN bedeutet, oder

$d_1$) 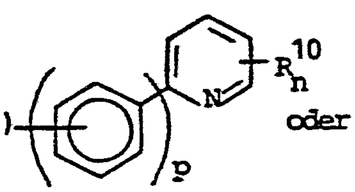 oder $d_2$) 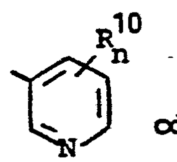 oder $d_3$) 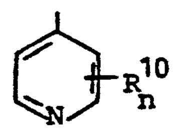 oder

$d_4$) 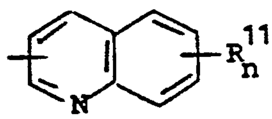 oder $d_5$) 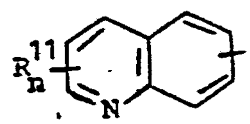 oder

$d_6$) 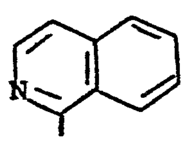 oder $d_7$) 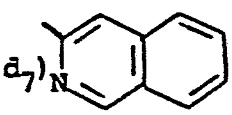

in denen

p 0 oder 1,

$R^{10}$ Cl, Br, $OCH_3$, $OC_2H_5$, $CH_3$ oder $C_2H_5$,

n 0, 1 oder 2 bedeutet, wobei $R^{10}$ in $d_1$ in 3,4,5 und/oder 6-, in $d_2$ in 2,4,5 und/oder 6- und in $d_3$ in 2,3, 5 und/oder 6-Stellung steht, und

$R^{11}$ Cl, $OCH_3$, $CH_3$ oder $C_2H_5$ bedeutet und in $d_4$ und $d_5$ in einer bzw. zwei der freien Positionen steht, und der heterocyclische Rest $d_4$ über die 2,3 oder 4-Stellung und der heterocyclische Rest $d_5$ über die 5,6,7 oder 8-Stellung und die Phenyleneinheit im Rest

$d_1$ über die 2,3 oder 4-(para)-Stellung gebunden ist, bedeutet,

sowie deren Säureadditionssalze.

Bevorzugt werden Verbindungen der Formel IIIa, bei denen mindestens einer der Substituenten oder Indizes $R^1$, g,Y, $R^2$-$R^{11}$, p und n die folgenden Bedeutungen hat:

$R^1$ $CH_3$ oder $C_2H_5$

g 0 oder 2

Y

zu den heterocyclischen Resten $a_1$, $a_2$, $b_1$, $b_2$ und c:

$R^2$ H, $C_1$-$C_4$-Alkyl, $CH_3O$ oder eine unsubstituierte oder eine im Phenylrest 1 oder 2 Substituenten tragende Phenyl- oder eine Benzylgruppe, wobei die Substituenten gleich oder verschieden sein können und F, Cl, Br oder $OCH_3$ bedeuten,

$R^3$ H oder $C_1$-$C_4$-Alkoxy und beim Rest $b_1$ zusätzlich eine unsubstituierte oder eine 1 oder 2 Substituenten tragende Phenyloxygruppe, wobei die Substituenten gleich oder verschieden sein können und F, Cl, Br, $OCH_3$ oder $OC_2H_5$ bedeuten,

$R^4$ H oder CN

$R^5$ $CH_3$

p 0 oder 1

$R^6$ H, $C_1$-$C_8$-Alkyl, ($C_5$-$C_6$-Cycloalkyl)-$C_1$-$C_2$-alkyl, eine unsubstituierte oder 1 oder 2 Substituenten tragende Phenylgruppe, wobei die Substituenten gleich oder verschieden sein können und F, Cl, Br, $OCH_3$, $OC_2H_5$ oder $CF_3$ bedeuten, eine unsubstituierte oder im Phenylrest durch $OCH_3$, $CF_3$, F oder Cl substituierte Benzylgruppe oder $CF_3$,

$R^7$, $R^8$ unabhängig voneinander $C_1$-$C_8$-Alkyl, $C_1$-$C_4$-Alkoxy oder eine unsubstituierte oder eine im Phenylrest durch F, Cl, $OCH_3$ oder $OC_2H_5$ substituierte Phenyl- oder Benzyl-

gruppe und $R^7$ darüber hinaus auch H oder OH

$R^9$ H oder CN

zu den heterocyclischen Resten $d_1$, $d_2$, $d_3$, $d_4$, $d_5$, $d_6$ und $d_7$:

p 0 oder 1,

$R^{10}$ Cl, $CH_3$ oder $C_2H_5$,

n 0 oder 1, wobei $R^{10}$ in $d_3$ bevorzugt in 2-Stellung steht, und

$R^{11}$ Cl, wobei die Phenyleneinheit im Rest $d_1$ bevorzugt über die 4-Stellung gebunden ist.

Besonders bevorzugt sind Verbindungen der Formel III a, in denen mindestens einer der Substituenten bzw. Indizes die folgenden Bedeutungen hat:

$R^1$ $CH_3$

g 0 oder 2

Y

zu den heterocyclischen Resten $a_1$, $a_2$, $b_1$, $b_2$ und c:

$R^2$ eine unsubstituierte oder eine im Phenylrest 1 oder 2 Substituenten tragende Phenyl- oder Benzylgruppe, wobei die Substituenten gleich oder verschieden sein können und F, Cl oder $OCH_3$ bedeuten, und beim Rest $b_1$ zusätzlich $C_1$-$C_4$-Alkyl

$R^3$ H und beim Rest $b_1$ zusätzlich eine unsubstituierte oder durch F, Cl, $OCH_3$ oder $OC_2H_5$ substituierte Phenyloxygruppe

$R^4$ H

$R^5$ $CH_3$

p bei den heterocyclischen Resten $a_1$ und $a_2$ 0 oder 1 und bei dem heterocyclischen Rest c 1

$R^6$ $C_1$-$C_8$-Alkyl, ($C_5$-$C_6$-Cycloalkyl)-$C_1$-$C_2$-alkyl, eine unsubstituierte oder durch F, Cl, $OCH_3$ oder $OC_2H_5$ substituierte Phenylgruppe, eine unsubstituierte

oder im Phenylrest durch F, Cl oder $OCH_3$ substituierte Benzylgruppe oder $CF_3$

$R^7$, $R^8$ unabhängig voneinander $C_1$-$C_3$-Alkyl oder eine unsubstituierte oder eine im Phenylrest durch F, Cl oder $OCH_3$ substituierte Phenyl- oder Benzylgruppe und $R^7$ darüber hinaus auch H oder $C_1$-$C_4$-Alkoxy

$R^9$ H

zu den heterocyclischen Resten $d_1$, $d_2$, $d_3$, $d_4$, $d_5$, $d_6$ und $d_7$:

p 1,

n 0,

wobei der Rest $d_4$ bevorzugt über die 2-Stellung und der Rest $d_5$ bevorzugt über die 6- oder 7-Stellung gebunden ist.

Die Verbindungen der Formel IIIa, in denen $R^1$, g und Y die angegebenen Bedeutungen haben, sind neu und stellen wertvolle Zwischenprodukte zur Herstellung der stark antimykotisch sowie fungizid wirkenden Verbindungen der Formel I dar. Ein Teil der Verbindungen der Formel IIIa wirkt ebenfalls antimykotisch sowie fungizid. Ein Teil der Verbindungen der Formel IIIa zeigt darüberhinaus pharmakologische Wirkungen, so z.B. Wirkungen auf das Herz-Kreislaufsystem.

Die Erfindung betrifft außerdem ein Verfahren zur Herstellung von Verbindungen der Formel IIIa, das dadurch gekennzeichnet ist, daß man eine Verbindung der Formel XIV,

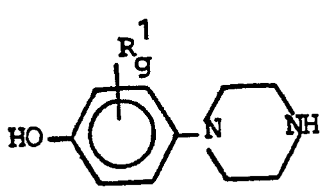

XIV,

in der $R^1$ und g die zu Formel I angegebenen Bedeutungen

0237962

haben oder ein Salz dieser Verbindung, mit einer Verbindung der Formel X,

$$E''-CH_2-Y \qquad X,$$

in der E" Cl, Br, J, Acyloxy, wie Acetoxy oder Benzoyloxy, Alkylsulfonyloxy, wie Methansulfonyloxy, oder Arylsulfonyloxy, wie Benzol-, Nitrobenzol- oder Toluolsulfonyloxy, bedeutet, und

Y die zur Formel I unter $a_1$, $a_2$, $b_1$, $b_2$, c, $d_1$, $d_2$, $d_3$, $d_4$, $d_5$, $d_6$ und $d_7$ angegebenen Bedeutungen hat,

umsetzt und gegebenenfalls die erhaltenen Verbindungen der Formel IIIa mit anorganischen oder organischen Säuren in ihre Säureadditionssalze überführt.

Bevorzugt verwendet werden für die erfindungsgemäße Umsetzung mit Verbindungen der Formel XIV entweder

a) eine Verbindung der Formel $Xa_1$ oder der Formel $Xa_2$,

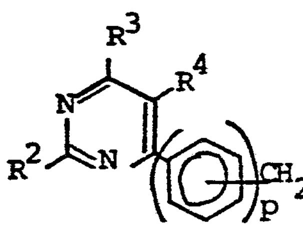 $Xa_1$, 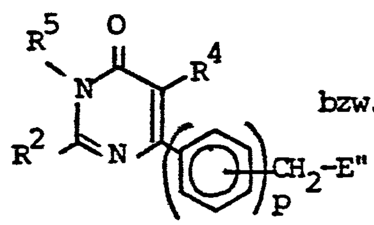 bzw. 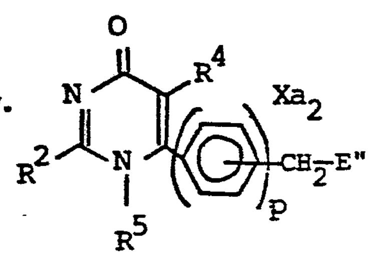 $Xa_2$

in denen $R^2$-$R^5$ die zu Formel I angegebenen Bedeutungen haben und E" Cl oder Br bedeutet, oder

b) eine Verbindung der Formel $Xb_1$ oder der Formel $Xb_2$

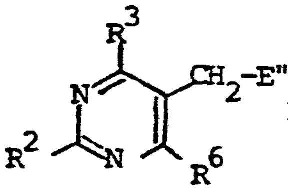 $Xb_1$, 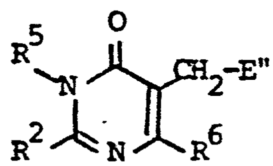 bzw. 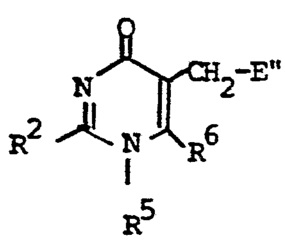 $Xb_2$

in denen $R^2$, $R^3$, $R^5$ und $R^6$ die zu Formel I angegebenen Bedeutungen haben und E" Br bedeutet, oder

c) eine Verbindung der Formel Xc,

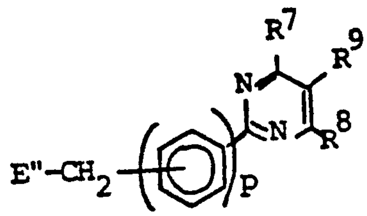

Xc,

in der $R^7$, $R^8$, $R^9$ und p die zu Formel I angegebenen Bedeutungen haben und

E" Cl oder Br bedeutet, oder

d) eine Verbindung der Formel $Xd_1$, der Formel $Xd_2$, der Formel $Xd_3$, der Formel $Xd_4$, der Formel $Xd_5$, der Formel $Xd_6$ oder der Formel $Xd_7$

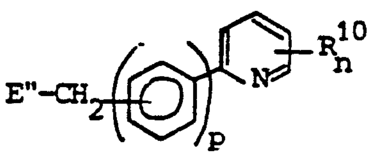

$Xd_1$,

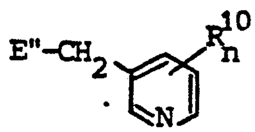

$Xd_2$,

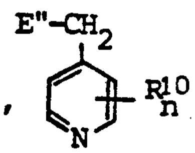

$Xd_3$

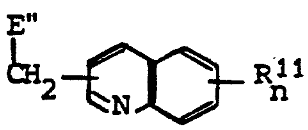

$Xd_4$,

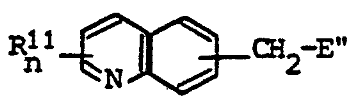

$Xd_5$

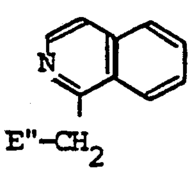

$Xd_6$

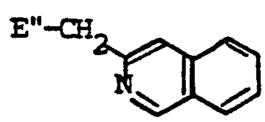

$Xd_7$

in denen $R^{10}$, $R^{11}$, p und n die zu Formel I angegebenen Bedeutungen haben und

E" Cl oder Br bedeutet.

Das erfindungsgemäße Verfahren zur Herstellung von Verbindungen der Formel IIIa wird zweckmäßig in einem inerten organischen Lösungsmittel in einem Temperaturbereich von 20° bis 180°C, vorzugsweise von 50° bis 120°C, vorteilhaft in Gegenwart einer Base, die vorzugsweise in äquivalenter Menge angewandt wird, durchgeführt. Werden für das Verfahren Salze der Verbindungen der Formel XIV verwandt, dann wird die dem Salzanteil entsprechende stöchiometrische Menge an Base zugesetzt. Wahlweise kann darüber hinaus dann ein weiterer Anteil an Base angewandt werden.
Die Synthese von Verbindungen der Formel IIIa aus den Verbindungen der Formeln XIV und X kann, sofern die Verbindungen XIV nicht als Salz angewandt werden, auch ohne Basenzusatz ausgeführt werden. Die Reaktanten der Formeln XIV und X können in unterschiedlichen Molverhältnissen angewandt werden, d.h. es können entweder jeweils die Verbindungen der Formel XIV oder diejenigen der Formel X im Überschuß angewandt werden, vorteilhaft werden äquimolare Mengen angewandt.

Als Lösungsmittel kommen beispielsweise Kohlenwasserstoffe, allgemein Ether, wie Diethylether, 1,2-Dimethoxyethan, Tetrahydrofuran oder Dioxan, oder Acetonitril, Butyronitril, Dimethylform- oder -acetamid, Aceton, 4-Methyl-2-pentanon, Methylenchlorid, Chloroform, Dimethylsulfoxid, Anisol, Chlorbenzol, Tetrachlorethen oder Gemische dieser Lösungsmittel in Frage.

Geeignete Basen sind die beispielhaft bei der Verfahrens-Variante A) erläuterten.

Die Reaktionszeiten betragen je nach Temperaturbereich wenige Minuten bis einige Stunden.

Falls erforderlich, kann eine Reinigung der Verfahrenserzeugnisse durch Umkristallisation aus einem geeigneten Lösungsmittel oder Lösungsmittelgemisch oder durch Säulenchromatographie an Kieselgel erfolgen.

Herstellung der Ausgangsstoffe:

Die Verbindung der Formel XIV mit g=0 ist bekannt. Verbindungen der Formel XIV, bei denen g 1 oder 2 bedeutet und $R^1$ die zu Formel I angegebenen Bedeutungen hat, werden in Analogie zu den bekannten Verbindungen durch Umsetzung von entsprechenden 4-Methoxy-Anilinen mit Bis-(2-Chlorethyl)-amin und anschließender Phenolether-Spaltung mit konz. wäßriger Bromwasserstoffsäure hergestellt. Die Herstellung der Verbindungen der Formel X, in der E" und Y die angegebenen Bedeutungen haben, sofern sie nicht bekannt sind, ist bereits bei Verfahrensvariante D) zur Herstellung von Verbindungen der Formel I beschrieben.

Die Verbindungen der Formel I und ihre Säureadditionssalze sind wertvolle Arzneimittel. Sie wirken antimikrobiell und eignen sich insbesondere zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen zweckmäßig in einer Konzentration von etwa 0,01 bis 99,0, vorzugsweise von etwa 0,05 bis 50 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,05 bis etwa 200, vorzugsweise 0,1 bis 100, insbesondere 0,5 bis 30 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8,, vorzugsweise in 1 bis 3 Einzeldosen verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen der Formel I eignen sich auch zur Behandlung von Protozoenerkrankungen beim Menschen und Tier wie sie z. B. durch Infektionen mit Trichomonas vaginalis und Entamoeba histolytica sowie durch Trypanosoma cruzi, Leishmania donovani hervorgerufen werden.

Die neuen Verbindungen können oral oder lokal angewendet werden. Die orale Anwendung erfolgt in pharmazeutisch üblichen Zubereitungen, z. B. in Form von Tabletten oder Kapseln.

Die Verbindungen der Formel I sind auch als Biozide wirksam. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die neuen Verbindungen der Formel I eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Die nachfolgenden Beispiele dienen zur näheren Erläuterung der Erfindung, ohne dieselbe einzuschränken.

Beispiele für das Herstellungsverfahren Variante A)

Beispiel 1

Zu einer Lösung von 2,26 g (6 m Mol) 4-(4-(4-Hydroxyphenyl)-piperazin-1-ylmethyl)-6-methoxy-2-phenyl-pyrimidin in 20 ml abs. N.N-Dimethylformamid (DMF) wurden 0,184 g (6,13 m Mol) 80 %ige Natriumhydrid-Öl-Dispersion zugesetzt. Nach Abklingen der Wasserstoffentwicklung wurde bei Zimmertemperatur eine Lösung von 2,49 g (6,1 m Mol) 2-S(R)-(2.4-Dichlorphenyl)-2-(1.2.4-triazol-1-ylmethyl)-4-R,(S)-methansulfonyloxymethyl-1.3-dioxolan(cis-Form) in 16 ml abs. DMF zugegeben und die Mischung 4 Std. bei 100°C gerührt. Anschließend destillierte man das DMF unter Vakuum (3-10 mbar) an einem Rotationsverdampfer ab, versetzte den Rückstand mit 50 ml Wasser und 50 ml $CH_2Cl_2$, schüttelte durch, trennte die Phasen und extrahierte die wäßrige Phase noch dreimal mit $CH_2Cl_2$. Die vereinigten $CH_2Cl_2$-Extrakte wurden mit $MgSO_4$ getrocknet, filtriert und im Vakuum eingedampft. Den Rückstand (5,5 g) löste man in 12 ml Methanol, woraufhin kristalliner Niederschlag ausfiel. Dieser wurde isoliert (3,02 g) und aus einer 2:3-Mischung von $CH_3CN$ und $CH_2Cl_2$ umkristallisiert, indem man nach dem Auflösen bei Siedetemperatur einen Großteil des $CH_2Cl_2$ bei Normaldruck abdampfte. Beim Abkühlen kristallisierte reines 2-S,(R)-(2.4-Dichlorphenyl)-2-(1.2.4-triazol-1-ylmethyl)-4-R,(S)-[4-(4-(4-methoxy-2-phenyl-pyrimidin-6-ylmethyl)-piperazin-1-yl)-phenoxymethyl]-1.3-dioxolan(cis-Form) aus. Nach dem Absaugen, Waschen mit $CH_3CN$ und Trocknen erhielt man 2,75 g (66,6 % Ausbeute) dieser Verbindung; Fp. 146-47°C, Elementaranalyse: $C_{35}H_{35}Cl_2N_7O_4$ (MG 688,64) Ber. C 61,05, H 5,12, Cl 10,30, N 14,24; Gef. C 60,8, H 5,2, Cl 10,3, N 14,3 %; das $^1$H-NMR-Spektrum ($CDCl_3$) bestätigt die Struktur.

## Beispiel 2

Gemäß Beispiel 1, jeweils unter Verwendung der Methansulfonate IIb bzw. IIc (vgl. Tabelle 1) und der entsprechenden Verbindungen der Formel IIIa (vgl. Tabelle 1) wurden die folgenden, in der Tabelle 1 aufgeführten Verbindungen der Formel I (Ar: 2.4-Dichlorphenyl, cis-Form) hergestellt.

Tabelle 1

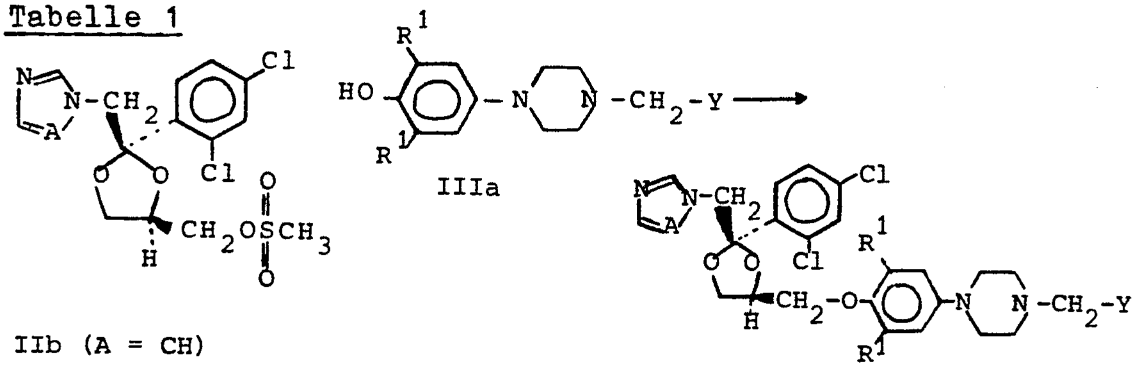

IIIa

IIb (A = CH)

IIc (A = N)

I (Ar = 2.4-Dichlorphenyl)

| Verb. Nr. | A | $R^1$ | Y | Analyse Ber. | % Gef. | Fp. [°C] |
|---|---|---|---|---|---|---|
| 1,1 | CH | H | 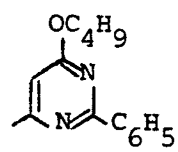 | C 64,19 | 64,0 | - |
| | | | | H 5,80 | 6,0 | |
| | | | | N 11,52 | 11,4 | |
| 1,2 | N | H | " | C 62,46 | 62,3 | 153-4 |
| | | | | H 5,66 | 5,7 | |
| | | | | N 13,42 | 13,3 | |
| 1,3 | CH | H | 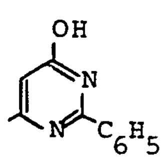 | C 62,41 | 61,2 | 125-6 |
| | | | | H 5,09 | 4,9 | |
| | | | | N 12,48 | 12,0 | |
| 1,4 | N | H | " | C 60,53 | 60,3 | 163-4 |
| | | | | H 4,93 | 4,9 | |
| | | | | N 14,55 | 14,1 | |

| Verb. Nr. | A | $R^1$ | Y | Analyse Ber. | % Gef. | Fp. [°C] |
|---|---|---|---|---|---|---|
| 1,5 | CH | H | 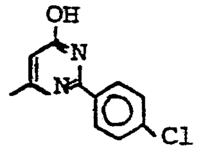 | C 59,37 | 58,9 | 153-4 |
| | | | | H 4,70 | 4,6 | |
| | | | | N 11,87 | 11,6 | |
| 1,6 | CH | H | 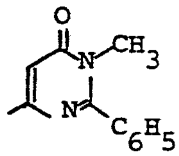 | C 62,88 | 62,3 | 154-5 |
| | | | | H 5,28 | 5,3 | |
| | | | | N 12,22 | 12,0 | |
| 1,7 | N | H | " | C 61,05 | 60,5 | 151-2 |
| | | | | H 5,12 | 5,2 | |
| | | | | N 14,24 | 14,0 | |
| 1,8 | CH | H | 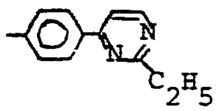 | C 64,81 | 64,1 | - |
| | | | | H 5,59 | 5,7 | |
| | | | | N 12,26 | 11,6 | |
| 1,9 | CH | $CH_3$ | 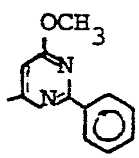 | C 63,77 | 63,4 | |
| | | | | H 5,63 | 5,5 | - |
| | | | | N 11,74 | 11,6 | |
| 1,10 | CH | H | 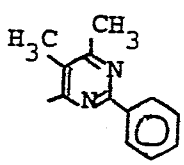 | C 64,81 | 64,6 | 134-35 |
| | | | | H 5,59 | 5,6 | |
| | | | | N 12,26 | 12,3 | |
| 1,11 | CH | H | 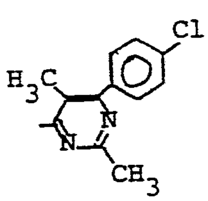 | C 61,71 | 61,2 | |
| | | | | H 5,18 | 5,3 | |
| | | | | N 11,67 | 11,3 | |

| Verb. Nr. | A | $R^1$ | Y | Analyse Ber. | % Gef. | Fp. [°C] |
|---|---|---|---|---|---|---|
| 1,12 | CH | H | 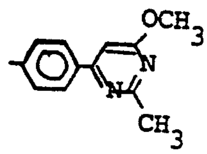 | C 63,34 | 62,8 | - |
| | | | | H 5,46 | 5,3 | |
| | | | | N 11,98 | 11,4 | |
| 1,13 | CH | $CH_3$ | 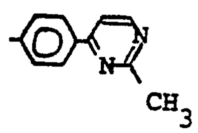 | C 65,23 | 64,5 | - |
| | | | | H 5,76 | 5,8 | |
| | | | | N 12,01 | 12,1 | |

Beispiel 3

Zu einer Lösung von 1,73 g (5 m Mol) 4-(4-Hydroxyphenyl)-1-(4-(2-pyridyl)-benzyl)-piperazin in 20 ml abs. DMF gab man bei Zimmertemperatur 0,58 g (5.15 m Mol) Kalium-tert.-butylat, rührte 8 Min., gab dann eine Lösung von 2,04 g (5 m Mol) IIb (vgl. Beisp. 2, Tab. 1) in 15 ml abs. 1.2-Dimethoxyethan (DME) zu und rührte die Mischung 5 Stdn. bei 91-92°C. Anschließend wurden die Lösungsmittel im Vakuum abgezogen, der Rückstand in Wasser/$CH_2Cl_2$ aufgenommen und nach Durchmischung die Phasen getrennt. Die wässrige Phase wurde noch zweimal mit $CH_2Cl_2$ extrahiert. Die vereinigten $CH_2Cl_2$-Auszüge wurden getrocknet, filtriert und eingedampft. Der verbliebene Rückstand (2,9 g) kristallisierte aus Methanol. Nach dem Absaugen, Waschen mit eiskaltem Methanol und Trocknen erhielt man 2,31 g (≙ 70,4 % Ausbeute) reines Cis-2-(2.4-Dichlorphenyl)-2-(imidazol-1-ylmethyl)-4-[4-(4-(4-(2-pyridyl)-benzyl)-piperazin-1-yl)-phenoxymethyl]-1.3-dioxolan, Fp. 89-90°C; Analyse: $C_{36}H_{35}Cl_2N_5O_3$ (MG 656,63) Ber. C 65,85 H 5,37 N 10,67; Gef. C 65,7 H 5,3 N. 10,5.

Beispiel 4

Nach der gleichen Arbeitsweise wie im Beispiel 3 beschrieben, ebenfalls im 5 m Mol-Maßstab, wurde bei Verwendung

des gleichen Piperazin-Derivats als Zwischenprodukt und bei Anwendung von IIb (vgl. Beisp. 2, Tab. 1) und bei Anwendung von 0,203 g (5,2 m Mol) Natriumamid, anstelle von Kalium-tert.-butylat als Base, bei sonst gleichen Reaktionsbedingungen wie im Beispiel 3 beschrieben, Cis-2-(2.4-Dichlorphenyl)-2-(imidazol-1-ylmethyl)-4-[4-(4-(4-(2-pyridyl)-benzyl)-piperazin-1-yl)-phenoxymethyl]-1.3-dioxolan, 2,19 g (≙ 66,8 % Ausbeute), Fp. 88-90°C, erhalten.

Beispiel 5

Zu einer Lösung von 1,79 g (4,7 m Mol) 5-(4-(4-Hydroxyphenyl)-piperazin-1-ylmethyl)-2-(4-chlorphenyl)-pyrimidin in 19 ml abs. DMF wurden bei Zimmertemperatur 0,145 g (4,83 m Mol) 80 %ige NaH-Öl-Dispersion zugesetzt. Nach Abklingen der Wasserstoffentwicklung wurde bei Zimmertemperatur eine Lösung von 1,974 g (4,85 m Mol) IIb (vgl. Beisp. 2, Tab. 1) in 15 ml abs. DMF zugegeben und die Mischung anschließend 2 Stdn. bei 102-104°C gerührt. Danach wurde wie im Beispiel 1 beschrieben aufgearbeitet. Der $CH_2Cl_2$-Extrakt-Rückstand (3,02 g) kristallisierte aus Methanol. Nach Absaugen, Auswaschen mit Methanol und Trocknen erhielt man 2,73 g (≙ 84 % Ausbeute) reines 2-S,(R)-(2.4-Dichlorphenyl)-2-(imidazol-1-ylmethyl)-4-R,(S)-[4-(4-(2-(4-chlorphenyl)-pyrimidin-5-ylmethyl)-piperazin-1-yl)-phenoxymethyl]-1.3-dioxolan(cis-Form); Fp. 153-54°C, Analyse: $C_{35}H_{33}Cl_3N_6O_3$ (MG 692,07) Ber. C 60,74, H 4,81 N 12,14; Gef. C 60,5 H 4,6 N 12,1 %; das $^1$H-NMR Spektrum ($CDCl_3$) bestätigt die Struktur.

Bei im Prinzip gleicher Arbeitsweise wie vorstehend beschrieben können anstelle von Dimethylformamid auch Dimethylsulfoxid, N,N-Dimethylacetamid oder N-Methylpyrrolidon-(2), sowie Gemische dieser Lösungsmittel mit z.B.

Tetrahydrofuran, Dioxan, 1,2-Dimethoxyethan oder Acetonitril verwendet werden.

Beispiel 6

Gemäß Beispiel 5, jeweils unter Verwendung der Methansulfonate IIb bzw. IIc (vgl. Tabelle 1) und der entsprechenden Verbindungen der Formel IIIb (Y vgl. Tabelle 2) wurden die folgenden in der Tabelle 2 aufgeführten Verbindungen der Formel I (Ar: 2.4-Dichlorphenyl, cis-Form) hergestellt.
Sofern nach der Aufarbeitung die Verbindungen der Formel I nicht durch Kristallisation bzw. Umkristallisieren zu reinigen waren, wurde der $CH_2Cl_2$-Extrakt-Rückstand jeweils an einer Kieselgel S (Riedel-de Haen, Korngröße 0,063-0,2 mm)-$CH_2Cl_2$-Säule chromatographiert. Man eluierte fraktionsweise mit $CH_2Cl_2$ und weiter mit $CH_2Cl_2/C_2H_5OH$-Mischungen sukzessiv steigenden $C_2H_5OH$-Gehalts (bis max. 5 Vol % $C_2H_5OH$) und überprüfte die Fraktionen dünnschichtchromatographisch (DC) (DC-Fertigplatten, Kieselgel 60, F 254, Merck). In der Tabelle 2 mit (*) angezeigte Verbindungen wurden auf diese Weise durch Säulenchromatographie rein erhalten.

Tabelle 2

IIb bzw. IIc +

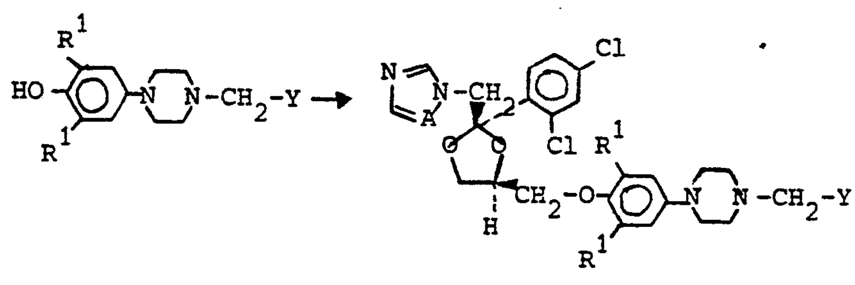

IIIb          Ia (Ar = 2.4-Dichlorphenyl)

| Verb. Nr. | A | $R^1$ | Y | Analyse Ber. | % Gef. | Fp. [°C] |
|---|---|---|---|---|---|---|
| 2,1* | CH | H | 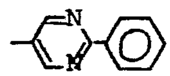 | C 63,92 | 63,0 | 120-1 |
| | | | | H 5,21 | 5,3 | |
| | | | | Cl 10,78 | 10,7 | |
| | | | | N 12,78 | 12,4 | |
| 2,2 | N | H | " | C 62,01 | 61,8 | 118-9 |
| | | | | H 5,05 | 5,0 | |
| | | | | Cl 10,77 | 10,8 | |
| 2,3 | N | H | 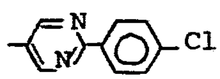 | C 58,92 | 58,5 | 79-80 |
| | | | | H 4,65 | 4,7 | |
| | | | | N 14,15 | 14,1 | |
| 2,4* | N | H | 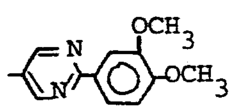 | C 60,17 | 59,9 | |
| | | | | H 5,19 | 5,2 | - |
| | | | | N 13,64 | 13,3 | |
| 2,5 | CH | H | 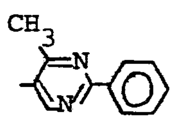 | C 64,38 | 64,3 | 160-1 |
| | | | | H 5,40 | 5,3 | |
| | | | | N 12,51 | 12,5 | |
| 2,6 | N | H | " | C 62,50 | 62,4 | 154-5 |
| | | | | H 5,25 | 5,2 | |
| | | | | N 14,58 | 14,2 | |
| 2,7* | CH | H | 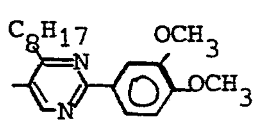 | C 65,13 | 64,3 | |
| | | | | H 6,56 | 6,4 | - |
| | | | | N 10,13 | 10,1 | |
| 2,8* | CH | $CH_3$ | 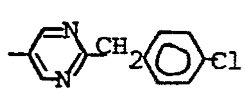 | C 62,17 | 61,7 | |
| | | | | H 5,35 | 5,2 | - |
| | | | | N 11,45 | 11,2 | |

| Verb. Nr. | A | $R^1$ | Y | Analyse Ber. | % Gef. | Fp. [°C] |
|---|---|---|---|---|---|---|
| 2,9 | CH | H | 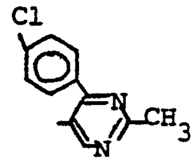 | C 61,24 | 59,7 | 120-21 |
| | | | | H 5,00 | 5,2 | |
| | | | | N 11,90 | 11,5 | |
| 2,10* | CH | H | 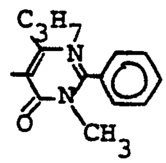 | C 64,19 | 64,1 | - |
| | | | | H 5,80 | 5,6 | |
| | | | | N 11,52 | 11,2 | |
| 2,11* | N | H | " | C 62,47 | 62,0 | - |
| | | | | H 5,64 | 5,4 | |
| | | | | N 13,42 | 13,0 | |
| 2,12* | CH | $CH_3$ | 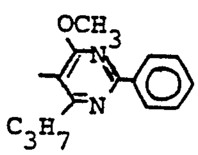 | C 64,99 | 64,4 | |
| | | | | H 6,12 | 6,0 | - |
| | | | | N 11,09 | 10,8 | |
| 2,13 | CH | $CH_3$ | 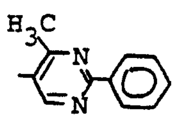 | C 65,23 | 64,8 | |
| | | | | H 5,76 | 5,8 | - |
| | | | | N 12,01 | 11,6 | |
| 2,14 | CH | H | 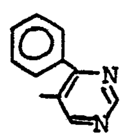 | C 63,92 | 63,5 | |
| | | | | H 5,21 | 5,0 | - |
| | | | | N 12,78 | 12,4 | |
| 2,15* | CH | H | 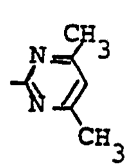 | C 61,08 | 60,1 | |
| | | | | H 5,62 | 5,3 | - |
| | | | | N 13,77 | 13,3 | |
| 2,16* | CH | H | 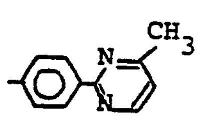 | C 64,38 | 64,1 | |
| | | | | H 5,40 | 5,5 | - |
| | | | | N 12,51 | 12,1 | |

Tabelle 2, Fortsetzung

| Verb. Nr. | A | $R^1$ | Y | Analyse Ber. | % Gef. | Fp. [°C] |
|---|---|---|---|---|---|---|
| 2,17* | CH | H | 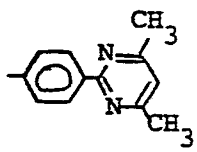 | C 64,81 | 65,0 | |
| | | | | H 5,59 | 5,5 | - |
| | | | | N 12,26 | 12,1 | |
| 2,18 | N | H | " | C 62,97 | 62,7 | 149-50 |
| | | | | H 5,43 | 5,2 | |
| | | | | N 14,28 | 14,2 | |
| 2,19* | CH | H | 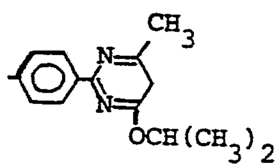 | C 64,19 | 64,0 | |
| | | | | H 5,80 | 5,8 | - |
| | | | | N 11,52 | 11,5 | |
| 2,20 | N | H | 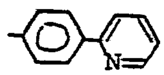 | C 63,92 | 63,8 | 126-27 |
| | | | | H 5,21 | 5,2 | |
| | | | | N 12,78 | 12,8 | |
| 2,21 | CH | H | 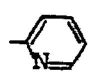 | C 62,07 | 61,3 | 133-34 |
| | | | | H 5,38 | 5,4 | |
| | | | | N 12,06 | 11,7 | |
| 2,22 | N | H | " | C 59,90 | 59,5 | 129-30 |
| | | | | H 5,20 | 5,3 | |
| | | | | N 14,45 | 14.1 | |
| 2,23 | N | H | 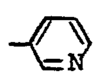 | C 59,90 | 59,8 | 106-07° |
| | | | | H 5,20 | 5,3 | |
| | | | | N 14,45 | 14,4 | |
| 2,24 | CH | $CH_3$ | 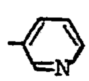 | C 63,15 | 63,0 | - |
| | | | | H 5,80 | 5,8 | |
| | | | | N 11,51 | 11,4 | |

Tabelle 2, Fortsetzung

| Verb. Nr. | A | $R^1$ | Y | Analyse | Ber. | % Gef. | Fp. [°C] |
|---|---|---|---|---|---|---|---|
| 2,25 | CH | H | 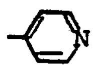 | C | 62,07 | 62,2 | |
| | | | | H | 5,38 | 5,1 | |
| | | | | N | 12,06 | 11,8 | |
| 2,26 | N | $CH_3$ | 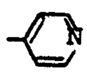 | C | 61,08 | 60,4 | 112-13 |
| | | | | H | 5,62 | 5,5 | |
| | | | | N | 13,79 | 13,5 | |
| 2,27 | CH | H | 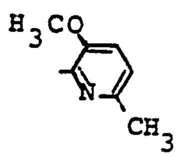 | C | 61,54 | 61,1 | 138-39 |
| | | | | H | 5,65 | 5,3 | |
| | | | | N | 11,35 | 11,0 | |
| 2,28 | CH | H | 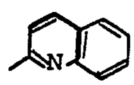 | C | 64,76 | 64,0 | - |
| | | | | H | 5,28 | 5,3 | |
| | | | | N | 11,11 | 10,6 | |
| 2,29 | N | H | " | C | 62,76 | 61,9 | 160-61 |
| | | | | H | 5,11 | 5,1 | |
| | | | | N | 13,31 | 13,0 | |
| 2,30 | CH | H | 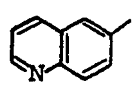 | C | 64,76 | 64,1 | - |
| | | | | H | 5,28 | 5,2 | |
| | | | | N | 11,11 | 10,5 | |
| 2,31 | CH | H | 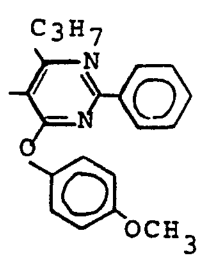 | C | 65,77 | 65,1 | - |
| | | | | H | 5,64 | 5,4 | |
| | | | | N | 10,23 | 9,8 | |

Beispiel 7

Eine Mischung aus : 1,37 g (3,8 m Mol) 4-(4-Hydroxyphenyl)-1-(4-(4-methyl-pyrimidin-2-yl)-benzyl)-piperazin, 35 ml Toluol, 5,5 ml 50 %ige Natronlauge, 1,55 g (3,8 m Mol) IIb (vgl. Tab. 1) und 0,29 g Tetrabutylammoniumbromid wurde 3 Stdn. bei 100°C intensiv gerührt. Danch wurden die Phasen getrennt und die konz. Natronlauge zweimal mit Ether ausgewaschen. Die vereinigten organischen Phasen wurden zweimal mit Wasser ausgewaschen, getrocknet, filtriert und i. Vak. eingedampft. Der Extraktrückstand (2,35 g) wurde wie im Beispiel 6 beschrieben an einer Kieselgel S (Riedel-de Haen, Korngröße 0,063-0,2 mm)-$CH_2Cl_2$-Säule (Ø 2,1 cm, Höhe 28 cm) chromatographiert. Die DC-einheitlichen, reinen Fraktionen wurden zusammengefaßt und im Vakuum eingedampft. Man erhielt 2,17 g (≙ 85,1 % Ausbeute) reines Cis-2-(2.4-Dichlorphenyl)-2-(imidazol-1-ylmethyl)-4-[4-(4-(4-(4-methyl-pyrimidin-2-yl)-benzyl)-piperazin-1-yl)-phenoxymethyl]-1.3-dioxolan als hochviskoses Öl; Analyse: $C_{36}H_{36}Cl_2N_6O_3$ (MG 671,65) Ber. C 64,38, H 5,40, Cl 10,56, N 12,51; Gef. C 64,2, H 5,5, Cl 10,7, N 12,3 %.

Beispiel 8

Nach der gleichen Arbeitsweise wie im Beispiel 7 beschrieben wurden ausgehend von IIb bzw. IIc (vgl. Tabelle 1) und jeweils der entsprechenden Verbindung IIIb (Y vgl. Tabelle 3) die in der Tabelle 3 aufgeführten Verbindungen der Formel I (g = 0 oder 2, $R^1$ H oder $CH_3$ in 2.6-Stellung) hergestellt.

Tabelle 3

IIb bzw. IIc + 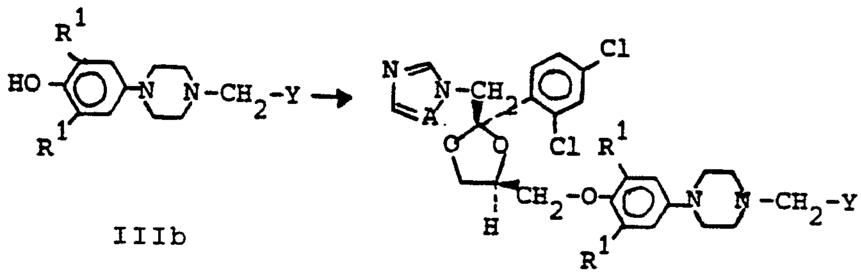

IIIb

| Verb. Nr. | A | $R^1$ | Y | Analyse Ber. | | % Gef. |
|---|---|---|---|---|---|---|
| 3,1 | CH | H | 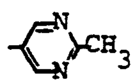 | C<br>H<br>N | 60,50<br>5,42<br>14,11 | 60,1<br>5,3<br>14,0 |
| 3,2 | N | $CH_3$ | 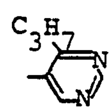 | C<br>H<br>N | 60,73<br>6,02<br>15,02 | 60,5<br>6,0<br>14,8 |
| 3,3 | CH | $CH_3$ | 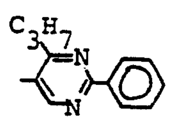 | C<br>H<br>N | 66,02<br>6,09<br>11,55 | 66,2<br>5,9<br>11,1 |
| 3,4 | CH | $CH_3$ | 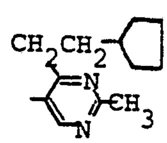 | C<br>H<br>N | 65,08<br>6,72<br>11,68 | 64,7<br>6,6<br>11,8 |
| 3,5 | N | $CH_3$ | 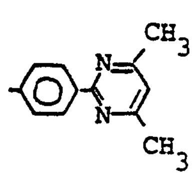 | C<br>H<br>N | 63,36<br>5,78<br>13,72 | 63,7<br>5,8<br>13,5 |
| 3,6 | CH | H | 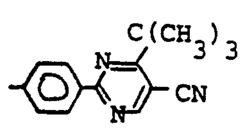 | C<br>H<br>N | 66,29<br>5,70<br>11,60 | 66,0<br>5,7<br>11,4 |

Tabelle 3, Fortsetzung

| Verb. Nr. | A | $R^1$ | Y | Analyse | Ber. | % Gef. |
|---|---|---|---|---|---|---|
| 3,7 | CH | H | 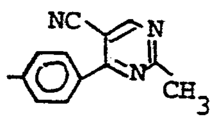 | C | 63,79 | 63,4 |
| | | | | H | 5,06 | 4,9 |
| | | | | N | 14,08 | 13,7 |
| 3,8 | N | H | 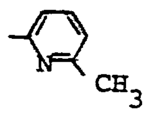 | C | 60,50 | 60,1 |
| | | | | H | 5,42 | 5,3 |
| | | | | N | 14,11 | 14,2 |
| 3,9 | CH | H | 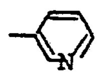 | C | 62,07 | 61,5 |
| | | | | H | 5,38 | 5,3 |
| | | | | N | 12,06 | 11,6 |
| 3,10 | N | $CH_3$ | 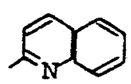 | C | 65,11 | 64,8 |
| | | | | H | 5,62 | 5,4 |
| | | | | N | 10,85 | 10,8 |
| 3,11 | CH | H | 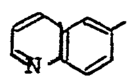 | C | 64,76 | 64,0 |
| | | | | H | 5,28 | 5,1 |
| | | | | N | 11,11 | 10,7 |
| 3,12 | CH | $CH_3$ | " | C | 65,65 | 65,3 |
| | | | | H | 5,66 | 5,5 |
| | | | | N | 10,63 | 10,3 |

Beispiel 9 (Salzbildung)

Eine Lösung von 580 mg (0,864 m Mol) 2-S,(R)-(2.4-Dichlorphenyl)-2-(imidazol-1-ylmethyl)-4-R,(S)-[4-(4-(4-methyl-2-phenyl-pyrimidin-5-ylmethyl)-piperazin-1-yl)-phenoxymethyl]-1.3-dioxolan (cis-Form) (vgl. Beispiel 6, Nr. 2,5) in 20 ml $CH_2Cl_2$ wurde mit 0,29 ml einer 6 m Lösung von HCl in Ether versetzt, woraufhin kristalliner Niederschlag ausfiel. Die Mischung wurde im Vakuum eingedampft, der verbliebene kristalline Rückstand mit 10 ml Ethylacetat aufgekocht und nach Abkühlung auf ca. 7°C abgesaugt und getrocknet. Man erhielt 620 mg (≙ 96 % Ausbeute an Dihydrochlorid) Cis-2-(2.4-Dichlorphenyl)-2-(imidazol-1-ylmethyl)-4-[4-(4-(4-methyl-2-phenyl-pyrimidin-5-ylmethyl)-piperazin-1-yl)-phenoxymethyl]-1.3-dioxolan-dihydrochlorid, Fp. 180-81°C, Analyse: $C_{36}H_{38}Cl_4N_6O_3$ (MG 744,58), Ber. Cl (gesamt) 19,04, $CL^{\ominus}$ 9,52, Gef. Cl (gesamt) 18,8, $Cl^{\ominus}$ 9,6%.

Beispiele für das Herstellungsverfahren Variante B)

Beispiel 10

a) Zu einer Lösung von 7,21 g (20 m Mol) 5-(4-(4-Hydroxyphenyl)-piperazin-1-ylmethyl)-4-methyl-2-phenyl-pyrimidin in 70 ml abs. N.N-Dimethylformamid (DMF) gab man bei Zimmertemperatur (unter Kühlung) 0,60 g 80 %ige Natriumhydrid-Öl-Dispersion. Nach Ende der Wasserstoffentwicklung wurde bei RT eine Lösung von 8,40 g (20 m Mol) Cis-2-Brommethyl-2-(2.4-dichlorphenyl)-4-methansulfonyloxymethyl-1.3-dioxolan (cis und trans bezieht sich auf die Brommethyl- und Methansulfonyloxymethyl-Gruppe in der 2- bzw. 4-Stellung des Dioxolanringes) in 25 ml abs. DMF zugetropft und die Mischung 8 Stdn. bei 60-63°C gerührt. Die anschließende Aufarbeitung und Säulenchromatographie erfolgte in der gleichen Weise wie in den Beispielen 1 und 6 beschrieben. Die chromatographische Reinigung wurde an einer Kieselgel S-$CH_2Cl_2$/Petrolether 3:1-Säule (Ø 3,6 cm, H = 37 cm) unter Elution mit $CH_2Cl_2$/Petrolether 3:1, $CH_2Cl_2$ und $CH_2Cl_2$/$C_2H_5OH$-Mischungen (steigenden $C_2H_5OH$-Gehalts, bis maximal 3 Vol. %) durchgeführt. Man erhielt 8,76 g (64,0 % d.Th.) Cis-2-Brommethyl-2-(2.4-dichlorphenyl)-4-[4-(4-(4-methyl-2-phenyl-pyrimidin-5-ylmethyl)-piperazin-1-yl)-phenoxymethyl]-1.3-dioxolan als hochviskoses Öl, Analyse: $C_{33}H_{33}BrCl_2N_4O_3$ (684,49) Ber. C 57,91, H 4,86, Br 11,68, Cl 10,36, N 8,19; Gef. C 57,3, H 4,6, Br 11,9, Cl 10,0, N 7,8 %.

b) Zu einer Lösung von 0,85 g (12,5 m Mol) Imidazol in 25 ml abs. N.N-Dimethylacetamid gab man 0,375 g (12,5 m Mol) 80 %ige NaH-Öl-Dispersion. Nach Ende der Wasserstoffentwicklung wurde eine Lösung von 6,85 g (10 m Mol) der unter a) hergestellten Brommethyl-Verbindung in 10 ml abs. N.N-Dimethylacetamid zugetropft und anschließend 24 Stdn. am Rückfluß gekocht. Danach wurde wie im Beispiel 1 beschrieben aufgearbeitet.

Das Dimethylacetamid wurde im Ölpumpenvakuum am Rotationsverdampfer abdestilliert. Der $CH_2Cl_2$-Extrakt-Rückstand (6,8 g) wurde an einer Kieselgel S-$CH_2Cl_2$-Säule (Ø 2,6 cm, H 40 cm) wie im Beispiel 6 beschrieben chromatographiert. Dabei wurden 2,55 g hochangereicherte Substanz erhalten, die aus Methanol kristallisiert 2,18 g (≙ 32,5 % Ausbeute) reines Cis-2-(2.4-Dichlorphenyl)-2-(imidazol-1-ylmethyl)-4-[4-(4-(4-methyl-2-phenyl-pyrimidin-5-ylmethyl)-piperazin-1-yl)-phenoxymethyl]-1.3-dioxolan; Fp. 160-61°C; Analyse: Gef. C 64,2, H 5,3, N 12,3 % (Berechnete Werte vgl. Beispiel 6 Nr. 2,5) ergaben.

Beispiel 11

Nach der gleichen Arbeitsweise wie im Beispiel 10a beschrieben wurden, ausgehend von 15,5 m Mol 4-(4-Hydroxyphenyl)-1-(4-(4-methyl-pyrimidin-2-yl)-benzyl)-piperazin, der entsprechenden Menge NaH und 15,5 m Mol Cis-2-Brommethyl-2-(2.4-dichlorphenyl)-4-methansulfonyloxymethyl-1.3-dioxolan, 6,92 g (≙ 65,2 % d.Th.) Cis-2-Brommethyl-2-(2.4-dichlorphenyl)-4-[4-(4-(4-(4-methyl-pyrimidin-2-yl)-benzyl)-piperazin-1-yl)-phenoxymethyl]-1.3-dioxolan als zähes Öl erhalten; Analyse: $C_{33}H_{33}BrCl_2N_4O_3$ (684,49) Ber. C 57,91, H 4,86, Br 11,68, Cl 10,36, N 8,19; Gef. C 56,8, H 4,6, Br 12,0, Cl 10,2, N 7,7 %.

Beispiel 12

Zu einer Suspension von 0,49 g (16,3 m Mol) 80 %iger NaH-Öl-Dispersion in 15 ml abs. Dimethylsulfoxid (DMSO) tropfte man bei Zimmertemperatur eine Lösung von 1,023 g (14,83 m Mol) 1.2.4-Triazol in 6 ml abs. DMSO, rührte 30 Min. bei RT nach und gab anschließend eine Lösung von 6,85 g (10 m Mol) gemäß Beispiel 11 hergestelltes Cis-2-Brommethyl-2-(2.4-dichlorphenyl)-4-[4-(4-(4-(4-methylpyrimidin-2-yl)-benzyl)-piperazin-1-yl)-phenoxymethyl]-1.3-dioxolan in 7 ml

abs. DMSO tropfenweise zu und rührte 20 Stdn. bei 130°C unter Stickstoffatmosphäre. Nach Abkühlung wurde die Reaktionsmischung in 120 ml Wasser eingerührt. Diese Mischung extrahierte man mehrmals mit $CH_2Cl_2$. Die vereinigten $CH_2Cl_2$-Extrakte wurden getrocknet, filtriert und im Vak. eingedampft. Der Rückstand (7,1 g) wurde wie im Beispiel 6 beschrieben an einer Kieselgel S-$CH_2Cl_2$-Säule (Ø 2,6 cm, H 42 cm) durch Chromatographie gereinigt. Aus den laut DC einheitlichen Fraktionen wurden 2,43 g (≙ 36,2 % Ausbeute) nahezu reines Cis-2-(2.4-Dichlorphenyl)-2-(1.2.4-triazol-1-ylmethyl)-4-[4-(4-(4-(4-methyl-pyrimidin-2-yl)-benzyl)-piperazin-1-yl)-phenoxymethyl]-1.3-dioxolan, Fp. 126-27°C, erhalten; Analyse: $C_{35}H_{35}Cl_2N_7O_3$ (672,64) Ber. C 62,50, H 5,25, N 14,58; Gef. C 62,5 H 5,2 N 14,2 %.

Beispiel 13

a) Eine Mischung von 2,16 g (6 m Mol) 4-(4-Hydroxyphenyl)-1-(4-methyl-2-phenyl-pyrimidin-5-ylmethyl)-piperazin, 60 ml Toloul 2,22 g (6 m Mol) Cis-2-Brommethyl-2-(4-fluorphenyl)-4-methansulfonyloxymethyl-1.3-dioxolan, 0,40 g Tetrabutylammoniumbromid und 8 ml 50 %ige Natronlauge wurde 3,75 Stdn. bei 56°C intensiv gerührt. Anschließend trennte man bei Raumtemperatur die Phasen, schüttelte die Natronlauge dreimal mit Ether aus und vereinigte Toluol- und Etherphasen. Diese wurden dreimal mit Wasser ausgeschüttelt, getrocknet, filtriert und im Vakuum eingedampft. Der verbliebene Rückstand (5,0 g) wurde an einer Kieselgel S/$CH_2Cl_2$-Säule (Ø 2,1 cm, H 30 cm) unter Elution mit $CH_2Cl_2$ und $CH_2Cl_2/C_2H_5OH$-Mischungen (100:0.25-100:1 v/v) chromatographiert. Nach Elution einer Vorzone (0.45 g) wurden die DC-einheitlichen Fraktionen vereinigt und i. Vak. eingedampft. Man erhielt 3,40 g (≙ 89,5 % Ausbeute) Cis-2-Brommethyl-2-(4-fluorphenyl)-4-[4-(4-(4-methyl-2-phenyl-pyrimidin-5-ylmethyl)-piperazin-1-yl)-phenoxymethyl]-1.3-

dioxolan als hochviskoses Öl ("Cis" bezieht sich auf die 2-Brommethylgruppe und die "4-ständige" Phenoxymethyl-Gruppe); Analyse: $C_{33}H_{34}BrFN_4O_3$ (MG 633,58) Ber. C 62,56, H 5,41, Br 12,61, F 3,00, N 8,84; Gef. C 62,1, H 5,3, Br 15,0, F 2,9, N 8,8%.

b) Wie im Beispiel 10b beschrieben wurden 3,40 g (5,36 m Mol) Cis-2-Brommethyl-2-(4-fluorphenyl)-4-[4-(4-(4-methyl-2-phenyl-pyrimidin-5-ylmethyl)-piperazin-1-yl)-phenoxymethyl]-1.3-dioxolan mit 0,714 g (10,5 m Mol) Imidazol und 0,315 g (10,5 m Mol) 80 %iger NaH-Öl-Dispersion in 33 ml abs. N.N-Dimethylacetamid umgesetzt. Nach 30 Stdn. Rückflußkochen wurde das Dimethylacetamid im Ölpumpenvakuum am Rotationsverdampfer abdestilliert. Der Rückstand wurde in $CH_2Cl_2$/Wasser aufgenommen. Nach Trennung der Phasen wurde die wässrige mehrmals mit $CH_2Cl_2$ extrahiert. Die vereinigten $CH_2Cl_2$-Extrakte wurden mit Wasser ausgeschüttelt, getrocknet, filtriert und i. Vak. eingedampft. Den Rückstand (3,96 g) chromatographierte man an einer KieselgelS/$CH_2Cl_2$-Säule (Ø 2,1 cm, Höhe 34 cm) unter Elution mit $CH_2Cl_2$ und $CH_2Cl_2/C_2H_5OH$-Mischungen (mit steigendem $C_2H_5OH$-Gehalt, bis maximal 5 Vol % $C_2H_5OH$). Nach Elution von 2,5 g 2-Brommethyl-dioxolan-Ausgangs-Verbindung wurden DC-einheitliche Fraktionen vereinigt und i. Vak. eingedampft. Diese ergaben als Rückstand 0,51 g nahezu reines Cis-2-(4-Fluorphenyl)-2-(imidazol-1-ylmethyl)-4-[4-(4-(4-methyl-2-phenyl-pyrimidin-5-ylmethyl)-piperazin-1-yl)-phenoxymethyl]-1.3-dioxolan (Ausbeute ≙ 15,3 %),

Analyse: $C_{36}H_{37}FN_6O_3$ (MG 620,74) Ber. C 69,66 H 6,01 F 3,06 N 13,54;

Gef. C 68,6, H 6,0, F 2,9, N 13,2%.

Beispiel 14

Eine Mischung von 3,77 g (14 m Mol) 4-(4-Hydroxyphenyl)-1-(pyrid-2-ylmethyl)-piperazin, 200 ml Toluol, 5,88 g (14 m Mol) 2-Brommethyl-2-(2.4-dichlorphenyl)-4-methansulfonyloxymethyl-1.3-dioxolan, 0,80 g Tetrabutylammoniumbromid und 27 ml 50 %ige Natronlauge wurde 3 Stdn. bei 65°C intensiv gerührt. Anschließend wurden bei Zimmertemperatur die Phasen getrennt. Die Toluollösung wurde dreimal mit Wasser ausgeschüttelt, getrocknet, filtriert und im Vak. eingedampft. Der verbliebene Rückstand (8,8 g) wurde an einer KieselgelS/$CH_2Cl_2$-Säule (⌀ 2,6 cm, H 28,0 cm) unter Elution mit $CH_2Cl_2$ und $CH_2Cl_2/C_2H_5OH$-Mischungen (bis maximal 4 Vol. % $C_2H_5OH$-Gehalt) chromatographiert. Nach Elution einer Vorzone (1,45 g) wurden die DC-einheitlichen Fraktionen vereinigt und i. Vak. eingedampft. Man erhielt 6,02 g (≙ 72,4 % Ausbeute) 2-Brommethyl-2-(2.4-dichlorphenyl)-4-[4-(4-(pyrid-2-ylmethyl)-piperazin-1-yl)-phenoxymethyl]-1.3-dioxolan (cis/trans-Mischung) als hochviskoses Öl, Analyse: $C_{27}H_{28}BrCl_2N_3O_3$ (MG 593,38)
Ber.C 54,65, H 4,76, Br 13,47, Cl 11,95, N 7,08; Gef. C 53,5, H 4,9, Br 13,1, Cl 13,2, N 6,5%.

Beispiel 15

Wie im Beispiel 10b beschrieben wurden 4,75 g (8 m Mol) 2-Brommethyl-2-(2.4-dichlorphenyl)-4-[4-(4-(pyrid-2-ylmethyl)-piperazin-1-yl)-phenoxymethyl]-1.3-dioxolan mit 0,68 g (10 m Mol) Imidazol und 0,30 g (10 m Mol) 80 %iger NaH-Öl-Dispersion in 28 ml abs. N.N-Dimethylacetamid unter Bildung von 1,53 g (≙ 33 % d.Th.) 2-(2.4-Dichlorphenyl)-2-(imidazol-1-ylmethyl)-4-[4-(4-(pyrid-2-ylmethyl)-piperazin-1-yl)-phenoxymethyl]-1.3-dioxolan;
Fp. 134-35°C, Analyse: Gef.C 61,7 H 5,2 N 12,0%
(Berechnete Werte vgl. Beispiel 6, Nr. 2,21) umgesetzt.

Beispiele für das Herstellungsverfahren Variante D)

Beispiel 16

Eine Lösung von 2,45 g (5 m Mol) 2-S,(R)-(2.4-Dichlorphenyl)-2-(imidazol-1-ylmethyl)-4-R,(S)-(4-piperazinophenoxymethyl)-1.3-dioxolan(cis-Form) und 1,87 g (5 m Mol) 2-(4-Brommethyl-1-phenyl)-4.6-dimethyl-pyrimidin in 20 ml abs. DMF wurde auf 80°C unter Schutz gegen Luftfeuchtigkeit erwärmt und nach 10 Min. mit 115 mg pulverisiertem Kaliumkarbonat unter Rühren versetzt. Nach weiteren 25 Min. gab man weitere 115 mg pulverisiertes $K_2CO_3$ und nach weiteren 60 Min. eine 3. Portion von 115 mg ebensolchen $K_2CO_3$ zu (insgesamt 345 mg (2,5 m Mol) $K_2CO_3$). Anschliessend rührte man 3 Stdn. bei 80°C nach, destillierte das DMF im Ölpumpenvakuum am Rotationsverdampfer ab und nahm den Rückstand in 30 ml Wasser und 100 ml $CH_2Cl_2$ auf. Nach Trennung der Phasen wurde die wässrige noch zweimal mit $CH_2Cl_2$ extrahiert. Die vereinigten $CH_2Cl_2$-Extrakte wurden getrocknet, fitriert und i. Vak. eingedampft. Der Rückstand (3,72 g) wurde durch Säulenchromatographie an KieselgelS/$CH_2Cl_2$ wie im Beispiel 6 beschrieben gereinigt. Man erhielt 2,71 g (79 % d.Th.) reines Cis-2-(2.4-Dichlorphenyl)-2-(imidazol-1-ylmethyl)-4-[4-(4-(4-(4.6-dimethyl-pyrimidin-2-yl)-benzyl)-piperazin-1-yl)-phenoxymethyl]-1.3-dioxolan als zähes Öl; Analyse: Gef.C 64,6, H 5,5, N 12,0 % (vgl. Beispiel 6, Nr. 2,17).

Beispiel 17

Nach der gleichen Arbeitsweise wie im Beispiel 16 beschrieben, ausgehend von IXa oder IXb (vgl. Tabelle 4) und jeweils der entsprechenden Verbindung der Formel Xa, wurden die in der Tabelle 4 aufgeführten Verbindungen der Formel I nach Verfahrensvariante D) hergestellt.

Tabelle 4

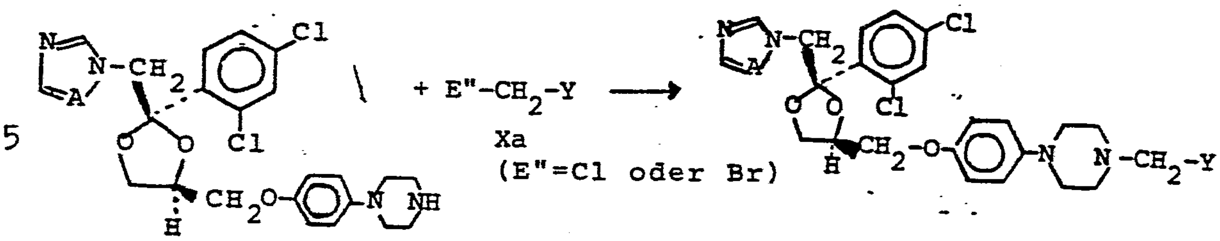


IXa (A = CH)
IXb (A = N)
cis

cis I (Ar = 2.4-$Cl_2$-$C_6H_3$-, g = O)

| Verb. Nr. | A | Y | Analyse Ber. | % Gef. | Fp. [°C] |
|---|---|---|---|---|---|
| 4,1 | CH | 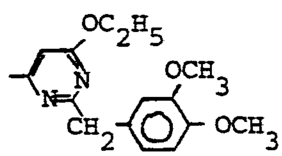 | C 61,93<br>H 5,72<br>N 10,83 | 61,0<br>5,6<br>10,3 | 129-30 |
| 4,2 | N | 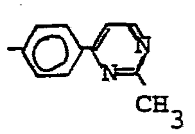 | C 62,50<br>H 5,25<br>N 14,58 | 62,4<br>5,3<br>14,2 | - |
| 4,3 | CH | 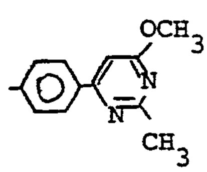 | C 63,34<br>H 5,46<br>N 11,98 | 62,0<br>5,4<br>11,4 | - |
| 4,4 | CH | 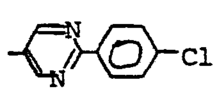 | C 60,74<br>H 4,81<br>N 12,14 | 60,5<br>4,7<br>11,9 | 153-54 |
| 4,5 | N | 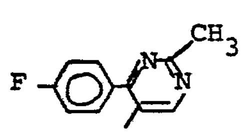 | C 60,87<br>H 4,96<br>N 14,20 | 60,5<br>4,8<br>13,9 | - |

Tabelle 4, Fortsetzung

| Verb. Nr. | A | Y | Analyse Ber. | % Gef. | Fp. [°C] |
|---|---|---|---|---|---|
| 4,6 | CH | 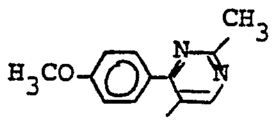 | C 63,34 | 63,0 | 105-06 |
| | | | H 5,46 | 5,2 | |
| | | | N 11,98 | 12,1 | |
| 4,7 | N | 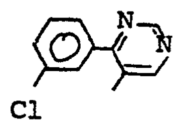 | C 58,92 | 58,7 | - |
| | | | H 4,65 | 4,7 | |
| | | | N 14,15 | 14,0 | |
| 4,8 | CH | 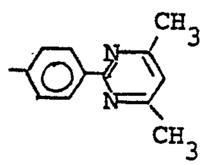 | C 64,81 | 64,5 | - |
| | | | H 5,59 | 5,3 | |
| | | | N 12,26 | 12,2 | |
| 4,9 | CH | 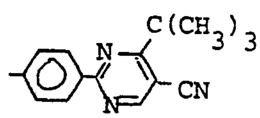 | C 66,29 | 65,9 | 142-43 |
| | | | H 5,70 | 5,5 | |
| | | | N 11,60 | 11,5 | |
| 4,10 | CH | 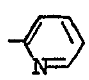 | C 62,07 | 62,1 | 133-34 |
| | | | H 5,38 | 5,1 | |
| | | | N 12,06 | 11,9 | |
| 4,11 | CH | 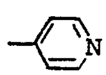 | C 62,07 | 61,7 | |
| | | | H 5,38 | 5,2 | |
| | | | N 12,06 | 11,8 | |
| 4,12 | N | " | C 59,90 | 59,6 | 110-11 |
| | | | H 5,20 | 4,9 | |
| | | | N 14,45 | 14,5 | |
| 4,13 | CH | 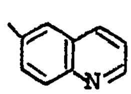 | C 64,76 | 64,5 | - |
| | | | H 5,28 | 5,0 | |
| | | | N 11,11 | 10,8 | |

Beispiel 18

Nach im Prinzip gleicher Arbeitsweise wie im Beispiel 16 beschrieben, ausgehend von 5 m Mol IXb und 5 m Mol 3-Chlormethyl-pyridin-hydrochlorid, jedoch in 25 ml abs. Dimethylsulfoxid (DMSO), anstelle von DMF als Lösungsmittel, und unter Verwendung von zusätzlichen 2,5 m Mol pulverisiertem Kaliumkarbonat (somit insgesamt 5 m Mol $K_2CO_3$) wurden, nach entsprechender Aufarbeitung der Reaktionsmischung wie im Beispiel 16 beschrieben, 1,80 g (62 % d.Th.) Cis-2-(2.4-Dichlorphenyl)-2-(1.2.4-triazol-1-ylmethyl)-4-[4-(4-(pyrid-3-ylmethyl)-piperazin-1-yl)-phenoxymethyl]-1.3-dioxolan, Fp. 106-107°C, erhalten; Analyse: Gef.C 59,6, H 5,0, N 14,3 % (vgl. Beispiel 6, Nr. 2,23).

Beispiele für die Herstellung von Verbindungen der Formel IIIa

Beispiel 19

Eine Mischung von 4,94 g (14,5 m Mol) 1-(4-Hydroxyphenyl)-piperazin-dihydrobromid, 3,29 g (14 m Mol) 4-Chlormethyl-6-methoxy-2-phenyl-pyrimidin und 2,00 g (14,5 m Mol) pulverisiertem Kaliumkarbonat in 50 ml abs. N.N-Dimethylformamid (DMF) wurde auf 80°C erwärmt und unter Rühren nach 10 Min., nach weiteren 25 Min. und nach weiteren 60 Min. mit jeweils 322 mg pulverisiertem $K_2CO_3$ unter Stickstoff-Überlagerung versetzt (insgesamt Zusatz von 966 mg (7 m Mol) $K_2CO_3$). Man rührte 2,5 Stdn. bei 80°C nach, destillierte im Ölpumpenvakuum am Rotationsverdampfer das DMF weitgehend ab und vermischte den kristallinen Rückstand mit 40 ml Wasser und 50 ml $CH_2Cl_2$, stellte mit 2 n HCl auf pH 5-6 ein, trennte die Phasen und extrahierte die wässrige dreimal mit $CH_2Cl_2$. Die vereinigten $CH_2Cl_2$-Extrakte wurden getrocknet, filtriert und im Vakuum eingedampft. Der Rückstand (4,25 g) wurde an einer KieselgelS-$CH_2Cl_2$-Säule (ø 2,0, Höhe 23 cm) durch Elution mit

$CH_2Cl_2$ und $CH_2Cl_2/C_2H_5OH$-Mischungen, steigenden $C_2H_5OH$-Gehalts (bis maximal 4 Vol %-$C_2H_5OH$) chromatographiert. Die DC-einheitlichen Fraktionen wurden vereinigt und aus $CH_3OH$ kristallisiert. Man erhielt auf diese Weise 3,06 g (≙ 58 % Ausbeute) 4-(4-Hydroxyphenyl)-1-(6-methoxy-2-phenyl-pyrimidin-4-ylmethyl)-piperazin; Fp. 166-67°C, Analyse: $C_{22}H_{24}N_4O_2$ (MG 376,46) Ber.C 70,19, H 6,43, N 14,88; Gef. C 69,9, H 6,4, N 14,5%.

Beispiel 20

Eine Mischung von 4,46 g (25 m Mol) 1-(4-Hydroxyphenyl)-piperazin und 5,52 g (25 m Mol) 6-Chlormethyl-2-phenyl-pyrimidon-(4) in 90 ml abs. N.N-Dimethylformamid (DMF) wurde auf 80°C erwärmt und unter Rühren nach 10 Min., nach weiteren 25 Min. und nach weiteren 60 Min. mit jeweils 576 mg pulverisiertem $K_2CO_3$ unter Stickstoff-Atmosphäre versetzt (insgesamt Zusatz von 1,73 g (12,5 m Mol) $K_2CO_3$) und 3,5 Stdn. bei 80°C nachgerührt. Danach wurde im Vakuum am Rotationsverdampfer das DMF weitgehend abdestilliert, der Rückstand mit 170 ml Wasser und 17 ml 4 nHCl versetzt und die entstandene Mischung dreimal mit $CH_2Cl_2$ extrahiert. Der $CH_2Cl_2$-Extrakt wurde verworfen. Die saure wässrige Lösung wurde mit Aktivkohle versetzt, durchgeschüttelt und abgesaugt. Das Filtrat neutralisierte man mit 2 nNaOH. Der dabei ausfallende kristalline Niederschlag wurde abgesaugt, mit Wasser nachgewaschen, mit Methanol 5 Min. aufgekocht und nach Abkühlung auf 10-15°C abgesaugt und getrocknet. Man erhielt 6,25 g (≙ 69 % Ausbeute) reines 4-(4-Hydroxyphenyl)-1-(2-phenyl-3.4-dihydro-pyrimidin-4-on-6-ylmethyl)-piperazin-hydrat, Fp. 246-48°C, Analyse: $C_{21}H_{24}N_4O_3$ (MG 380,45) Ber.C 66,30 H 6,36 N 14,73; Gef.C 65,9 H 6,1 N 14,5 %.

Beispiel 21

Nach der gleichen Arbeitsweise wie im Beispiel 20 beschrieben, wurden ausgehend von 3,39 g (19 m Mol) 1-(4-Hydroxyphenyl)-piperazin und 4,20 g (17,9 m Mol)6-Chlormethyl-3.4-dihydro-3-methyl-2-phenyl-pyrimidin-4-on, unter Verwendung von 1,24 g (9 m Mol) $K_2CO_3$, 4,42 g (≙ 65,6 % Ausbeute) 4-(4-Hydroxyphenyl)-1-(3.4-dihydro-3-methyl-2-phenyl-pyrimidin-4-on-6-ylmethyl)-piperazin; Fp. 206-07°C, Analyse; $C_{22}H_{24}N_4O_2$ (MG 376,46) Ber.C 70,19, H 6,43, N 14,88; Gef.C 69,2, H 6,4, N 14,5 %; erhalten. Zur Aufarbeitung der Reaktionsmischung war das DMF i. Vak. abgedampft, der Rückstand in Wasser/$CH_2Cl_2$ aufgenommen und neutralisiert worden und der $CH_2Cl_2$-Extrakt-Rückstand aus $CH_3OH$ kristallisiert worden.

Beispiel 22

Eine Mischung von 2,59 g (14,5 m Mol) 1-(4-Hydroxyphenyl)-piperazin und 3,90 g (14 m Mol) 2-(4-Brommethyl-1-phenyl)-4.6-dimethyl-pyrimidin in 40 ml abs. DMF wurde bei 80°C unter Rühren nach 10 Min., nach weiteren 30 Min. und nach weiteren 60 Min. mit jeweils 325 mg (insgesmat 975 mg (7 m Mol)) pulverisiertem $K_2CO_3$ versetzt und danach noch 3 Stdn. bei 80° gerührt. Nach Verdampfen des DMF im Vakuum wurde der kristalline Rückstand in etwa 50 ml Wasser aufgenommen, die Mischung neutralisiert, 20 Min. geschüttelt und anschließend abgesaugt. Den Filterrückstand wusch man mit Wasser aus und kochte ihn danach mit 30 ml $CH_3OH$ 5 Min. am Rückfluß. Die abgekühlte Mischung wurde abgesaugt und der Feststoff getrocknet. Man erhielt so 3.15 g (≙ 59,7 % Ausbeute) 4-(4-Hydroxyphenyl)-1-(4-(4.6-dimethyl-pyrimidin-2-yl)-benzyl)-piperazin, Fp. 165-67°C, Analyse: $C_{23}H_{26}N_4O$ (MG 374,49) Ber.C 73,77 H 7,00 N 14,96; Gef.C 73,6, H 7,0, N 14,7 %.

Beispiel 23

Gemäß der in den Beispielen 19-22 beschriebenen Arbeitsmethode wurden ausgehend von einer Verbindung der Formel XIVa und jeweils der entsprechenden Brom- oder Chlormethyl-Verbindung der Formel X (E"=Br oder Cl; Y vgl. Tab. 5) die in der Tabelle 5 aufgeführten Verbindungen der Formel IIIa hergestellt.
Sofern der nach Abdestillieren des DMF verbliebene Rückstand beim Aufnehmen in Wasser und Neutralisieren ein kristallines Produkt lieferte , wurde dieses wie im Beispiel 22 beschrieben weiter aufgearbeitet. Fiel an dieser Stelle keine kristalline Substanz an, wurde mit $CH_2Cl_2$ extrahiert. Der $CH_2Cl_2$-Extrakt-Rückstand wurde aus $CH_3OH$ oder $CH_3CN$ kristallisiert. Falls auf diese Weise keine kristalline Substanz zu erhalten oder die anfallende Substanz zu stark verunreinigt war, wurde der $CH_2Cl_2$-Extrakt-Rückstand an einer KieselgelS/$CH_2Cl_2$-Säule durch Elution mit $CH_2Cl_2$ und $CH_2Cl_2/C_2H_5OH$-Mischungen chromatographisch gereinigt. Auf chromatographischem Weg gereinigte, in der Tabelle 5 enthaltene Verbindungen der Formel IIIa sind mit "*" gekennzeichnet.

Tabelle 5

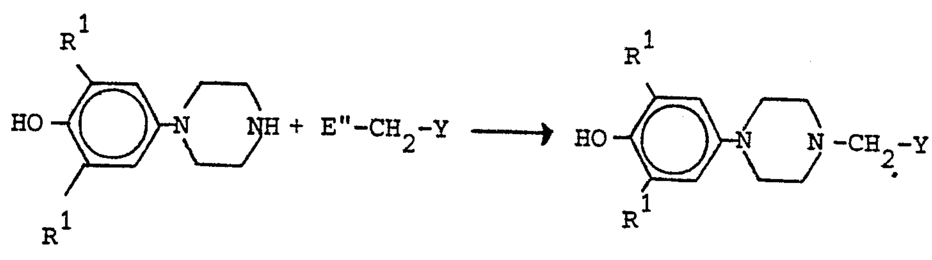

XIVa

X

(E"= Br oder Cl;

Y vgl. Tabelle)

IIIa

| Verb. Nr. | $R^1$ | Y | Ausbeute [%] | Fp. [°C] | Summenformel | Analyse % Ber. | Gef. |
|---|---|---|---|---|---|---|---|
| 5,1 | H | 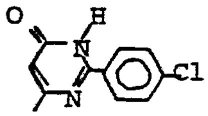 | 71 | 269-70 | $C_{21}H_{23}ClN_4O_3$ ‡ | C 60,79<br>H 5,59<br>N 13,50 | 60,2<br>5,7<br>13,1 |
| 5,2 | H | 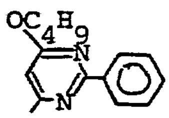 | 69 | 146-47 | $C_{25}H_{30}N_4O_2$ | C 71,74<br>H 7,22<br>N 13,39 | 71,6<br>7,0<br>13,3 |
| 5,3* | $CH_3$ | 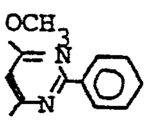 | 62 | 119-20 | $C_{24}H_{28}N_4O_2$ | C 71,26<br>H 6,98<br>N 13,85 | 71,0<br>7,0<br>13,8 |
| 5,4* | H | 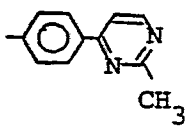 | 57 | 250-52 | $C_{22}H_{24}N_4O$ | C 73,31<br>H 6,71<br>N 15,54 | 73,1<br>6,5<br>15,3 |
| 5,5* | $CH_3$ | " | 55 | 185-86 | $C_{24}H_{28}N_4O$ | C 74,20<br>H 7,26<br>N 14,42 | 74,3<br>7,2<br>14,1 |
| 5,6* | H | 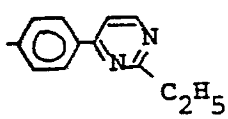 | 35 | 192-93 | $C_{23}H_{26}N_4O$ | C 73,77<br>H 7,00<br>N 14,96 | 73,8<br>7,1<br>14,7 |
| 5,7 | H | 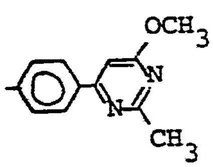 | 68 | 175-76 | $C_{23}H_{26}N_4O_2$ | C 70,74<br>H 6,71<br>N 14,35 | 70,8<br>6,6<br>14,3 |
| 5,8* | H | 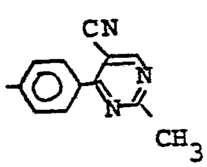 | 51 | 234-35 | $C_{23}H_{23}N_5O$ | C 71,67<br>H 6,01<br>N 18,17 | 71,3<br>5,9<br>17,9 |

| Verb. Nr. | $R^1$ | Y | Ausbeute [%] | Fp. [°C] | Summenformel | Analyse % Ber. | Gef. |
|---|---|---|---|---|---|---|---|
| 5,9* | H | 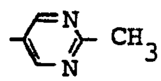 | 63 | - | $C_{16}H_{20}N_4O$ | C 67,58 | 67,2 |
| | | | | | | H 7,09 | 6,9 |
| | | | | | | N 19,70 | 19,3 |
| 5,10* | $CH_3$ | 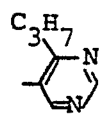 | 50 | - | $C_{20}H_{28}N_4O$ | C 70,55 | 70,0 |
| | | | | | | H 8,29 | 8,2 |
| | | | | | | N 16,46 | 16,0 |
| 5,11 | H | 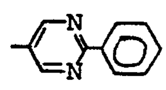 | 83 | 176-77 | $C_{21}H_{22}N_4O$ | C 72,81 | 72,5 |
| | | | | | | H 6,40 | 6,2 |
| | | | | | | N 16,17 | 15,9 |
| 5,12* | $CH_3$ | " | 56 | 165-67+ | $C_{23}H_{27}ClN_4O^+$ | C 67,22 | 67,1 |
| | | | | | | H 6,62 | 7,1 |
| | | | | | | Cl 8,63 | 9,1 |
| | | | | | | N 13,63 | 12,8 |
| 5,13 | H | 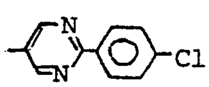 | 66 | 208-09 | $C_{21}H_{21}ClN_4O$ | C 66,22 | 66,0 |
| | | | | | | H 5,56 | 5,7 |
| | | | | | | N 14,71 | 14,4 |
| 5,14 | H | 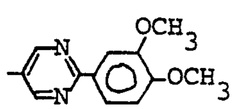 | 62 | 179-80 | $C_{23}H_{26}N_4O_3$ | C 67,96 | 67,5 |
| | | | | | | H 6,45 | 6,3 |
| | | | | | | N 13,78 | 13,4 |
| 5,15 | H | 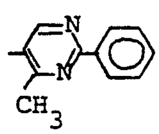 | 80 | 202-03 | $C_{22}H_{24}N_4O$ | C 73,31 | 73,4 |
| | | | | | | H 6,71 | 6,6 |
| | | | | | | N 15,54 | 15,3 |
| 5,16 | $CH_3$ | 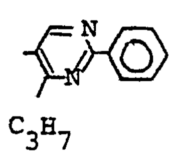 | 51 | 81-83 | $C_{26}H_{32}N_4O$ | C 74,97 | 74,7 |
| | | | | | | H 7,74 | 7,8 |
| | | | | | | N 13,45 | 13,4 |

| Verb. Nr. | $R^1$ | Y | Ausbeute [%] | Fp. [°C] | Summenformel | Analyse % Ber. | Gef. |
|---|---|---|---|---|---|---|---|
| 5,17* | H | 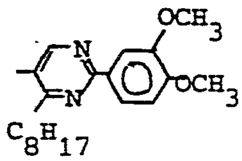 | 73 | 145-46 | $C_{31}H_{42}N_4O_3$ | C 71,78 | 71,4 |
| | | | | | | H 8,16 | 8,0 |
| | | | | | | N 10,80 | 10,8 |
| 5,18* | H | 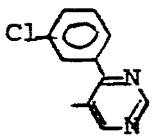 | 55 | 182-83 | $C_{21}H_{21}ClN_4O$ | C 66,22 | 66,1 |
| | | | | | | H 5,56 | 5,7 |
| | | | | | | N 14,71 | 14,6 |
| 5,19* | H | 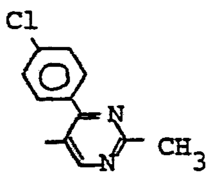 | 57 | 155-56 | $C_{22}H_{23}ClN_4O$ | C 66,91 | 66,8 |
| | | | | | | H 5,87 | 5,8 |
| | | | | | | N 14,19 | 14,2 |
| 5,20* | H | 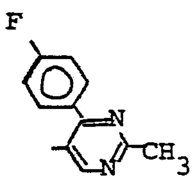 | 63 | 190-91 | $C_{22}H_{23}FN_4O$ | C 69,82 | 69,6 |
| | | | | | | H 6,13 | 6,3 |
| | | | | | | N 14,81 | 14,7 |
| | | | | | | F 5,02 | 4,9 |
| 5,21* | $CH_3$ | 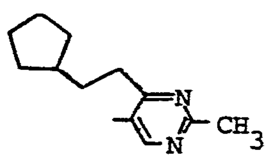 | 59 | - | $C_{25}H_{36}N_4O$ | C 73,49 | 73,1 |
| | | | | | | H 8,88 | 8,7 |
| | | | | | | N 13,71 | 13,4 |
| 5,22* | H | 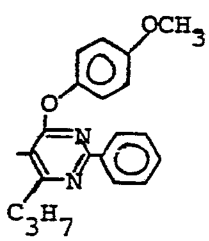 | 81 | 168-69 | $C_{31}H_{35}N_4O_3$ | C 72,77 | 72,2 |
| | | | | | | H 6,90 | 6,8 |
| | | | | | | N 10,95 | 10,6 |
| 5,23 | H | 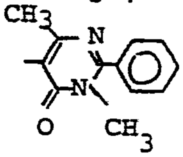 | 94 | 180-81 | $C_{23}H_{26}N_4O_2$ | C 70,74 | 70,5 |
| | | | | | | H 6,71 | 6,6 |
| | | | | | | N 14,35 | 14,1 |

| Verb. Nr. | $R^1$ | Y | Ausbeute [%] | Fp. [°C] | Summenformel | Analyse % Ber. | Gef. |
|---|---|---|---|---|---|---|---|
| 5,24* | H | 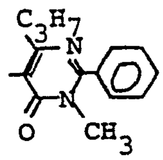 | 67 | 194-95 | $C_{25}H_{30}N_4O_2$ | C 71,74<br>H 7,22<br>N 13,39 | 71,8<br>7,1<br>13,2 |
| 5,25* | H | 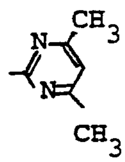 | 53 | 202-04 | $C_{17}H_{22}N_4O$ | C 68,43<br>H 7,43<br>N 18,78 | 68,3<br>7,4<br>18,7 |
| 5,26* | H | 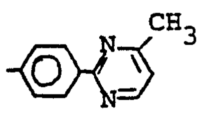 | 79 | 163-64 | $C_{22}H_{24}N_4O$ | C 73,31<br>H 6,71<br>N 15,54 | 73,3<br>6,6<br>15,2 |
| 5,27* | H | 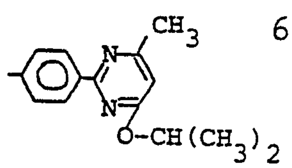 | 61 | 128-29 | $C_{25}H_{30}N_4O_2$ | C 71,74<br>H 7,22<br>N 13,39 | 71,5<br>7,2<br>13,3 |
| 5,28* | $CH_3$ | 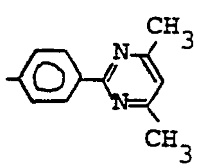 | 63 | 109-10 | $C_{25}H_{30}N_4O$ | C 74,59<br>H 7,51<br>N 13,92 | 74,6<br>7,4<br>13,7 |
| 5,29* | H | 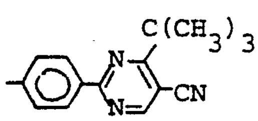 | 44 | - | $C_{28}H_{33}N_5O$ | C 73,81<br>H 7,30<br>N 15,37 | 73,6<br>7,1<br>15,1 |
| 5,30 | H | 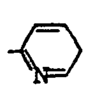 | 67 | 168-69 | $C_{16}H_{19}N_3O$ | C 71,35<br>H 7,11<br>N 15,60 | 71,3<br>7,0<br>15,5 |
| 5,31 | H | 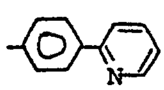 | 53 | 179-80 | $C_{22}H_{23}N_3O$ | C 76,49<br>H 6,71<br>N 12,17 | 76,5<br>6,6<br>12,1 |

| Verb. Nr. | $R^1$ | Y | Ausbeute [%] | Fp. [°C] | Summenformel | Analyse % Ber. | Gef. |
|---|---|---|---|---|---|---|---|
| 5,32 | H | 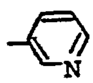 | 83 | 194-95 | $C_{16}H_{19}N_3O$ | C 71,35<br>H 7,11<br>C 15,60 | 71,2<br>7,0<br>15,6 |
| 5,33 | H | 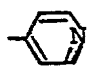 | 74 | 251-53 | $C_{16}H_{19}N_3O$ | C 71,35<br>H 7,11<br>N 15,60 | 71,1<br>7,1<br>15,4 |
| 5,34 | $CH_3$ | " | 56 | 156-57 | $C_{18}H_{23}N_3O$ | C 72,69<br>H 7,80<br>N 14,13 | 72,6<br>7,9<br>14,1 |
| 5,35* | H | 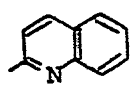 | 67 | 83-84 | $C_{20}H_{21}N_3O$ | C 75,21<br>H 6,63<br>N 13,16 | 75,3<br>6,6<br>13,0 |
| 5,36* | $CH_3$ | 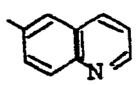 | 60 | - | $C_{22}H_{25}N_3O$ | C 76,05<br>H 7,25<br>N 12,09 | 75,7<br>7,0<br>11,7 |
| 5,37 | H | 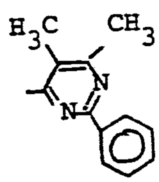 | 83 | 115-16 | $C_{23}H_{26}N_4O$ | C 73,77<br>H 7,00<br>N 14,96 | 73,6<br>7,1<br>14,9 |
| 5,38 | H | 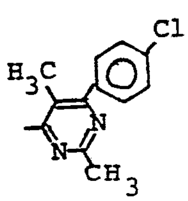 | 67 | 152-53 | $C_{23}H_{25}ClN_4O$ | C 67,55<br>H 6,16<br>N 13,70 | 67,2<br>6,2<br>13,3 |
| 5,39 | H | 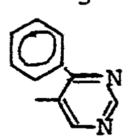 | 29 | 168-69 | $C_{21}H_{22}N_4O$ | C 72,81<br>H 6,40<br>N 16,17 | 72,6<br>6,2<br>16,2 |

*) Hydrat +) Hydrochlorid

0237962

Beispiel 24

Zu einer Mischung von 1,78 g (10 m Mol) 1-(4-Hydroxyphenyl)-piperazin, 3,05 g 95 %igem 5-Brommethyl-4-methyl-2-phenyl-pyrimidin (≙ 11 m Mol) und 80 ml abs. 1.2-Dimethoxyethan wurde unter Rühren in 30 Min. bei 45°C eine Lösung von 1,05 g Triethylamin in 20 ml abs. Dimethoxyethan zugetropft. Man rührte 1 Std. bei 45°C und 3 Stdn. bei 80°C nach, verdampfte im Vakuum das Lösungsmittel, nahm den Rückstand in $CH_2Cl_2$/Wasser auf, neutralisierte auf pH 6-7, trennte die Phasen und extrahierte die wässrige Phase dreimal mit $CH_2Cl_2$. Die vereinigten $CH_2Cl_2$-Extrakte wurden getrocknet, filtriert und i. Vak. eingedampft. Der kristalline Rückstand (3,7 g) wurde aus Methanol umkristallisiert.
Man erhielt 2,35 g (65% d.Th.) reines 4-(4-Hydroxyphenyl)-1-(4-methyl-2-phenyl-pyrimidin-5-ylmethyl)-piperazin, Fp. 201-202°C.
Diese Synthese kann ebenso mit 1-(4-Hydroxyphenyl)-piperazin-dihydrobromid ausgeführt werden. In diesem Fall werden bei einem 10 m Mol Ansatz 3,10 g (30,7 m Mol) Trietylamin verwendet und die Reaktionszeit bei 45°C wird auf 12 Stdn. und die bei 80°C auf 6 Stdn. ausgedehnt. Man erhält etwa die gleiche Menge reines 4-(4-Hydroxyphenyl)-1-(4-methyl-2-phenyl-pyrimidin-5-ylmethyl)-piperazin wie bei der vorstehenden Ausführung mit der Base des Piperazin-Derivats.

Beispiele zur Herstellung von Brommethyl-pyrimidinen — Ausgangsstoffen zur Synthese von Verbindungen der Formel IIIa:

Beispiel 25

Eine Lösung von 5,53 g (30 m Mol) 4.5-Dimethyl-2-phenyl-pyrimidin in 110 ml $CCl_4$ wurde mit 5,54 g (31,1 m Mol) N-Bromsuccinimid (NBS) und 6 mg Dibenzoylperoxid versetzt und unter Rühren und Bestrahlung mit einer Tageslichtlampe mit erhöhtem kurzwelligen Anteil (Typ: Osram Ultra-Vitalux, 300 W, GUR 53) 5 Min. auf Siedetemperatur erwärmt. Danach war das NBS verbraucht und in spezifisch leichtes Succinimid übergegangen. Bei Zimmertemperatur wurde dieses abgesaugt und mit wenig $CCl_4$ nachgewaschen. Das Filtrat wurde i. Vak. eingedampft, der Rückstand in 100 ml Diisopropylether gelöst, die Lösung über Kieselgur geklärt und das klare Filtrat wiederum i. Vak. eingedampft. Man erhielt 7,70 g, laut $^1$H-NMR Spektrum 94,5 %iges, 5-Brommethyl-4-methyl-2-phenyl-pyrimidin, (≙ 92 % Ausbeute), Fp. 65-66°C, Analyse: $C_{12}H_{11}BrN_2$ (MG 263,15) Ber. C 54,77 H 4,21 Br 30,37 N 10,65; Gef. C 53,5, H 3,9, Br 31,3, N 10,0 %.

Beispiel 26

Eine Lösung von 5,12 g (21,2 m Mol) 3.4-Dihydro-3.5-dimethyl-2-phenyl-6-propyl-pyrimidin-4-on in 80 ml $CCl_4$ wurde mit 4,19 g (23,5 m Mol) NBS und 5 mg Dibenzoylperoxid versetzt und unter Rühren und Bestrahlung (vgl. Beispiel 25) 7 Min. auf Siedetemperatur erwärmt. Danach war das NBS in Succinimid übergegangen. Man saugte ab, wusch mit $CCl_4$ nach und dampfte das Filtrat i. Vak. ein. Der Rückstand wurde in n-Pentan/Diisopropylether 2:1 gelöst, die trübe Lösung mit Kieselgur geklärt und das klare Filtrat i. Vak. wiederum bis zur Gewichtskonstanz eingedampft. Der Rückstand (7,1 g) bestand laut $^1$H-NMR-Spektrum aus 65 %igem 5-Brommethyl-3.4-dihydro-3-methyl-2-phenyl-6-propyl-pyrimidin-4-on (oder der entsprechenden 1.4-Dihydro-1-methyl-Verbindung; vgl. bei für Y unter $b_2$ genannte Pyrimidone). In dieser Form wurde diese Brommethyl-Verbindung mit 1-(4-Hydroxyphenyl)-piperazin unter Bildung der im Beispiel 23, Tabelle 5, Nr. 5.24 beschriebenen Verbindung der Formel IIIa umgesetzt.

Beispiel 27

Eine Lösung von 4,50 g (22,7 m Mol) 2-Ethyl-4-(4-tolyl)-pyrimidin in 90 ml $CCl_4$ wurde mit 4,04 g (22,7 m Mol) NBS und 3 mg Dibenzoylperoxid versetzt und unter Rühren und Bestrahlung (vgl. Beispiel 25) 3 Min. auf Siedetemperatur erwärmt. Danach war das NBS in Succinimid übergegangen. Man saugte nach Abkühlung auf Raumtemperatur ab, wusch mit $CCl_4$ nach und dampfte das Filtrat i. Vak. ein. Der Rückstand wurde in Diisopropylether gelöst, die trübe Lösung mit Kieselgur geklärt und das klare Filtrat i. Vak. wiederum bis zur Gewichtskonstanz eingedampft. Der Rückstand (5,83 g) bestand laut $^1$H-NMR-Spektrum zu ca. 43 % aus [4-(4-Brommethyl-phenyl)-2-ethyl-pyrimidin, zu ca. 34 % aus 2-(1-Bromethyl)-4-(4-tolyl)-pyrimidin und zu ca. 23 % aus unverändertem 2-Ethyl-4-(4-tolyl)-pyrimidin. Diese Mischung wurde mit 1-(4-Hydroxyphenyl)-piperazin unter Bildung der entsprechenden 4-(4-Hydroxyphenyl)-piperazin-Derivate umgesetzt. Durch Chromatographie an einer KieselgelS-$CH_2Cl_2$-Säule wurden die beiden strukturisomeren Piperazin-Derivate getrennt und die im Beispiel 23, Tabelle 5, Nr. 5.6 beschriebene Verbindung der Formel IIIa rein erhalten.

Beispiel 28

Eine Lösung von 2,73 g (12,5 m Mol) 4-(4-Chlorphenyl)-2.5-dimethyl-pyrimidin in 48 ml $CCl_4$ wurde mit 2,26 g (12,7 m Mol) NBS und 3 mg Dibenzoylperoxid versetzt und unter Rühren und Bestrahlung (vgl. Beispiel 25) 5 Min. am Rückfluß gekocht. Danach war das NBS in Succinimid übergegangen. Bei Raumtemperatur saugte man über Filtrierhilfe ab, wusch mit $CCl_4$ nach und dampfte das Filtrat im Vakuum ein. Den Rückstand löste man in ca. 60 ml Diisopropylether/n-Pentan 1:1, klärte die schwach trübe Lösung mit Kieselgur, saugte wiederum ab und dampfte das klare Filtrat im Ölpumpenvakuum bis zur Gewichtskonstanz ein . Der Rückstand

(3,4 g) bestand laut $^{1}$H-NMR-Spektrum aus 69,5 %igem 5-Brommethyl-4-(4-chlorphenyl)-2-methyl-pyrimidin (≙ 63,5 % Ausbeute). In dieser Form wurde diese Brommethyl-Verbindung zur Umsetzung mit 1-(4-Hydroxyphenyl)-piperazin verwendet (vgl. Beispiel 23, Nr. 5,19).

Beispiel 29

Nach der im Beispiel 28 beschriebenen Arbeitsweise wurden ausgehend von 2,37 g (14 m Mol) 2-(4-Tolyl)-pyridin in 80 ml $CCl_4$, 2,60 g (14,6 m Mol) NBS und 3 mg Dibenzoylperoxid als kristalliner Eindampfrückstand 3,61 g, laut $^{1}$H-NMR-Spektrum ca. 82 %iges 2-(4-Brommethyl-phenyl)-pyridin, Fp. 51-52°C erhalten (Ausbeute ≙ 85 % d.Th.). In dieser Form wurde die Brommethyl-Verbindung mit 1-(4-Hydroxyphenyl)-piperazin unter Bildung der im Beispiel 23, Nr. 5.31 beschriebenen Verbindung der Formel IIIa umgesetzt.

Als Beispiel für die hohe lokale in vivo Wirksamkeit der erfindungsgemäßen Verbindungen werden Behandlungsergebnisse an experimentell mit Trichophyton mentagrophytes infizierten Labortieren angeführt.
Zur Feststellung der lokalen Wirksamkeit wurden jeweils zwei 450-500 g schwere Meerschweinchen (Stamm Pirbright white) mit 1,5 x $10^4$ Konidien/Tier in die Epidermis, verteilt auf 6 Infektionspunkte, infiziert.
Die Behandlung der Tiere erfolgte dermal ab dem 3. Tag nach der Infektion an 5 aufeinanderfolgenden Tagen durch Aufbringen einer 0,1 %igen Präparatlösung auf 3 Infektionsstellen einer Rückenseite. Die andere Rückenseite wurde mit Vehikel ohne Präparat auf gleiche Weise behandelt.
Neben den mit den erfindungsgemäßen Substanzen behandelten Tieren wurden zwei Tiere mit der Referenzsubstanz Terconazol behandelt, zwei Tiere blieben nach der Infektion unbehandelt.

Wie in Tabelle 6 ersichtlich, zeigten die erfindungsgemäßen Verbindungen eine deutlich stärkere Reduzierung der Mykosendurchmesser als das Standardpräparat Terconazol, d.h. der antimykotische Effekt der erfindungsgemäßen Verbindung war demjenigen von Terconazol bis zu 60 % überlegen.

Tabelle 6

| Konzentration | Präparat Beispiel Nr. | Vehikelkontrollen $x_1$ | (s)* | n** | Präparat + Vehikel $x_2$ | (s)* | n** | Differenz $x_1 - x_2$ (%) |
|---|---|---|---|---|---|---|---|---|
| Dermal | (6) 2.17 | 12,7 | (1,3) | 6 | 2,8 | (0,8) | 6 | 9,9 (157,1) |
| 5 x 0.1 % | (3) | 14,0 | (1,2) | 6 | 4,3 | (1,5) | 6 | 9,7 (153,9) |
| | (6) 2.28 | 13,7 | (1,6) | 6 | 3,5 | (1,0) | 6 | 10,2 (161,9) |
| | Terconazol | 13,9 | (1,2) | 6 | 7,6 | (1,4) | 6 | 6,3 (100) |
| Kontrollen infizierte Tiere unbehandelt | - | 14,1 | (2,8) | 12 | | - | | - |

* (s) = Standardabweichung
** n = Anzahl Meßwerte

Als Beispiel für die hohe orale und parenterale in-vivo-Wirksamkeit der erfindungsgemäßen Verbindungen werden Behandlungsergebnisse an experimentell mit Candida albicans infizierten Labortieren angeführt.

Zur Feststellung der oralen und parenteralen Wirksamkeit wurden Gruppen von je 5 18-20 g schweren Mäusen (Stamm HOE: NMRKF; SPF 71) mit 2 . $10^6$ Keimen/Tier infiziert.

Die Behandlung der Tiere erfolgte oral bzw. subcutan in 8 gleichen Einzeldosen zu je 30 mg/kg bzw. 10 mg/kg Körpergewicht (-24/-18/-2h/+2/24/30/48/54h).

Neben der mit den erfindungsgemäßen Substanzen I behandelten Gruppe von 5 Tieren wurde zum Vergleich eine Gruppe von ebenfalls 5 Tieren mit der Referenzsubstanz Ketoconazol behandelt. Eine Kontrollgruppe von 10 Tieren blieb nach der Infektion unbehandelt.

Wie aus Tabelle 7 hervorgeht, fand sich bei den erfindungsgemäßen Verbindungen eine bis um das Doppelte verlängerte Überlebenszeit der Tiere nach der Infektion, verglichen mit dem derzeitigen Standardpräparat Ketoconazol.

Tabelle 7

| Dosis | Präparat Beispiel Nr. | Anzahl Tiere | Überlebenszeiten* Tage nach Infektion | | | | | x Tage | Überlebenszeit in % (Standardpräp.=100%) |
|---|---|---|---|---|---|---|---|---|---|
| oral 8 x 30 mg/kg | (6) 2.3 | 5 | 8 | 9 | 10 | 10 | 10 | 9,4 | 127,0 |
| | (6) 2.20 | 5 | 7 | 9 | 10 | 12 | 12 | 10,0 | 135,1 |
| | (6) 2.18 | 5 | 7 | 9 | 10 | 11 | 12 | 9,8 | 132,4 |
| | Ketoconazol | 5 | 6 | 7 | 7 | 8 | 9 | 7,4 | 100 |
| oral 8 x 10 mg/kg | (6) 2.3 | 5 | 7 | 7 | 7 | 7 | 7 | 7,0 | 129,6 |
| | (6) 2.20 | 5 | 5 | 6 | 6 | 7 | 7 | 6,2 | 114,8 |
| | (6) 2.18 | 5 | 6 | 6 | 8 | 9 | 11 | 8,0 | 148,1 |
| | Ketoconazol | 5 | 5 | 5 | 5 | 6 | 6 | 5,4 | 100 |
| subcutan 8 x 30 mg/kg | (6) 2.3 | 5 | 10 | 13 | 14 | 14 | 14 | 13,0 | 158,5 |
| | (6) 2.20 | 5 | 14 | 14 | 14 | 14 | 14 | 14,0 | 170,7 |
| | (6) 2.18 | 5 | 14 | 14 | 14 | 14 | 14 | 14,0 | 170,7 |
| | Ketoconazol | 5 | 8 | 8 | 8 | 8 | 9 | 8,2 | 100 |
| subcutan 8 x 10 mg/kg | (6) 2.3 | 5 | 8 | 10 | 11 | 13 | 14 | 11,2 | 175 |
| | (6) 2.20 | 5 | 10 | 12 | 12 | 14 | 14 | 12,4 | 193,7 |
| | (6) 2.18 | 5 | 9 | 11 | 12 | 12 | 13 | 11,4 | 178,1 |
| | Ketoconazol | 5 | 5 | 5 | 5 | 8 | 9 | 6,4 | 100 |
| Kontrollen infizierte Tiere unbehandelt | - | 10 | 2 | 2 | 2 | 2 | 2 | 2,4 | 37,5 |
| | | | 2 | 2 | 3 | 3 | 4 | | |

* Versuch abgebrochen nach 14 Tagen

0237962
HOE 86/F 061

Patentansprüche:

1. Verbindung der Formel I

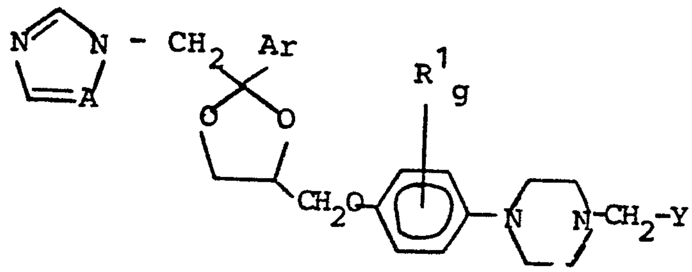

in der

A CH oder N,

Ar Naphthyl, Thienyl, Halothienyl oder eine unsubstituierte oder eine bis zu 3 Substituenten tragende Phenylgruppe bedeutet, wobei die Substituenten gleich oder verschieden sein können und F, Cl, Br, J, $CF_3$, $OCH_3$, $OC_2H_5$, $CH_3$, $C_2H_5$ oder Phenoxy bedeuten,

$R^1$ $C_1$-$C_3$-Alkyl, F oder Cl,

g 0, 1 oder 2 und

Y die folgenden heterocyclischen Reste

$a_1$) 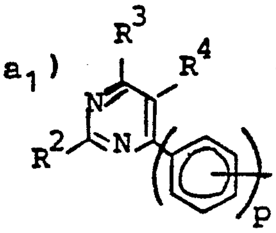 oder $a_2$) 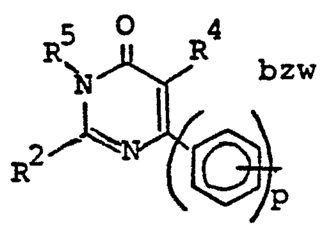 bzw. 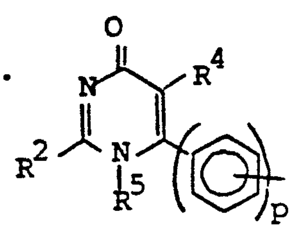

oder

$b_1$) 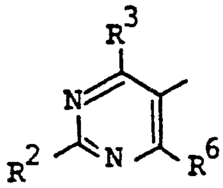 oder $b_2$) 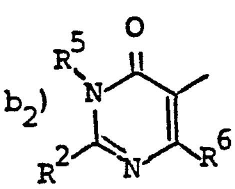 bzw. 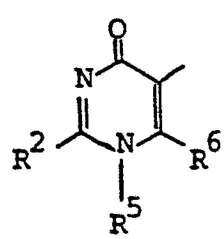

oder

c) 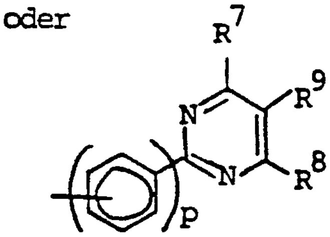

in denen

$R^2$ H, $C_1$-$C_8$-Alkyl, $C_1$-$C_4$-Alkoxy, eine unsubstituierte oder 1 oder 2 Substituenten tragende Phenylgruppe, wobei die Substituenten gleich oder verschieden sein können und F, Cl, Br, J, $CF_3$, $OCH_3$, $OC_2H_5$, $NO_2$ oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Br, $OCH_3$, $OC_2H_5$, $CH_3$ oder $C_2H_5$ bedeuten, oder beim Rest $a_1$ zusätzlich $C_1$-$C_4$-Alkylthio, eine unsubstituierte oder eine im Phenylrest 1 oder 2 Substituenten tragende Benzylthiogruppe, wobei die Substituenten gleich oder verschieden sein können und F, Cl, $OCH_3$, $OC_2H_5$, $CH_3$ oder $C_2H_5$ bedeuten, bedeutet und

$R^3$ H, OH, Cl, $C_1$-$C_4$-Alkoxy oder eine unsubstituierte oder eine im Phenylrest 1 oder 2 Substituenten tragende Benzyloxygruppe, wobei die Substituenten gleich oder verschieden sein können und F, Cl, Br, $OCH_3$, $OC_2H_5$, $CH_3$ oder $C_2H_5$ bedeuten und beim heterocyclischen Rest $a_1$ zusätzlich $C_1$-$C_4$-Alkyl oder eine unsubstituierte oder mit einem F, Cl, Br, $OCH_3$, $OC_2H_5$, $CH_3$ oder $C_2H_5$ substituierte Phenylgruppe und beim heterocyclischen Rest $b_1$ zusätzlich $C_1$-$C_4$-Alkyl oder eine unsubstituierte oder eine 1 oder 2 Substituenten tragende Phenyloxygruppe, wobei die Substituenten gleich oder verschieden sein können und F, Cl, Br, $OCH_3$, $OC_2H_5$, $CH_3$ oder $C_2H_5$ bedeuten,

$R^4$ H, Cl, Br, $C_1$-$C_4$-Alkyl oder CN,

$R^5$ H, $C_1$-$C_4$-Alkyl, Prop-2-en-1-yl, Prop-2-in-1-yl oder Benzyl,

p 0 oder 1 bedeutet, wobei in $a_1$, $a_2$ und c der Phenylenrest über die 2,3 oder 4-Position gebunden ist, und

$R^6$ H, $C_1$-$C_8$-Alkyl, ($C_3$-$C_6$-Cycloalkyl)-$C_1$-$C_3$-alkyl, eine unsubstituierte oder 1 oder 2 Substituenten tragende Phenylgruppe, wobei die Substituenten gleich oder verschieden sein können und F, Cl, Br, J, $OCH_3$, $OC_2H_5$, 1,2--O-$CH_2$-O-, $CF_3$ oder $C_1$-$C_4$-Alkyl bedeuten, eine unsubstituierte oder im Phenylrest durch $OCH_3$, $OC_2H_5$, 1,2--O-$CH_2$-O-, $CF_3$, F, Cl oder $C_1$-$C_4$-Alkyl substituierte Phenyl-$C_1$-$C_2$-alkylgruppe, oder $CF_3$, und

$R^7$ und $R^8$ unabhängig voneinander H, $C_1$-$C_8$-Alkyl, $C_1$-$C_4$-Alkoxy oder eine unsubstituierte oder eine im Phenylrest 1 oder 2 Substituenten tragende Phenyl- oder Benzylgruppe, wobei die Substituenten gleich oder verschieden sein können und F, Cl, Br, $OCH_3$ oder $OC_2H_5$ bedeuten, und wobei $R^7$ darüberhinaus auch OH bedeuten kann, und

$R^9$ H, Cl,Br oder CN bedeutet, oder

$d_1$) 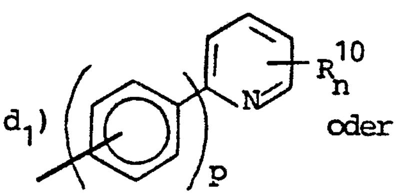 oder $d_2$) 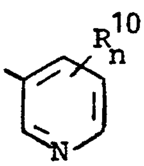 oder $d_3$) 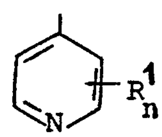

$d_4$) 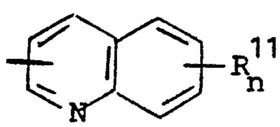 oder $d_5$) 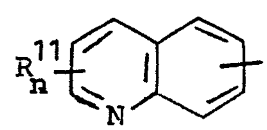 oder

$d_6$) 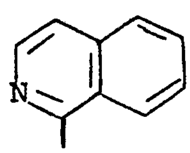 oder $d_7$) 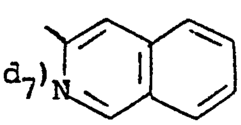

in denen

p 0 oder 1,

$R^{10}$ Cl, Br, $OCH_3$, $OC_2H_5$, $CH_3$ oder $C_2H_5$,
n 0, 1 oder 2 bedeutet, wobei $R^{10}$ in $d_1$ in 3-,4-,5- und/oder 6-, in $d_2$ in 2-,4-,5- und/oder 6- und in $d_3$ in 2-,3-,5- und/oder 6-Stellung stehen kann, und
$R^{11}$ Cl, $OCH_3$, $CH_3$ oder $C_2H_5$ bedeutet und in $d_4$ und $d_5$ in einer bzw. zwei der freien Positionen stehen kann, und der heterocyclische Rest $d_4$ über die 2-, 3- oder 4-Stellung und der heterocyclische Rest $d_5$ über die 5-,6-,7- oder 8-Stellung und die Phenyleneinheit im Rest $d_1$ über die 2,3 oder 4-(para)-Stellung gebunden ist, bedeutet,
sowie deren physiologisch verträgliche Säureadditionssalze.

2. Verbindung I nach Anspruch 1, dadurch gekennzeichnet, daß mindestens einer der Substituenten oder Indices die folgende Bedeutung hat:

A CH oder N,
Ar eine durch 1 oder 2 F- oder Cl-Atome substituierte Phenylgruppe,
$R^1$ $CH_3$ oder $C_2H_5$,
g 0 oder 2,
Y
zu den heterocyclischen Resten $a_1$, $a_2$, $b_1$, $b_2$ und c:
$R^2$ H, $C_1$-$C_4$-Alkyl, $CH_3O$ oder eine unsubstituierte oder eine im Phenylrest 1 oder 2 Substituenten tragende Phenyl- oder eine Benzylgruppe, wobei die Substituenten gleich oder verschieden sein können und F, Cl, Br oder $OCH_3$ bedeuten,
$R^3$ H oder $C_1$-$C_4$-Alkoxy und beim heterocyclischen Rest $a_1$ zusätzlich $CH_3$ oder eine unsubstituierte oder eine mit einem F, Cl, $OCH_3$ oder $CH_3$ substituierte Phenylgruppe und beim Rest $b_1$ zusätzlich eine unsubstituierte oder eine 1 oder 2 Substituenten tragende Phenyloxygruppe, wobei die Substituenten

gleich oder verschieden sein können und F, Cl, Br, $OCH_3$ oder $OC_2H_5$ bedeuten,

$R^4$ H oder CN und beim heterocyclischen Rest $a_1$ zusätzlich $C_1$-$C_4$-Alkyl,

$R^5$ $CH_3$,

p 0 oder 1,

$R^6$ H, $C_1$-$C_8$-Alkyl, ($C_5$-$C_6$-Cycloalkyl)-$C_1$-$C_2$-alkyl, eine unsubstituierte oder 1 oder 2 Substituenten tragende Phenylgruppe, wobei die Substituenten gleich oder verschieden sein können und F, Cl, Br, $OCH_3$, $OC_2H_5$ oder $CF_3$ bedeuten, eine unsubstituierte oder im Phenylrest durch $OCH_3$, $CF_3$, F oder Cl substituierte Benzylgruppe oder $CF_3$,

$R^7$, $R^8$ unabhängig voneinander $C_1$-$C_8$-Alkyl, $C_1$-$C_4$-Alkoxy, eine unsubstituierte oder eine im Phenylrest durch F, Cl, $OCH_3$ oder $OC_2H_5$ substituierte Phenyl- oder Benzylgruppe und $R^7$ darüber hinaus auch H oder OH,

$R^9$ H oder CN,

zu den heterocyclischen Resten $d_1$, $d_2$, $d_3$, $d_4$, $d_5$, $d_6$ und $d_7$:

p 0 oder 1,

$R^{10}$ Cl, $CH_3$ oder $C_2H_5$

n 0 oder 1, wobei $R^{10}$ in $d_3$ bevorzugt in 2-Stellung steht, und

$R^{11}$ Cl, wobei die Phenyleneinheit im Rest $d_1$ bevorzugt über die 4-Stellung gebunden ist.

3. Verbindung I nach Anspruch 1, d.g., daß mindestens einer der Substituenten bzw. Indizes die folgende Bedeutung hat:

A CH oder N,
Ar 2,4-Dichlorphenyl,
$R^1$ $CH_3$,
g 0 oder 2,
Y

zu den heterocyclischen Resten $a_1$, $a_2$, $b_1$, $b_2$ und c:

$R^2$ $CH_3$ oder eine unsubstituierte oder eine im Phenylrest 1 oder 2 Substituenten tragende Phenyl- oder eine Benzylgruppe, wobei die Substituenten gleich oder verschieden sein können und F oder Cl und in der Phenylgruppe und beim Rest $a_1$ bei der Phenyl- und der Benzylgruppe zusätzlich $OCH_3$ bedeuten, und beim Rest $a_1$ zusätzlich $C_2$-$C_4$-Alkyl,

$R^3$ beim Rest $a_1$, falls p=0 ist, $CH_3$ oder eine unsubstituierte oder durch ein F, Cl oder $OCH_3$ substituierte Phenylgruppe, und, falls p=1 ist, H, beim Rest $b_1$ H oder eine durch $OC_2H_5$ substituierte Phenyloxygruppe,

$R^4$ H oder beim Rest $a_1$, falls p=0 ist, zusätzlich $CH_3$

$R^5$ $CH_3$

p bei den heterocyclischen Resten $a_1$ und $a_2$ 0 oder 1 und bei dem heterocyclischen Rest c 1

$R^6$ H, $CH_3$ oder eine unsubstituierte oder durch F, Cl, $OCH_3$ oder $OC_2H_5$ einfach substituierte Phenylgruppe,

$R^7$,$R_8$ unabhängig voneinander $CH_3$ oder eine unsubstituierte oder eine durch F, Cl oder $OCH_3$ substituierte Phenylgruppe und $R^7$ darüber hinaus auch H oder $C_1$-$C_4$-Alkoxy,

$R^9$ H

wobei der Rest d4 bevorzugt über die 2-Stellung und der Rest d5 bevorzugt über die 6- oder 7-Stellung gebunden ist.

4. Verfahren zum Herstellen von Verbindungen I nach Anspruch 1, dadurch gekennzeichnet, daß man

A) eine Verbindung der Formel II

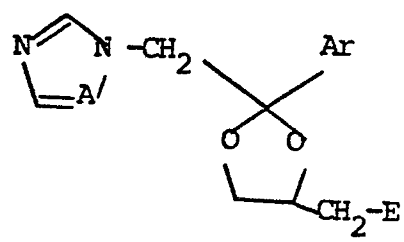

II,

in der
A und Ar die zu Formel I angegebenen Bedeutungen haben und
E Halogen, Acyloxy, $C_1$-$C_3$-Alkylsulfonyloxy, oder Arylsulfonyloxy bedeutet,
mit einer Verbindung der Formel III

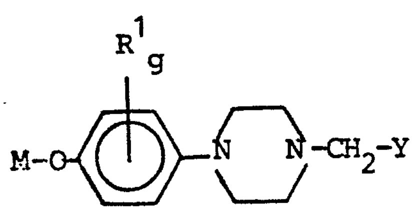

III,

in der
M H, ein Alkali- oder Erdalkalimetall bedeutet und
$R^1$, g und Y die zu Formel I angegebenen Bedeutungen haben, umsetzt, oder daß man

B) eine Verbindung der Formel IV,

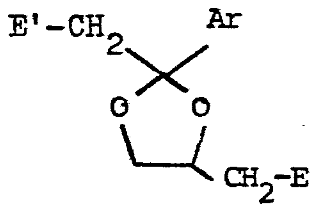

IV

in der
Ar die zu Formel I und E und E' die zu Formel II für E angegebenen Bedeutungen haben,
zunächst mit einer Verbindung der Formel III umsetzt und hierbei eine Verbindung der Formel V herstellt,

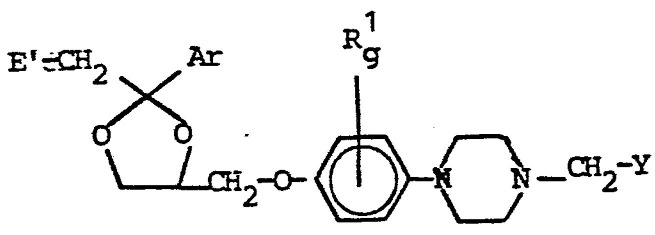

V

in der
Ar, $R^1$, g und Y die zu Formel I und E' die zu Formel II für E angegebenen Bedeutungen haben,
und anschließend eine Verbindung der Formel V mit einer Verbindung der Formel VI umsetzt,

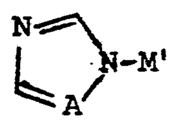

VI

in der A, CH oder N und
M' H, ein Alkali- oder Erdalkalimetall oder $Si(CH_3)_3$ bedeutet,
oder daß man
C) eine Verbindung der Formel VII,

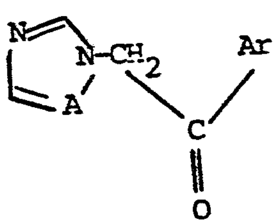

VII

in der A und Ar die zu Formel I angegebenen Bedeutungen haben, mit einem 1,2-Diol der Formel VIII,

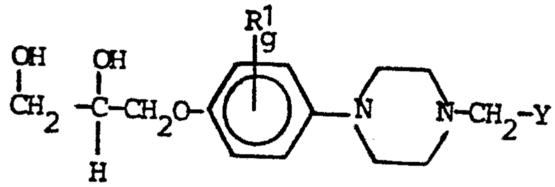

VIII

in der $R^1$, g und Y die zu Formel I angegebenen Bedeutungen haben, umsetzt,

oder daß man

D) eine Verbindung der Formel IX,

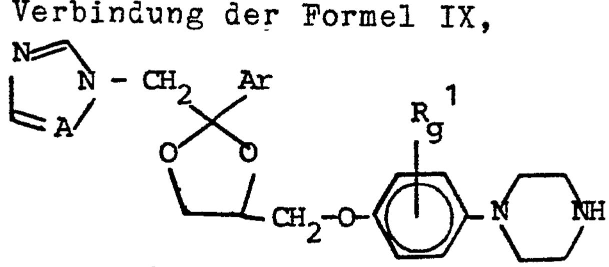

IX

in der A, Ar, $R^1$ und g die zu Formel I angegebenen Bedeutungen haben, mit einer Verbindung der Formel X,

$$E''-CH_2-Y \qquad X$$

in der E" Cl, Br, J, Acyloxy, Alkylsulfonyloxy oder Arylsulfonyloxy bedeutet und Y die zu Formel I unter $a_1$, $a_2$, $b_1$, $b_2$, c, $d_1$, $d_2$, $d_3$, $d_4$, $d_5$, $d_6$ und $d_7$ angegebenen Bedeutungen hat, umsetzt, und gegebenenfalls die nach Weg A)-D) erhaltenen Verbindungen der Formel I mit anorganischen oder organischen Säuren in ihre physiologisch verträglichen Säureadditionssalze überführt.

5. Verbindung der Formel IIIa

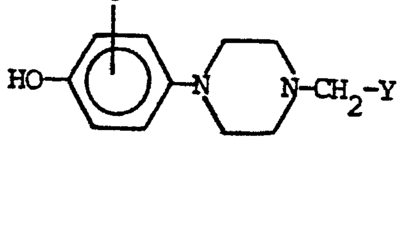

IIIa,

in der bedeuten:

$R^1$ $C_1$-$C_3$-Alkyl, F oder Cl,

g 0, 1 oder 2 und

Y die folgenden heterocyclischen Reste

a$_1$) 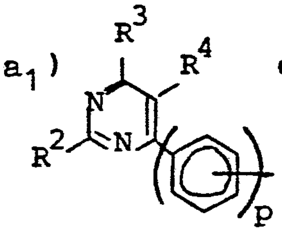 oder a$_2$) 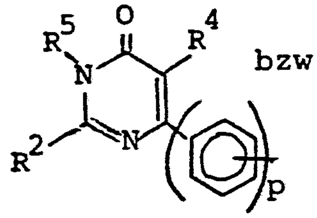 bzw. 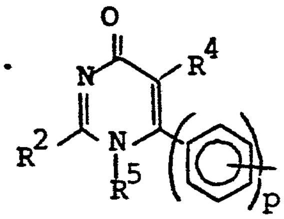

oder

b$_1$) 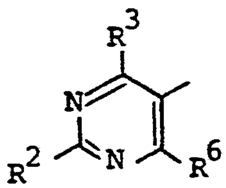 oder b$_2$) 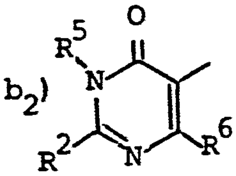 bzw. 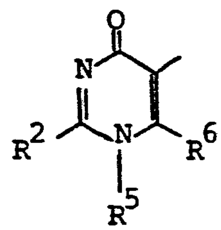

oder

c) 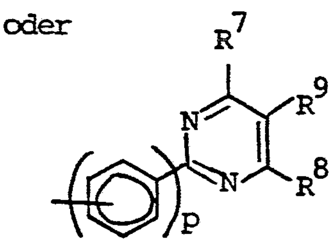

in denen

$R^2$ H, $C_1$-$C_8$-Alkyl, $C_1$-$C_4$-Alkoxy, eine unsubstituierte oder 1 oder 2 Substituenten tragende Phenylgruppe, wobei die Substituenten gleich oder verschieden sein können und F, Cl, Br, J, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Br, $OCH_3$, $OC_2H_5$, $CH_3$ oder $C_2H_5$ bedeuten,
oder bei den heterocyclischen Resten a$_1$ und a$_2$, falls p Null bedeutet, darüberhinaus auch $C_1$-$C_4$-Alkylthio oder eine unsubstituierte oder eine im Phenylrest 1 oder 2 Substituenten tragende Benzylthiogruppe, wobei die Substituenten gleich

oder verschieden sein können und F, Cl, $OCH_3$, $OC_2H_5$, $CH_3$ oder $C_2H_5$ bedeuten, bedeutet und

$R^3$ H, OH, Cl, $C_1$-$C_4$-Alkoxy oder eine unsubstituierte oder eine im Phenylrest 1 oder 2 Substituenten tragende Benzyloxygruppe, wobei die Substituenten gleich oder verschieden sein können und F, Cl, Br, $OCH_3$, $OC_2H_5$, $CH_3$ oder $C_2H_5$ bedeuten, und beim heterocyclischen Rest $a_1$ zusätzlich $C_1$-$C_4$-Alkyl oder eine unsubstituierte oder mit einem F, Cl, Br, $OCH_3$, $OC_2H_5$, $CH_3$ oder $C_2H_5$ substituierte Phenylgruppe und beim heterocyclischen Rest b1 zusätzlich $C_1$-$C_4$-Alkyl oder eine unsubstituierte oder eine 1 oder 2 Substituenten tragende Phenyloxygruppe, wobei die Substituenten gleich oder verschieden sein können und F, Cl, Br, $OCH_3$, $OC_2H_5$, $CH_3$ oder $C_2H_5$ bedeuten,

$R^4$ H, Cl, Br oder CN oder $C_1$-$C_4$-Alkyl

$R^5$ H, $C_1$-$C_4$-Alkyl, Prop-2-en-1-yl, Prop-2-in-1-yl oder Benzyl

p 0 oder 1 bedeutet, wobei in $a_1$, $a_2$ und c der Phenylenrest über die 2,3 oder 4-Position gebunden ist und

$R^6$ H, $C_1$-$C_8$-Alkyl, ($C_3$-$C_6$-Cycloalkyl)-$C_1$-$C_3$-alkyl, eine unsubstituierte oder 1 oder 2 Substituenten tragende Phenylgruppe, wobei die Substituenten gleich oder verschieden sein können und F, Cl, Br, J, $OCH_3$, $OC_2H_5$, 1.2--O-$CH_2$-O-, $CF_3$ oder $C_1$-$C_4$-Alkyl bedeuten, eine unsubstituierte oder im Phenylrest durch $OCH_3$, $OC_2H_5$, 1.2--O-$CH_2$-O-, $CF_3$, F, Cl oder $C_1$-$C_4$-Alkyl substituierte Phenyl-$C_1$-$C_2$-alkylgruppe, oder $CF_3$, und

$R^7$ und $R^8$ unabhängig voneinander H, $C_1$-$C_8$-Alkyl, $C_1$-$C_4$-Alkoxy oder eine unsubstituierte oder eine im Phenylrest 1 oder 2 Substituenten tragende Phenyl- oder Benzylgruppe, wobei die Substituenten gleich oder verschieden sein können und F, Cl, Br, $OCH_3$ oder $OC_2H_5$ bedeuten, und wobei $R^7$ darüberhinaus auch OH bedeuten kann, und

$R^9$ H, Cl, Br oder CN bedeutet, oder

d1) 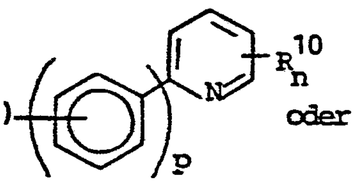 oder d2) 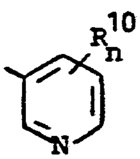 oder d3) 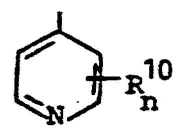 oder

d4) 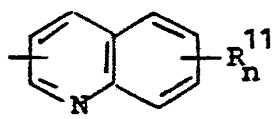 oder d5) 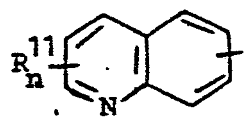 oder

d6) 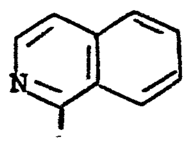 oder d7) 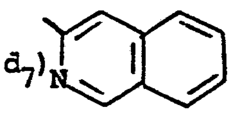

in denen

p 0 oder 1,

$R^{10}$ Cl, Br, $OCH_3$, $OC_2H_5$, $CH_3$ oder $C_2H_5$,

n 0, 1 oder 2 bedeutet, wobei $R^{10}$ in $d_1$ in 3-,4-,5-und/oder 6-, in $d_2$ in 2-,4-,5-und/oder 6- und in $d_3$ in 2-,3-,5-und/oder 6-Stellung steht, und

$R^{11}$ Cl, $OCH_3$, $CH_3$ oder $C_2H_5$ bedeutet und in $d_4$ und $d_5$ in einer bzw. zwei der freien Positionen steht, und der heterocyclische Rest $d_4$ über die 2-,3-oder 4-Stellung und der heterocyclische Rest $d_5$ über die 5-,6-,7-oder 8-Stellung und die Phenyleneinheit im Rest $d_1$ über die 2-,3-oder 4-(para)-Stellung gebunden ist, bedeutet,

sowie deren Säureadditionssalze.

6. Verbindung der Formel III a nach Anspruch 5, dadurch gekennzeichnet, daß mindestens einer der Substituenten oder Indizes $R^1$, g,Y, $R^2$-$R^{11}$, p und n die folgenden Bedeutungen hat:

$R^1$ $CH_3$ oder $C_2H_5$

g 0 oder 2

Y

zu den heterocyclischen Resten $a_1$, $a_2$, $b_1$, $b_2$ und c:

$R^2$ H, $C_1$-$C_4$-Alkyl, $CH_3O$ oder eine unsubstituierte oder eine im Phenylrest 1 oder 2 Substituenten tragende Phenyl- oder eine Benzylgruppe, wobei die Substituenten gleich oder verschieden sein können und

$R^3$ H oder $C_1$-$C_4$-Alkoxy und beim Rest $a_1$ zusätzlich $CH_3$ oder eine unsubstituierte oder eine mit einem F, Cl, $OCH_3$ oder $CH_3$ substituierte Phenylgruppe und beim Rest $b_1$ zusätzlich eine unsubstituierte oder eine 1 oder 2 Substituenten tragende Phenyloxygruppe, wobei die Substituenten gleich oder verschieden sein können und F, Cl, Br, $OCH_3$ oder $OC_2H_5$ bedeuten,

$R^4$ H oder CN,

$R^5$ $CH_3$,

p 0 oder 1

$R^6$ H, $C_1$-$C_8$-Alkyl, ($C_5$-$C_6$-Cycloalkyl)-$C_1$-$C_2$-alkyl, eine unsubstituierte oder 1 oder 2 Substituenten tragende Phenylgruppe, wobei die Substituenten gleich oder verschieden sein können und F, Cl, Br, $OCH_3$, $OC_2H_5$ oder $CF_3$ bedeuten, eine unsubstituierte oder im Phenylrest durch $OCH_3$, $CF_3$, F oder Cl substituierte Benzylgruppe oder $CF_3$,

$R^7$, $R^8$ unabhängig voneinander $C_1$-$C_8$-Alkyl, $C_1$-$C_4$-Alkoxy- oder eine unsubstituierte oder eine im Phenylrest durch F, Cl, $OCH_3$ oder $OC_2H_5$ substituierte Phenyl- oder Benzylgruppe und $R^7$ darüber hinaus auch H oder OH,

$R^9$ H oder CN,

zu den heterocyclischen Resten $d_1$, $d_2$, $d_3$, $d_4$, $d_5$, $d_6$ und $d_7$:

p 0 oder 1,

$R^{10}$ Cl, $CH_3$ oder $C_2H_5$,

n 0 oder 1, wobei $R^{10}$ in $d_3$ bevorzugt in 2-Stellung steht, und

$R^{11}$ Cl, wobei die Phenyleneinheit im Rest $d_1$ bevorzugt über die 4-Stellung gebunden ist.

7. Verfahren zum Herstellen von Verbindungen der Formel III a nach Anspruch 5, dadurch gekennzeichnet, daß man eine Verbindung der Formel XIV,

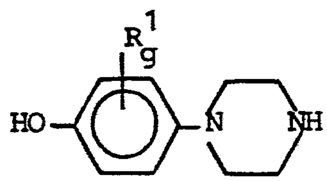

XIV,

in der $R^1$ und g die zu Formel I angegebenen Bedeutungen haben, oder ein Salz dieser Verbindung, mit einer Verbindung der Formel X,

$$E''-CH_2-Y \qquad X,$$

in der E" Cl, Br, J, Acyloxy, Alkylsulfonyloxy oder Arylsulfonyloxy bedeutet und

Y die zur Formel I unter $a_1$, $a_2$, $b_1$, $b_2$, c, $d_1$, $d_2$, $d_3$, $d_4$, $d_5$, $d_6$ und $d_7$ angegebenen Bedeutungen hat,

umsetzt und gegebenenfalls die erhaltenen Verbindungen der Formel IIIa mit anorganischen oder organischen Säuren in ihre Säureadditionssalze überführt.

8. Verfahren nach Anspruch 7, dadurch gekennzeichnet, daß man für die Umsetzung mit Verbindungen der Formel XIV entweder

a) eine Verbindung der Formel $Xa_1$ oder der Formel $Xa_2$,

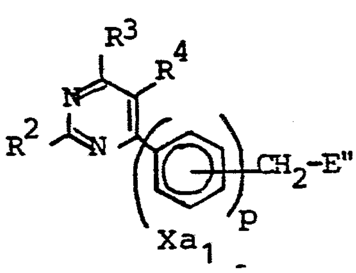

$Xa_1$

,

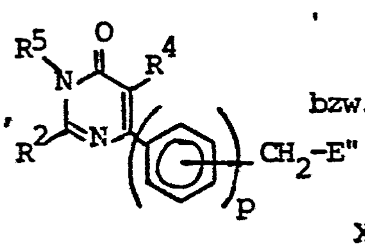

bzw.

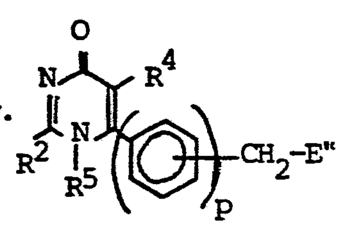

$Xa_2$

in denen $R^2$-$R^5$ die zu Formel I angegebenen Bedeutungen haben und E" Cl oder Br bedeutet, oder

b) eine Verbindung der Formel $Xb_1$ oder der Formel $Xb_2$,

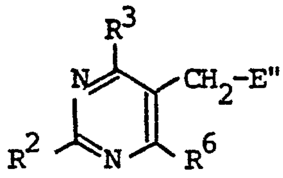

$Xb_1$

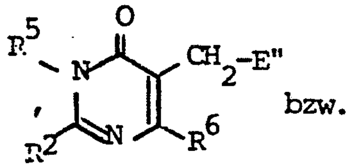

bzw.

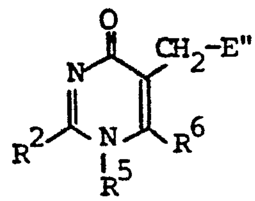

$Xb_2$

in denen $R^2$, $R^3$, $R^5$ und $R^6$ die zu Formel I angegebenen Bedeutungen haben und E" Br bedeutet, oder

c) eine Verbindung der Formel Xc,

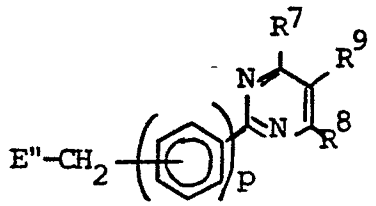

Xc,

in der $R^7$, $R^8$, $R^9$ und p die zu Formel I angegebenen Bedeutungen haben und

E" Cl oder Br bedeutet, oder

d) eine Verbindung der Formel $Xd_1$, der Formel $Xd_2$, der Formel $Xd_3$, der Formel $Xd_4$, der Formel $Xd_5$, der Formel $Xd_6$ oder der Formel $Xd_7$

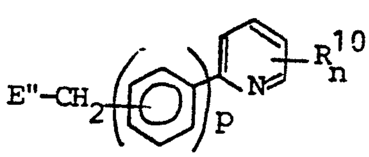

$Xd_1$,

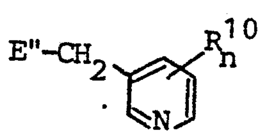

$Xd_2$,

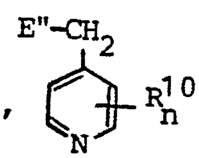

$Xd_3$

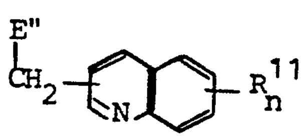

$Xd_4$,

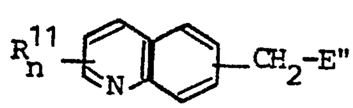

$Xd_5$

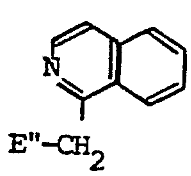

$Xd_6$

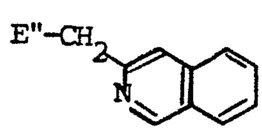

$Xd_7$

in denen $R^{10}$, $R^{11}$, p und n die zu Formel I angegebenen Bedeutungen haben und

E" Cl oder Br bedeutet, einsetzt.

9. Arzneimittel mit antimykotischer Wirkung, dadurch gekennzeichnet, daß es mindestens eine Verbindung I nach Anspruch 1 enthält oder aus ihr besteht.

10. Verfahren zur Herstellung einer pharmazeutischen Zubereitung, dadurch gekennzeichnet, daß man eine Verbindung der Formel (I) nach Anspruch 1 oder eines ihrer Salze, gegebenenfalls zusammen mit einem üblichen pharmazeutischen Trägerstoff und/oder Hilfsstoff, in eine für pharmazeutische Zwecke geeignete Darreichungsform bringt.

11. Verwendung einer Verbindung I nach Anspruch 1 als Antimykotikum.

12. Verwendung einer Verbindung der Formel I nach Anspruch 1 zur Herstellung eines antimykotisch wirkenden Arzneimittels.

13. Verfahren zum Behandeln von Mykosen, dadurch gekennzeichnet, daß man eine wirksame Menge einer Verbindung I nach Anspruch 1 zusammen mit pharmazeutisch geeigneten Trägerstoffen appliziert.

14. Verbindung I nach Anspruch 1, dadurch gekennzeichnet, daß der Azolylmethylrest und die 4-ständige Piperazinophenoxymethylgruppe am Dioxolanring in cis-Stellung stehen.

Patentansprüche Griechenland, Spanien und Österreich:

1. Verfahren zum Herstellen von Verbindungen I

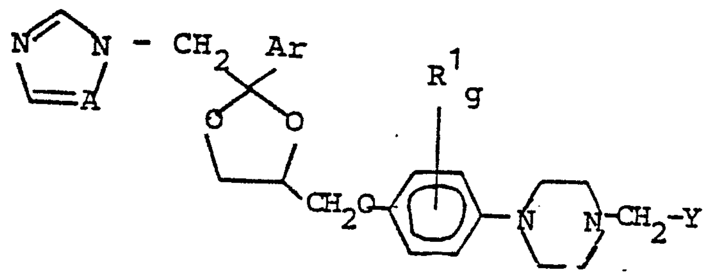

in der

A CH oder N,

Ar Naphthyl, Thienyl, Halothienyl oder eine unsubstituierte oder eine bis zu 3 Substituenten tragende Phenylgruppe bedeutet, wobei die Substituenten gleich oder verschieden sein können und F, Cl, Br, J, $CF_3$, $OCH_3$, $OC_2H_5$, $CH_3$, $C_2H_5$ oder Phenoxy bedeuten,

$R^1$ $C_1$-$C_3$-Alkyl, F oder Cl,

g 0, 1 oder 2 und

Y die folgenden heterocyclischen Reste

$a_1$) 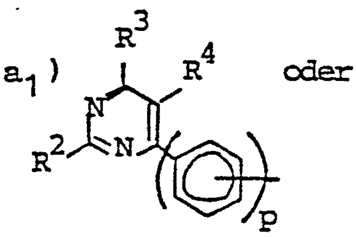 oder $a_2$) 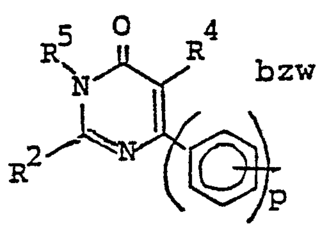 bzw. 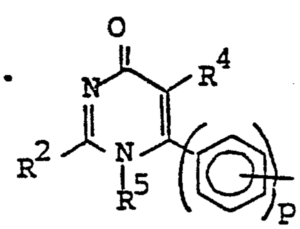

oder

$b_1$) 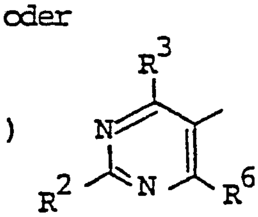 oder $b_2$) 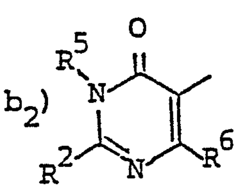 bzw. 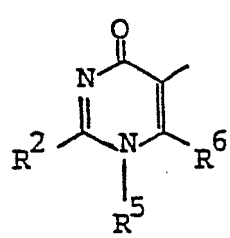

oder

c) 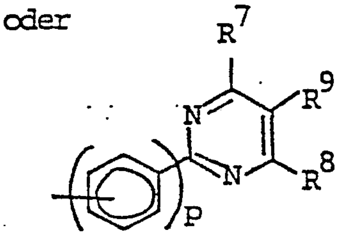

in denen

$R^2$ H, $C_1$-$C_8$-Alkyl, $C_1$-$C_4$-Alkoxy, eine unsubstituierte oder 1 oder 2 Substituenten tragende Phenylgruppe, wobei die Substituenten gleich oder verschieden sein können und F, Cl, Br, J, $CF_3$, $OCH_3$, $OC_2H_5$, $NO_2$ oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Br, $OCH_3$, $OC_2H_5$, $CH_3$ oder $C_2H_5$ bedeuten, oder beim Rest $a_1$ zusätzlich $C_1$-$C_4$-Alkylthio, eine unsubstituierte oder eine im Phenylrest 1 oder 2 Substituenten tragende Benzylthiogruppe, wobei die Substituenten gleich oder verschieden sein können und F, Cl, $OCH_3$, $OC_2H_5$, $CH_3$ oder $C_2H_5$ bedeuten, bedeutet und

$R^3$ H, OH, Cl, $C_1$-$C_4$-Alkoxy oder eine unsubstituierte oder eine im Phenylrest 1 oder 2 Substituenten tragende Benzyloxygruppe, wobei die Substituenten gleich oder verschieden sein können und F, Cl, Br, $OCH_3$, $OC_2H_5$, $CH_3$ oder $C_2H_5$ bedeuten und beim heterocyclischen Rest $a_1$ zusätzlich $C_1$-$C_4$-Alkyl oder eine unsubstituierte oder mit einem F, Cl, Br, $OCH_3$, $OC_2H_5$, $CH_3$ oder $C_2H_5$ substituierte Phenylgruppe und beim heterocyclischen Rest $b_1$ zusätzlich $C_1$-$C_4$-Alkyl oder eine unsubstituierte oder eine 1 oder 2 Substituenten tragende Phenyloxygruppe, wobei die Substituenten gleich oder verschieden sein können und F, Cl, Br, $OCH_3$, $OC_2H_5$, $CH_3$ oder $C_2H_5$ bedeuten,

$R^4$ H, Cl, Br, $C_1$-$C_4$-Alkyl oder CN,

$R^5$ H, $C_1$-$C_4$-Alkyl, Prop-2-en-1-yl, Prop-2-in-1-yl oder Benzyl,

p 0 oder 1 bedeutet, wobei in $a_1$, $a_2$ und c der Phenylenrest über die 2,3 oder 4-Position gebunden ist, und

$R^6$ H, $C_1$-$C_8$-Alkyl, ($C_3$-$C_6$-Cycloalkyl)-$C_1$-$C_3$-alkyl, eine unsubstituierte oder 1 oder 2 Substituenten tragende Phenylgruppe, wobei die Substituenten gleich oder verschieden sein können und F, Cl, Br, J, $OCH_3$, $OC_2H_5$, 1,2--O-$CH_2$-O-, $CF_3$ oder $C_1$-$C_4$-Alkyl bedeuten, eine unsubstituierte oder im Phenylrest durch $OCH_3$, $OC_2H_5$, 1,2--O-$CH_2$-O-, $CF_3$, F, Cl oder $C_1$-$C_4$-Alkyl substituierte Phenyl-$C_1$-$C_2$-alkylgruppe, oder $CF_3$, und

$R^7$ und $R^8$ unabhängig voneinander H, $C_1$-$C_8$-Alkyl, $C_1$-$C_4$-Alkoxy oder eine unsubstituierte oder eine im Phenylrest 1 oder 2 Substituenten tragende Phenyl- oder Benzylgruppe, wobei die Substituenten gleich oder verschieden sein können und F, Cl, Br, $OCH_3$ oder $OC_2H_5$ bedeuten, und wobei $R^7$ darüberhinaus auch OH bedeuten kann, und

$R^9$ H, Cl, Br oder CN bedeutet, oder

$d_1$) 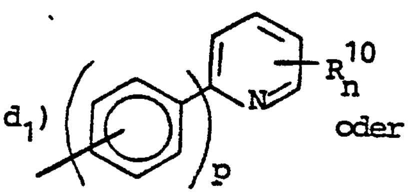 oder $d_2$) 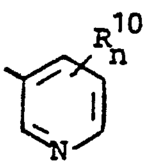 oder $d_3$) 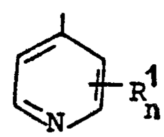

$d_4$) 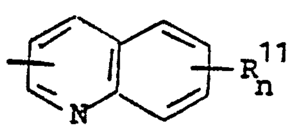 oder $d_5$) 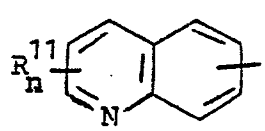 oder

$d_6$) 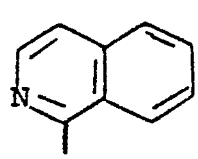 oder $d_7$) 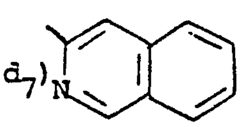

in denen

p 0 oder 1,

$R^{10}$ Cl, Br, $OCH_3$, $OC_2H_5$, $CH_3$ oder $C_2H_5$,

n 0, 1 oder 2 bedeutet, wobei $R^{10}$ in $d_1$ in 3-,4-,5- und/oder 6-, in $d_2$ in 2-,4-,5- und/oder 6- und in $d_3$ in 2-,3-,5- und/oder 6-Stellung stehen kann, und

$R^{11}$ Cl, $OCH_3$, $CH_3$ oder $C_2H_5$ bedeutet und in $d_4$ und $d_5$ in einer bzw. zwei der freien Positionen stehen kann, und der heterocyclische Rest $d_4$ über die 2-, 3- oder 4-Stellung und der heterocyclische Rest $d_5$ über die 5-,6-,7- oder 8-Stellung und die Phenyleneinheit im Rest $d_1$ über die 2,3 oder 4-(para)-Stellung gebunden ist, bedeutet,

sowie von deren physiologisch verträglichen Säureadditionssalzen, dadurch gekennzeichnet, daß man

A) eine Verbindung der Formel II

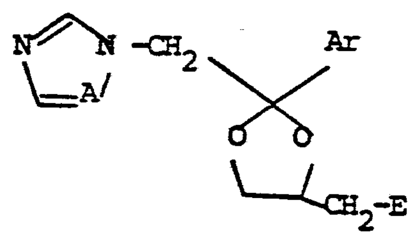

II,

in der

A und Ar die zu Formel I angegebenen Bedeutungen haben und

E Halogen, Acyloxy, $C_1$-$C_3$-Alkylsulfonyloxy, oder Arylsulfonyloxy bedeutet,

mit einer Verbindung der Formel III

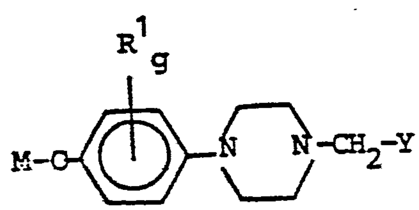

III,

in der

M H, ein Alkali- oder Erdalkalimetall bedeutet und

$R^1$, g und Y die zu Formel I angegebenen Bedeutungen haben, umsetzt, oder daß man

B) eine Verbindung der Formel IV,

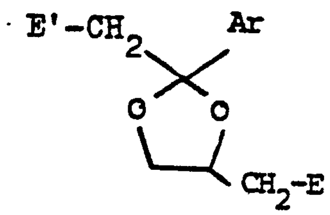

IV

in der

Ar die zu Formel I und E und E' die zu Formel II für E angegebenen Bedeutungen haben,
zunächst mit einer Verbindung der Formel III umsetzt und hierbei eine Verbindung der Formel V herstellt,

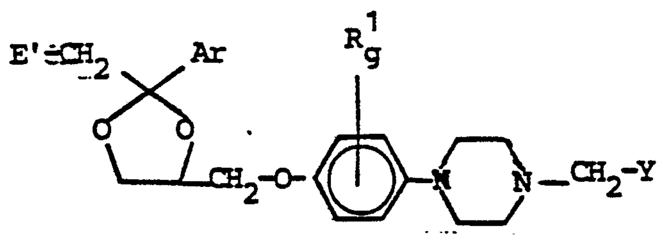

V

in der

Ar, $R^1$, g und Y die zu Formel I und E' die zu Formel II für E angegebenen Bedeutungen haben,
und anschließend eine Verbindung der Formel V mit einer Verbindung der Formel VI umsetzt,

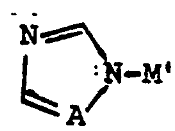

VI

in der A, CH oder N und

M' H, ein Alkali- oder Erdalkalimetall oder $Si(CH_3)_3$ bedeutet,

oder daß man

C) eine Verbindung der Formel VII,

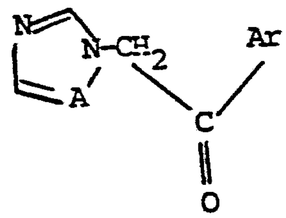

VII

in der A und Ar die zu Formel I angegebenen Bedeutungen haben, mit einem 1,2-Diol der Formel VIII,

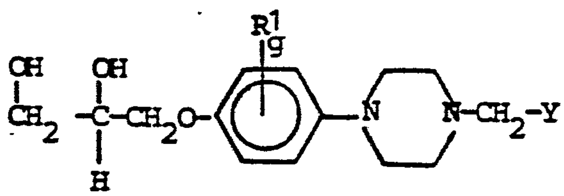

VIII

in der $R^1$, g und Y die zu Formel I angegebenen Bedeutungen haben, umsetzt,

oder daß man

D) eine Verbindung der Formel IX,

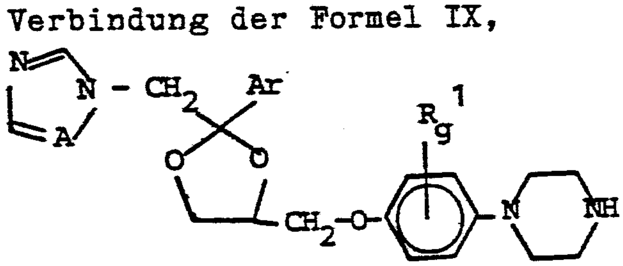

IX

in der A, Ar, $R^1$ und g die zu Formel I angegebenen Bedeutungen haben, mit einer Verbindung der Formel X,

$$E''-CH_2-Y \qquad X$$

in der E" Cl, Br, J, Acyloxy, Alkylsulfonyloxy oder Arylsulfonyloxy bedeutet und Y die zu Formel I unter $a_1$, $a_2$, $b_1$, $b_2$, c, $d_1$, $d_2$, $d_3$, $d_4$, $d_5$, $d_6$ und $d_7$ angegebenen Bedeutungen hat, umsetzt, und gegebenenfalls die nach Weg A)-D) erhaltenen Verbindungen der Formel I mit anorganischen oder organischen Säuren in ihre physiologisch verträglichen Säureadditionssalze überführt.

2. Verfahren zum Herstellen einer Verbindung I nach Anspruch 1, dadurch gekennzeichnet, daß mindestens einer der Substituenten oder Indices die folgende Bedeutung hat:

A CH oder N,

Ar eine durch 1 oder 2 F- oder Cl-Atome substituierte Phenylgruppe,

$R^1$ $CH_3$ oder $C_2H_5$,

g 0 oder 2,

Y

zu den heterocyclischen Resten $a_1$, $a_2$, $b_1$, $b_2$ und c:

$R^2$ H, $C_1$-$C_4$-Alkyl, $CH_3O$ oder eine unsubstituierte oder eine im Phenylrest 1 oder 2 Substituenten tragende Phenyl- oder eine Benzylgruppe, wobei die Substituenten gleich oder verschieden sein können und F, Cl, Br oder $OCH_3$ bedeuten,

$R^3$ H oder $C_1$-$C_4$-Alkoxy und beim heterocyclischen Rest $a_1$ zusätzlich $CH_3$ oder eine unsubstituierte oder eine mit einem F, Cl, $OCH_3$ oder $CH_3$ substituierte Phenylgruppe und beim Rest $b_1$ zusätzlich eine unsubstituierte oder eine 1 oder 2 Substituenten tragende Phenyloxygruppe, wobei die Substituenten gleich oder verschieden sein können und F, Cl, Br, $OCH_3$ oder $OC_2H_5$ bedeuten,

$R^4$ H oder CN und beim heterocyclischen Rest $a_1$ zusätzlich $C_1$-$C_4$-Alkyl,

$R^5$ $CH_3$,

p 0 oder 1,

$R^6$ H, $C_1$-$C_8$-Alkyl, ($C_5$-$C_6$-Cycloalkyl)-$C_1$-$C_2$-alkyl, eine unsubstituierte oder 1 oder 2 Substituenten tragende Phenylgruppe, wobei die Substituenten gleich oder verschieden sein können und F, Cl, Br, $OCH_3$, $OC_2H_5$ oder $CF_3$ bedeuten, eine unsubstituierte oder im Phenylrest durch $OCH_3$, $CF_3$, F oder Cl substituierte Benzylgruppe oder $CF_3$,

$R^7$, $R^8$ unabhängig voneinander $C_1$-$C_8$-Alkyl, $C_1$-$C_4$-Alkoxy, eine unsubstituierte oder eine im Phenylrest durch F, Cl, $OCH_3$ oder $OC_2H_5$ substituierte Phenyl- oder Benzylgruppe und $R^7$ darüber hinaus auch H oder OH,

$R^9$ H oder CN,

zu den heterocyclischen Resten $d_1$, $d_2$, $d_3$, $d_4$, $d_5$, $d_6$ und $d_7$:

p 0 oder 1,

$R^{10}$ Cl, $CH_3$ oder $C_2H_5$

n 0 oder 1, wobei $R^{10}$ in $d_3$ bevorzugt in 2-Stellung steht, und

$R^{11}$ Cl, wobei die Phenyleneinheit im Rest $d_1$ bevorzugt über die 4-Stellung gebunden ist.

3. Verfahren zum Herstellen einer Verbindung I nach Anspruch 1, dadurch gekennzeichnet, daß mindestens einer der Substituenten bzw. Indices die folgende Bedeutung hat:

A CH oder N,

Ar 2,4-Dichlorphenyl,

$R^1$ $CH_3$,

g 0 oder 2,

Y

zu den heterocyclischen Resten $a_1$, $a_2$, $b_1$, $b_2$ und c:

$R^2$ $CH_3$ oder eine unsubstituierte oder eine im Phenylrest 1 oder 2 Substituenten tragende Phenyl- oder eine Benzylgruppe, wobei die Substituenten gleich oder verschieden sein können und F oder Cl und in der Phenylgruppe und beim Rest $a_1$ bei der Phenyl- und der Benzylgruppe zusätzlich $OCH_3$ bedeuten, und beim Rest $a_1$ zusätzlich $C_2$-$C_4$-Alkyl,

$R^3$ beim Rest $a_1$, falls p=0 ist, $CH_3$ oder eine unsubstituierte oder durch ein F, Cl oder $OCH_3$ substituierte Phenylgruppe, und, falls p=1 ist, H, beim Rest $b_1$ H oder eine durch $OC_2H_5$ substituierte Phenyloxygruppe,

$R^4$ H oder beim Rest $a_1$, falls p=0 ist, zusätzlich $CH_3$

$R^5$ $CH_3$

p bei den heterocyclischen Resten $a_1$ und $a_2$ 0 oder 1 und bei dem heterocyclischen Rest c 1

$R^6$ H, $CH_3$ oder eine unsubstituierte oder durch F, Cl, $OCH_3$ oder $OC_2H_5$ einfach substituierte Phenylgruppe,

$R^7$,$R_8$ unabhängig voneinander $CH_3$ oder eine unsubstituierte oder eine durch F, Cl oder $OCH_3$ substituierte Phenylgruppe und $R^7$ darüber hinaus auch H oder $C_1$-$C_4$-Alkoxy,

$R^9$ H

wobei der Rest d4 bevorzugt über die 2-Stellung und der Rest d5 bevorzugt über die 6- oder 7-Stellung gebunden ist.

4. Verfahren zum Herstellen von Verbindungen der Formel IIIa

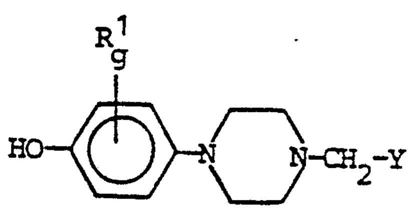

IIIa,

in der bedeuten:

$R^1$ $C_1$-$C_3$-Alkyl, F oder Cl,

g 0, 1 oder 2 und

Y die folgenden heterocyclischen Reste

a₁) 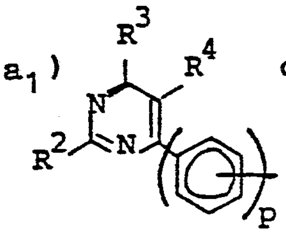 oder a₂) 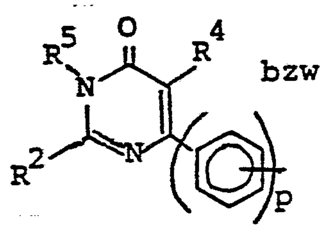 bzw. 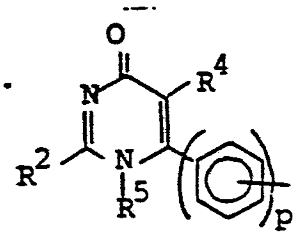

oder

b₁) 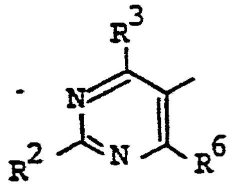 oder b₂) 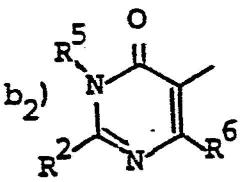 bzw. 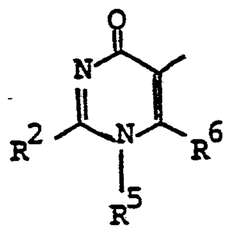

oder

c) 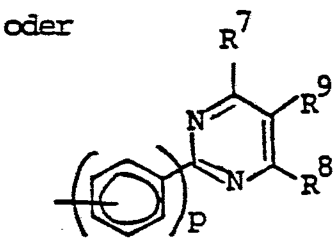

in denen

$R^2$ H, $C_1$-$C_8$-Alkyl, $C_1$-$C_4$-Alkoxy, eine unsubstituierte oder 1 oder 2 Substituenten tragende Phenylgruppe, wobei die Substituenten gleich oder verschieden sein können und F, Cl, Br, J, Trifluormethyl, Methoxy, Ethoxy, Nitro oder $C_1$-$C_4$-Alkyl bedeuten, oder eine unsubstituierte oder eine im Phenylrest 1 oder 2 Substituenten tragende Phenyl-$C_1$-$C_2$-alkylgruppe, wobei die Substituenten gleich oder verschieden sein können und F, Cl, Br, $OCH_3$, $OC_2H_5$, $CH_3$ oder $C_2H_5$ bedeuten,

oder bei den heterocyclischen Resten $a_1$ und $a_2$, falls p Null bedeutet, darüberhinaus auch $C_1$-$C_4$-Alkylthio oder eine unsubstituierte oder eine im Phenylrest 1 oder 2 Substituenten tragende Benzylthiogruppe, wobei die Substituenten gleich

oder verschieden sein können und F, Cl, $OCH_3$, $OC_2H_5$, $CH_3$ oder $C_2H_5$ bedeuten, bedeutet und

$R^3$ H, OH, Cl, $C_1$-$C_4$-Alkoxy oder eine unsubstituierte oder eine im Phenylrest 1 oder 2 Substituenten tragende Benzyloxygruppe, wobei die Substituenten gleich oder verschieden sein können und F, Cl, Br, $OCH_3$, $OC_2H_5$, $CH_3$ oder $C_2H_5$ bedeuten, und beim heterocyclischen Rest $a_1$ zusätzlich $C_1$-$C_4$-Alkyl oder eine unsubstituierte oder mit einem F, Cl, Br, $OCH_3$, $OC_2H_5$, $CH_3$ oder $C_2H_5$ substituierte Phenylgruppe und beim heterocyclischen Rest b1 zusätzlich $C_1$-$C_4$-Alkyl oder eine unsubstituierte oder eine 1 oder 2 Substituenten tragende Phenyloxygruppe, wobei die Substituenten gleich oder verschieden sein können und F, Cl, Br, $OCH_3$, $OC_2H_5$, $CH_3$ oder $C_2H_5$ bedeuten,

$R^4$ H, Cl, Br oder CN oder $C_1$-$C_4$-Alkyl

$R^5$ H, $C_1$-$C_4$-Alkyl, Prop-2-en-1-yl, Prop-2-in-1-yl oder Benzyl

p 0 oder 1 bedeutet, wobei in $a_1$, $a_2$ und c der Phenylenrest über die 2,3 oder 4-Position gebunden ist und

$R^6$ H, $C_1$-$C_8$-Alkyl, ($C_3$-$C_6$-Cycloalkyl)-$C_1$-$C_3$-alkyl, eine unsubstituierte oder 1 oder 2 Substituenten tragende Phenylgruppe, wobei die Substituenten gleich oder verschieden sein können und F, Cl, Br, J, $OCH_3$, $OC_2H_5$, 1.2--O-$CH_2$-O-, $CF_3$ oder $C_1$-$C_4$-Alkyl bedeuten, eine unsubstituierte oder im Phenylrest durch $OCH_3$, $OC_2H_5$, 1.2--O-$CH_2$-O-, $CF_3$, F, Cl oder $C_1$-$C_4$-Alkyl substituierte Phenyl-$C_1$-$C_2$-alkylgruppe, oder $CF_3$, und

$R^7$ und $R^8$ unabhängig voneinander H, $C_1$-$C_8$-Alkyl, $C_1$-$C_4$-Alkoxy oder eine unsubstituierte oder eine im Phenylrest 1 oder 2 Substituenten tragende Phenyl- oder Benzylgruppe, wobei die Substituenten gleich oder verschieden sein können und F, Cl, Br, $OCH_3$ oder $OC_2H_5$ bedeuten, und wobei $R^7$ darüberhinaus auch OH bedeuten kann, und

$R^9$ H, Cl, Br oder CN bedeutet, oder

$d_1$) 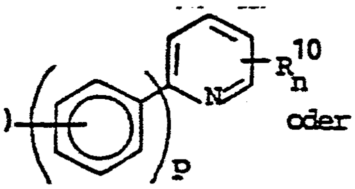 oder $d_2$) 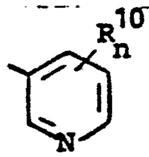 oder $d_3$) 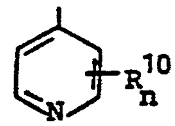 oder

$d_4$) 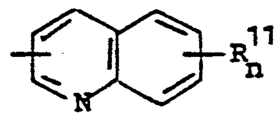 oder $d_5$) 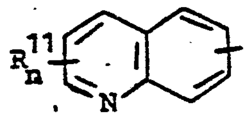 oder

$d_6$) 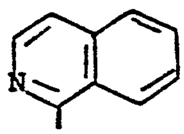 oder $d_7$) 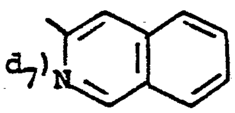

in denen

p 0 oder 1,

$R^{10}$ Cl, Br, $OCH_3$, $OC_2H_5$, $CH_3$ oder $C_2H_5$,

n 0, 1 oder 2 bedeutet, wobei $R^{10}$ in $d_1$ in 3-,4-,5-und/oder 6-, in $d_2$ in 2-,4-,5-und/oder 6- und in $d_3$ in 2-,3-,5-und/oder 6-Stellung steht, und

$R^{11}$ Cl, $OCH_3$, $CH_3$ oder $C_2H_5$ bedeutet und in $d_4$ und $d_5$ in einer bzw. zwei der freien Positionen steht, und der heterocyclische Rest $d_4$ über die 2-,3-oder 4-Stellung und der heterocyclische Rest $d_5$ über die 5-,6-,7- oder 8-Stellung und die Phenyleneinheit im Rest $d_1$ über die 2-,3-oder 4-(para)-Stellung gebunden ist, bedeutet,

sowie von deren Säureadditionssalzen, dadurch gekennzeichnet, daß man eine Verbindung der Formel XIV

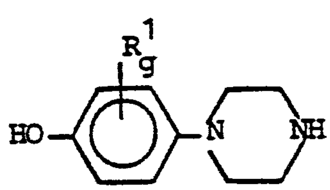

XIV,

in der $R^1$ und g die zu Formel I angegebenen Bedeutungen haben, oder ein Salz dieser Verbindung, mit einer Verbin-

dung der Formel X,

$$E''\text{-}CH_2\text{-}Y \qquad X,$$

in der E" Cl, Br, J, Acyloxy, Alkylsulfonyloxy oder Arylsulfonyloxy bedeutet und

Y die zur Formel I unter $a_1$, $a_2$, $b_1$, $b_2$, c, $d_1$, $d_2$, $d_3$, $d_4$, $d_5$, $d_6$ und $d_7$ angegebenen Bedeutungen hat,

umsetzt und gegebenenfalls die erhaltenen Verbindungen der Formel IIIa mit anorganischen oder organischen Säuren in ihre Säureadditionssalze überführt.

5. Verfahren nach Anspruch 4, dadurch gekennzeichnet, daß man für die Umsetzung mit Verbindungen der Formel XIV entweder

a) eine Verbindung der Formel $Xa_1$ oder der Formel $Xa_2$,

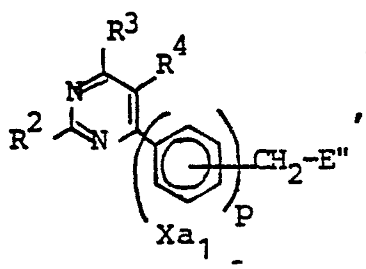

$Xa_1$ ,

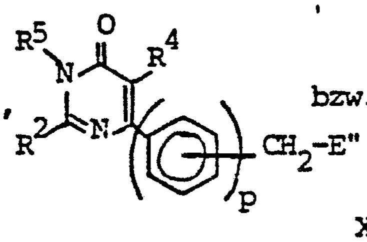

bzw.

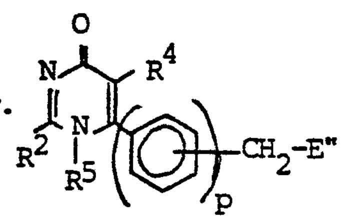

$Xa_2$

in denen $R^2$-$R^5$ die zu Formel I angegebenen Bedeutungen haben und E" Cl oder Br bedeutet, oder

b) eine Verbindung der Formel $Xb_1$ oder der Formel $Xb_2$,

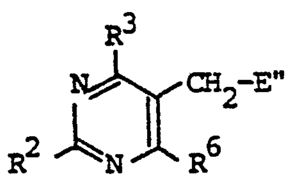

$Xb_1$

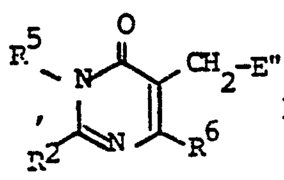

bzw.

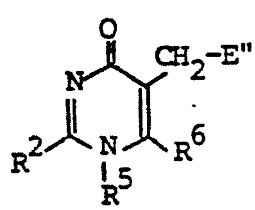

$Xb_2$

in denen $R^2$, $R^3$, $R^5$ und $R^6$ die zu Formel I angegebenen Bedeutungen haben und E" Br bedeutet, oder

c) eine Verbindung der Formel Xc,

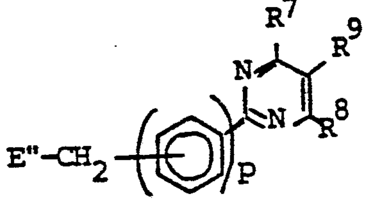

Xc,

in der $R^7$, $R^8$, $R^9$ und p die zu Formel I angegebenen Bedeutungen haben und

E" Cl oder Br bedeutet, oder

d) eine Verbindung der Formel $Xd_1$, der Formel $Xd_2$, der Formel $Xd_3$, der Formel $Xd_4$, der Formel $Xd_5$, der Formel $Xd_6$ oder der Formel $Xd_7$

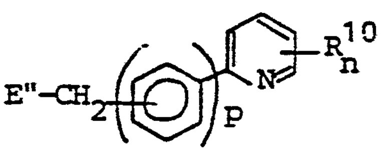

$Xd_1$,

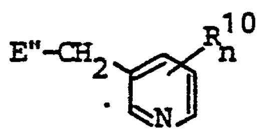

$Xd_2$,

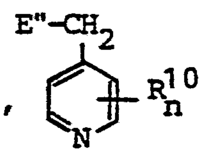

$Xd_3$

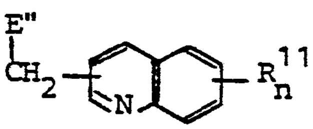

$Xd_4$,

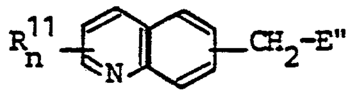

$Xd_5$

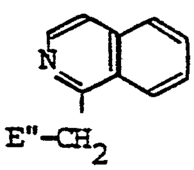

$Xd_6$

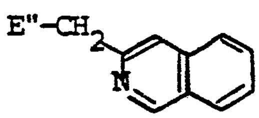

$Xd_7$

in denen $R^{10}$, $R^{11}$, p und n die zu Formel I angegebenen Bedeutungen haben und

E" Cl oder Br bedeutet, einsetzt.

6. Verfahren zum Herstellen einer Verbindung Verbindung der Formel IIIa nach Anspruch 4, dadurch gekennzeichnet, daß mindestens einer der Substituenten oder Indices $R^1$ g, Y, $R^2$-$R^{11}$, p und n die folgenden Bedeutungen hat:

$R^1$ $CH_3$ oder $C_2H_5$
g 0 oder 2
Y

zu den heterocyclischen Resten $a_1$, $a_2$, $b_1$, $b_2$ und c:

$R^2$ H, $C_1$-$C_4$-Alkyl, $CH_3O$ oder eine unsubstituierte oder eine im Phenylrest 1 oder 2 Substituenten tragende Phenyl- oder eine Benzylgruppe, wobei die Substituenten gleich oder verschieden sein können und
$R^3$ H oder $C_1$-$C_4$-Alkoxy und beim Rest $a_1$ zusätzlich $CH_3$ oder eine unsubstituierte oder eine mit einem F, Cl, $OCH_3$ oder $CH_3$ substituierte Phenylgruppe und beim Rest $b_1$ zusätzlich eine unsubstituierte oder eine 1 oder 2 Substituenten tragende Phenyloxygruppe, wobei die Substituenten gleich oder verschieden sein können und F, Cl, Br, $OCH_3$ oder $OC_2H_5$ bedeuten,
$R^4$ H oder CN,
$R^5$ $CH_3$,
p 0 oder 1
$R^6$ H, $C_1$-$C_8$-Alkyl, ($C_5$-$C_6$-Cycloalkyl)-$C_1$-$C_2$-alkyl, eine unsubstituierte oder 1 oder 2 Substituenten tragende Phenylgruppe, wobei die Substituenten gleich oder verschieden sein können und F, Cl, Br, $OCH_3$, $OC_2H_5$ oder $CF_3$ bedeuten, eine unsubstituierte oder im Phenylrest durch $OCH_3$, $CF_3$, F oder Cl substituierte Benzylgruppe oder $CF_3$,
$R^7$, $R^8$ unabhängig voneinander $C_1$-$C_8$-Alkyl, $C_1$-$C_4$-Alkoxy- oder eine unsubstituierte oder eine im Phenylrest durch F, Cl, $OCH_3$ oder $OC_2H_5$ substituierte Phenyl- oder Benzylgruppe und $R^7$ darüber hinaus auch H oder OH,
$R^9$ H oder CN,

zu den heterocyclischen Resten $d_1$, $d_2$, $d_3$, $d_4$, $d_5$, $d_6$ und $d_7$:

p — 0 oder 1,

$R^{10}$ — Cl, $CH_3$ oder $C_2H_5$,

n — 0 oder 1, wobei $R^{10}$ in $d_3$ bevorzugt in 2-Stellung steht, und

$R^{11}$ — Cl, wobei die Phenyleneinheit im Rest $d_1$ bevorzugt über die 4-Stellung gebunden ist.

7. Verfahren zur Herstellung einer pharmazeutischen Zubereitung, dadurch gekennzeichnet, daß man eine Verbindung der Formel (I) nach Anspruch 1 oder eines ihrer Salze, gegebenenfalls zusammen mit einem üblichen pharmazeutischen Trägerstoff und/oder Hilfsstoff, in eine für pharmazeutische Zwecke geeignete Darreichungsform bringt.

8. Verwendung einer Verbindung I nach Anspruch 1 als Antimykotikum.

9. Verwendung einer Verbindung der Formel I nach Anspruch 1 zur Herstellung eines antimykotisch wirkenden Arzneimittels.

10. Verfahren zum Behandeln von Mykosen, dadurch gekennzeichnet, daß man eine wirksame Menge einer Verbindung I nach Anspruch 1 zusammen mit pharmazeutisch geeigneten Trägerstoffen appliziert.